# EUROPEAN PATENT SPECIFICATION

(11) **EP 1 608 759 B1**
(45) Date of publication and mention of the grant of the patent: **02.01.2008**
(21) Application number: 04722310.2
(22) Date of filing: 22.03.2004
(51) Int. Cl.: C12N 15/82, C12N 15/53, C12N 15/54, C12N 15/62, A01H 5/10, A01H 5/00, C07C 403/00

(54) **ENHANCED ACCUMULATION OF CAROTENOIDS IN PLANTS**
ERHÖHTE ANSAMMLUNG VON KAROTENOIDEN IN PFLANZEN
ACCUMULATION RENFORCEE DE CAROTENOIDES DANS LES PLANTES

(30) Priority: 24.03.2003 US 457053 P
(43) Date of publication of application: 28.12.2005
(73) Proprietor: Syngenta Limited, Guildford Surrey GU2 7YH (GB)
(72) Inventor: DRAKE, Caroline, Rachel, Syngenta Limited, Bracknell, Berks RG42 6EY (GB); PAINE, Jacqueline, Ann, Mary, Syngenta Limited, Bracknell, Berks RG42 6EY (GB); SHIPTON, Catherine, Ann, Syngenta Limited, Bracknell, Berks RG42 6EY (GB)
(74) Representative: Kock, Michael Andreas
(86) International application number: PCT/GB2004/001241
(87) International publication number: WO 2004/085656

(56) References cited:
- WO-A-00/53768
- WO-A-02/16583
- WO-A-98/06862
- ROEMER S ET AL: "ELEVATION OF THE PROVITAMIN A CONTENT OF TRANSGENIC TOMATO PLANTS" NATURE BIOTECHNOLOGY, NATURE PUBLISHING, US, vol. 18, no. 6, June 2000 (2000-06), pages 666-669, XP001155851 ISSN: 1087-0156
- BEYER P ET AL: "GOLDEN RICE: INTRODUCING THE BETA-CAROTENE BIOSYNTHESIS PATHWAY INTO RICE ENDOSPERM BY GENETIC ENGINEERING TO DEFEAT VITAMIN A DEFICIENCY" JOURNAL OF NUTRITION, WISTAR INSTITUTE OF ANATOMY AND BIOLOGY, PHILADELPHIA, PA,, US, vol. 132, no. 3, March 2002 (2002-03), pages 506S-510S, XP001069128 ISSN: 0022-3166
- KUMAGAI M H ET AL: "CYTOPLASMIC INHIBITION OF CAROTENOID BIOSYNTHESIS WITH VIRUS-DERIVED RNA" PROCEEDINGS OF THE NATIONAL ACADEMY OF SCIENCES OF USA, NATIONAL ACADEMY OF SCIENCE. WASHINGTON, US, vol. 92, 1 February 1995 (1995-02-01), pages 1679-1683, XP002012921 ISSN: 0027-8424
- BARTLEY G E ET AL: "TWO ARABIDOPSIS THALIANA CAROTENE DESATURASES, PHYTOENE DESATURASE AND DZETA-CAROTENE DESATURASE, EXPRESSED IN ESCHERICHIA COLI, CATALYZE A POLY-CIS PATHWAY TO YIELD PRO-LYCOPENE" EUROPEAN JOURNAL OF BIOCHEMISTRY, BERLIN, DE, vol. E-259, no. E-1/2, 1999, pages 396-403, XP000925505 ISSN: 0014-2956
- RAVANELLO MONICA P ET AL: "Coordinate expression of multiple bacterial carotenoid genes in canola leading to altered carotenoid production." METABOLIC ENGINEERING. OCT 2003, vol. 5, no. 4, October 2003 (2003-10), pages 255-263, XP002295220 ISSN: 1096-7176
- PAINE J. ET AL: 'Improving the nutritional value of Gloden Rice through increased pro-vitamin A content' NATURE BIOTECHNOLOGY vol. 23, no. 4, April 2005, pages 482 - 487

## Description

The present invention relates *inter alia,* to recombinant DNA technology. More specifically the invention relates to the provision of improved polynucleotides which provide for an enhanced accumulation of carotenoids in plants and in particular in the seeds of said plants. The invention also provides plant material, plants and seeds which comprise the polynucleotides, in particular rice plant material, rice plants and rice seeds.

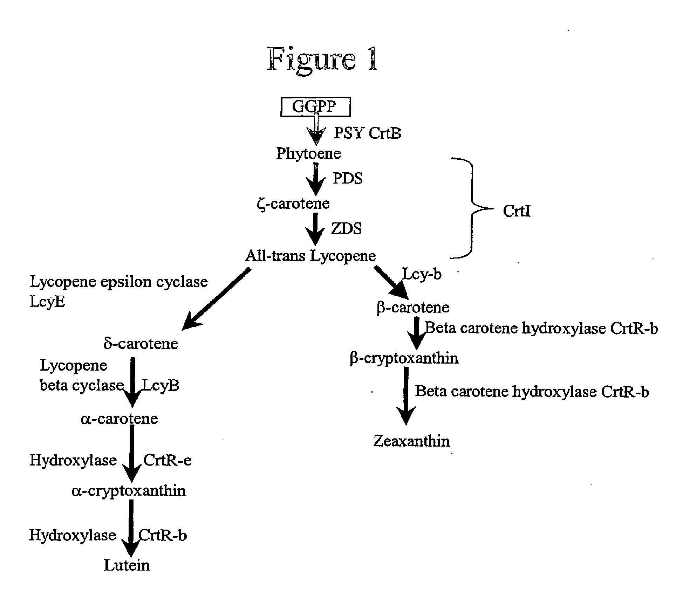

Carotenoids are 40-carbon (C₄₀) isoprenoids formed by condensation of eight isoprene units derived from the biosynthetic precursor isopentenyl diphosphate. By nomenclature, carotenoids basically fall into two classes, namely, carotenes and xanthophylls. Their essential function in plants is to protect against photo-oxidative damage in the photosynthetic apparatus of plastids. In addition to this, carotenoids participate in light harvesting during photosynthesis and represent integral components of photosynthetic reaction centres. Carotenoids are the direct precursors of the phytohormone abscisic acid. A part of the carotenoid biosynthesis pathway is shown in Figure 1.

Carotenoids with provitamin A activity are essential components of the human diet. Additionally, there is compelling evidence suggesting that a diet rich in carotenoids can prevent a number of serious medical conditions from developing, including certain cancers (especially lung and prostate), macular degeneration, cateract and cardiovascular disease. Carotenoids have also been reported to have immunomodulatory effects, such as the reduction in UV-induced immunosuppression. Carotenoids are able to act as efficient quenchers of harmful reactive oxygen species such as singlet oxygen and thereby have antioxidant properties.

With the population of the world increasing, there remains a need for the production of foods which are high in nutrition, healthy, tasty and visually appealing.

The general insertion of genes involved in the carotenoid biosynthesis pathway into plants is disclosed in WO00/53768. US6,429,356 describes methods for the production of plants and seeds having altered fatty acid, tocopherol and carotenoid compositions via insertion of a crtB gene. The present invention provides, *inter alia,* improved polynucleotides which when inserted into plant material provide for a surprisingly high accumulation of carotenoids in at least the seeds of plants derived from said material. More specifically, the present invention provides particular combinations of nucleotide sequences which, when expressed in plant material provide for a surprisingly high accumulation of carotenoids in at least the seeds of plants derived from said material.

According to the present invention there is provided a polynucleotide comprising:
(a) a region which comprises as operably linked components (i) a promoter which provides for seed preferred expression; and (ii) a nucleotide sequence derived from a bacterium which sequence encodes a carotene desaturase; and (iii) a transcription termination region; and (b) a further region which comprises as operably linked components (i) a promoter which provides for seed preferred expression; and (ii) a nucleotide sequence encoding a phytoene synthase which sequence is derived from maize (*Zea* sp.) or rice (*Orzya* sp.); and (iii) a transcription termination region.

Further disclosed is a polynucleotide comprising: (a) a region which comprises as operably linked components (i) a promoter which provides for seed preferred expression; and (ii) a nucleotide sequence derived from a bacterium which sequence encodes a carotene desaturase; and (iii) a transcription termination region; and
(b) a further region which comprises as operably linked components (i) a promoter which provides for seed preferred expression; and (ii) a nucleotide sequence encoding a phytoene synthase which sequence is derived from tomato (*Lycopersicon* sp.) or pepper (*Capsicum* sp.) or a bacterium; and (iii) a transcription termination region.

In a further embodiment said carotene desaturase is derivable from *Streptomyces; Staphylococcus; Synechocystis; Rhodobacter; Paracoccus; Erwinia;* and *Xanthophyllomyces.* In a further embodiment said carotene desaturase is a phytoene desaturase.

In a further embodiment of the invention said phytoene synthase is obtainable from *Zea mays or Orzya sativa.*

In a further embodiment said phytoene synthase is obtained from *Zea mays* or *Orzya sativa.* In a still further embodiment said phytoene synthase comprises, is comprised by or consists of the sequence selected from the group depicted as SEQ ID NOS: 10; 11; 12; and 13. In a further embodiment said phytoene synthase comprises, is comprised by or consists of a sequence which encodes the protein depicted as SEQ ID NO 14. Alternative phytoene synthase encoding sequences may also be available from databases known to the person skilled in the art. For example, the sequences depicted in the EMBL Database under Accession Numbers - AY024351, AK073290, AK108154 and AY078162.

Further disclosed is a phytoene synthase derived, obtainable or obtained from *Lycopersicon esculentum* or *Capsicum anuum.* Still further disclosed is a wherein the phytoene synthase comprises, is comprised by or consists of SEQ ID NO: 15 or SEQ ID NO: 16.

Further disclosed is a synthase derived, obtainable or obtained from the CrtB gene from bacteria. In a particular said crtB comprises, is comprised by or consists of the crtB sequence depicted in Shewmaker et al. (1999) Plant J. 20:401-12. In a further embodiment of the invention the sequence which encodes a carotene desaturase from a bacteria is derived from *Erwinia sp.,* more specifically, *Erwinia uredovora.* In a still further embodiment the carotene desaturase is derived from the CrtI gene from *Erwinia uredovora.* In a still further embodiment the carotene desaturase is the CrtI gene from *Erwinia uredovora.* In a still further embodiment the carotene desaturase comprises, is comprised by or consists of the sequence depicted as SEQ ID NO: 18 or SEQ ID NO: 19.

The present invention further provides a polynucleotide as described above wherein said promoter is selected from the Glutelin 1 promoter and the Prolamin promoter and said transcription termination region is selected from the Nos; CaMV 35S and PotP1-II transcription termination regions. Said Glutelin 1 and Prolamin promoters may be isolated from rice. In a particular embodiment said promoter comprises the sequence depicted as SEQ ID NO: 20 or 21. Further promoters include the promoter derived from the napin gene from *Brassica napus* and other promoters which are derived from genes normally expressed in the endosperm of the seed. Further transcription termination regions include the terminator region of a gene of alpha-tubulin (EP-A 652,286). It is equally possible to use, in association with the promoter regulation sequence, other regulation sequences which are situated between the promoter and the sequence encoding the protein, such as transcriptional or translational enhancers, for example, tobacco etch virus (TEV) translation activator described in International Patent application, PCT publication number WO87/07644.

The present invention still further provides a polynucleotide as described above wherein the sequence which encodes carotene desaturase and the sequence which encodes phytoene synthase further comprises a plastid targeting sequence. In a particular embodiment the plastid targeting sequence is derived from the ribulose bisphosphate carboxylase small-subunit (RUBISCO Ssu) from *Pisum sativum.* In a further embodiment said RUBISCO Ssu plastid targeting sequence is located 5' to the translational start point of said carotene desaturase gene derived from CrtI. In a still further embodiment said RUBISCO Ssu plastid targeting sequence is located 5' to the translational start point of said phytoene synthase gene derived from CrtB. In a further embodiment said plastid targeting sequence is heterologous with respect to said phytoene synthase and/or said carotene desaturase. In a still further embodiment said plastid targeting sequence is autologous with respect to said phytoene synthase and/or said carotene desaturase. By "heterologous" is meant from a different source, and correspondingly "autologous" means from the same source - but at a gene rather than organism or tissue level. In a still further embodiment the plastid targeting sequence associated with the carotene desaturase is heterologous therewith and the plastid targeting sequence associated with the phytoene synthase is autologous therewith. In a still further embodiment the plastid targeting sequence provides for the accumulation of carotenoids in the amyloplast of the seed.

The present invention still further provides a polynucleotide as described above wherein the sequence which encodes said carotene desaturase and/or the sequence which encodes said phytoene synthase further comprises an intron. In a particular embodiment said intron region is located between the promoter region and the region encoding the carotene desaturase/phytoene synthase. In a further embodiment said intron region is located between the promoter region and the plastid targeting sequence. In a still further embodiment said intron region is located upstream of said carotene desaturase and/or said phytoene synthase encoding sequence(s). In a still further embodiment said intron is derived from the intron of the first gene from the catalase gene of the castor bean plant. In a still further embodiment said intron is from the first intron of the catalase gene from the castor bean plant. In a still further embodiment said intron comprises the sequence depicted as SEQ ID NO: 22. In a still further embodiment the intron is the intron of the maize polyubiquitin gene.

The present invention further provides a polynucleotide as described above wherein said sequence encoding carotene desaturase is located 5' to said sequence encoding phytoene synthase.

The present invention further provides a polynucleotide as described above wherein said sequence encoding phytoene synthase is located 5' to said sequence encoding carotene desaturase.

The present invention still further provides a polynucleotide as described above which comprises the sequence selected from the group depicted as SEQ ID NOS: 1; 2; 3; 4; 5; and 6. In a particular embodiment of the invention the polynucleotide consists of a sequence selected from the group depicted as SEQ ID NO: 1; 2; 3; 4; 5 and 6. In a further embodiment of the invention the polynucleotide comprises or consists of SEQ ID NO: 1. In a still further embodiment of the invention the polynucleotide comprises or consists of SEQ ID NO: 2. In a still further embodiment of the invention the polynucleotide comprises or consists of SEQ ID NO: 3. In a still further embodiment of the invention the polynucleotide comprises or consists of SEQ ID NO: 4. In a still further embodiment of the invention the polynucleotide comprises or consists of SEQ ID NO: 6. The present invention still further provides a polynucleotide as described above which comprises or consists of a sequence selected from the group depicted as SEQ ID NOS: 7; 8; and 9.

The present invention still further provides a polynucleotide sequence which is the complement of one which hybridises to a polynucleotide as described in the preceding paragraph at a temperature of about 65°C in a solution containing 6 x SSC, 0.01% SDS and 0.25% skimmed milk powder, followed by rinsing at the same temperature in a solution containing 0.2 x SSC and 0.1% SDS wherein said polynucleotide sequence still comprises a region encoding a carotene desaturase and a further region encoding a phytoene synthase and when said polynucleotide sequence is inserted into plant material the seed of a plant regenerated from said material produce an increased amount of carotenoids when compared to a control like-seed. The skilled person may alternatively select the following hybridisation conditions, *viz.,* hybridisation at a temperature of between 60°C and 65°C in 0.3 strength citrate buffered saline containing 0.1% SDS followed by rinsing at the same temperature with 0.3 strength citrate buffered saline containing 0.1% SDS followed by confirmation that when the polynucleotide sequence so identified is inserted into plant material the seed of a plant regenerated from said material produce an increased amount of carotenoids when compared to a control like-seed. The person skilled in the art may also select further hybridisation conditions that are equally understood to be "high stringency" conditions. In a particular embodiment of the present invention when said polynucleotide sequence is inserted into plant material, the seed of a plant regenerated from said material produces at least a sixty fold increase in carotenoids when compared to a control like-seed. In a further embodiment of the invention when said polynucleotide sequence is inserted into plant material, the seed of a plant regenerated from said material produces at least a one hundred fold increase in carotenoids when compared to a control like-seed. In a still further embodiment of the invention when said polynucleotide sequence is inserted into plant material, the seed of a plant regenerated from said material produces at least a one hundred and fifty fold increase in carotenoids when compared to a control like-seed. In a still further embodiment of the invention when said polynucleotide sequence is inserted into plant material, the seed of a plant regenerated from said material produces at least a two hundred fold increase in carotenoids when compared to a control like-seed. In a still further embodiment of the invention when said polynucleotide sequence is inserted into plant material, the seed of a plant regenerated from said material produces at least a two hundred and fifty fold increase in carotenoids when compared to a control like-seed. In a still further embodiment of the invention when said polynucleotide sequence is inserted into plant material, the seed of a plant regenerated from said material produces at least a three hundred fold increase in carotenoids when compared to a control like-seed. In a still further embodiment of the invention when said polynucleotide sequence is inserted into plant material, the seed of a plant regenerated from said material produces at least a three hundred and fifty fold increase in carotenoids when compared to a control like-seed. In a still further embodiment of the invention when said polynucleotide sequence is inserted into plant material, the seed of a plant regenerated from said material produces at least a four hundred fold increase in carotenoids when compared to a control like-seed. In a still further embodiment of the invention when said polynucleotide sequence is inserted into plant material, the seed of a plant regenerated from said material produces at least a five hundred fold increase in carotenoids when compared to a control like-seed.

The term control like-seed relates to seeds which are substantially similar to those according to the invention but which control like-seed does not contain the polynucleotides or polynucleotide sequences according to the invention. Typically, a control like-seed will comprise a seed of the same or similar plant species which control like-seed has not been transformed. The increased carotenoid content of the seeds comprising the polynucleotides or polynucleotide sequences according to the invention may also be demonstrated via comparison of said seeds with seeds that comprise the TDNA depicted in Plasmid A of Figure 4 of WO00/53768, wherein the phytoene synthase (psy) is from daffodil (*Narcissus pseudonarcissus).* Typically, such a comparison would be made when the seed to be compared are of the same or a substantially similar plant species. In a particular embodiment the seed comprising the polynucleotides or polynucleotide sequences according to the invention contain at least three times the amount of carotenoids when compared to a seed that comprise the TDNA depicted in Plasmid A of Figure 4 of WO00/53768, wherein the phytoene synthase (psy) is from daffodil (*Narcissus pseudonarcissus).* In a still further embodiment the seed comprising the polynucleotides or polynucleotide sequences according to the invention contains at least four times, or at least five times, or at least six times, or at least seven times, or at least eight times or at least nine times or at least ten times or at least fifteen times or at least twenty times or at least thirty times, or at least forty times or at least fifty times the amount of carotenoids when compared to a seed that comprise the TDNA depicted in Plasmid A of Figure 4 of WO00/53768, wherein the phytoene synthase (psy) is from daffodil (*Narcissus pseudonarcissus).*

The present invention still further provides a polynucleotide sequence as described above wherein when said polynucleotide sequence is inserted into plant material, the seed of a plant regenerated from said material produces carotenoids at a level of at least 3µg/g of endosperm of said seed. In a further embodiment when said polynucleotide sequence as described above is inserted into plant material, the seed of a plant regenerated from said material produces carotenoids at a level of at least 4µg/g of endosperm of said seed. In a further embodiment when said polynucleotide sequence as described above is inserted into plant material, the seed of a plant regenerated from said material produces carotenoids at a level of at least 5µg/g of endosperm of said seed. In a still further embodiment when said polynucleotide sequence as described above is inserted into plant material, the seed of a plant regenerated from said material produces carotenoids at a level of at least 6µg/g of endosperm of said seed In a still further embodiment when said polynucleotide sequence as described above is inserted into plant material, the seed of a plant regenerated from said material produces carotenoids at a level of at least 7µg/g of endosperm of said seed. In a still further embodiment when said polynucleotide sequence as described above is inserted into plant material, the seed of a plant regenerated from said material produces carotenoids at a level of at least 8µg/g of endosperm of said seed. In a still further embodiment when said polynucleotide sequence as described above is inserted into plant material, the seed of a plant regenerated from said material produces carotenoids at a level of at least 9µg/g of endosperm of said seed. In a still further embodiment when said polynucleotide sequence as described above is inserted into plant material, the seed of a plant regenerated from said material produces carotenoids at a level of at least 10µg/g of endosperm of said seed. In a still further embodiment when said polynucleotide sequence as described above is inserted into plant material, the seed of a plant regenerated from said material produces carotenoids at a level of at least 11 µg/g of endosperm of said seed. In a still further embodiment when said polynucleotide sequence as described above is inserted into plant material, the seed of a plant regenerated from said material produces carotenoids at a level of at least 12µg/g of endosperm of said seed. In a still further embodiment when said polynucleotide sequence as described above is inserted into plant material, the seed of a plant regenerated from said material produces carotenoids at a level of at least 13µg/g of endosperm of said seed. In a still further embodiment when said polynucleotide sequence as described above is inserted into plant material, the seed of a plant regenerated from said material produces carotenoids at a level of at least 14µg/g of endosperm of said seed. In a still further embodiment when said polynucleotide sequence as described above is inserted into plant material, the seed of a plant regenerated from said material produces carotenoids at a level of at least 15µg/g of endosperm of said seed. In a still further embodiment when said polynucleotide sequence as described above is inserted into plant material, the seed of a plant regenerated from said material produces carotenoids at a level of at least 20µg/g of endosperm of said seed. In a still further embodiment when said polynucleotide sequence as described above is inserted into plant material, the seed of a plant regenerated from said material produces carotenoids at a level of at least 25µg/g of endosperm of said seed. In a still further embodiment when said polynucleotide sequence as described above is inserted into plant material, the seed of a plant regenerated from said material produces carotenoids at a level of at least 30µg/g of endosperm of said seed. In a still further embodiment when said polynucleotide sequence as described above is inserted into plant material, the seed of a plant regenerated from said material produces carotenoids at a level of at least 35µg/g of endosperm of said seed. In a still further embodiment when said polynucleotide sequence as described above is inserted into plant material, the seed of a plant regenerated from said material produces carotenoids at a level of at least 40µg/g of endosperm of said seed. In a still further embodiment when said polynucleotide sequence as described above is inserted into plant material, the seed of a plant regenerated from said material produces carotenoids at a level of at least 45µg/g of endosperm of said seed. In a still further embodiment when said polynucleotide sequence as described above is inserted into plant material, the seed of a plant regenerated from said material produces carotenoids at a level of at least 50µg/g of endosperm of said seed. In a still further embodiment when said polynucleotide sequence as described above is inserted into plant material, the seed of a plant regenerated from said material produces carotenoids at a level of at least 55µg/g of endosperm of said seed. In a still further embodiment when said polynucleotide sequence as described above is inserted into plant material, the seed of a plant regenerated from said material produces carotenoids at a level of at least 60µg/g of endosperm of said seed. In a still further embodiment when said polynucleotide sequence as described above is inserted into plant material, the seed of a plant regenerated from said material produces carotenoids at a level of at least 65µg/g of endosperm of said seed. In a particular embodiment the amount of carotenoids is calcluated as µg/g of dry weight of endosperm of said seed.

The present invention still further provides a polynucleotide sequence which is the complement of one which hybridises to a polynucleotide which consists of a sequence selected from the group depicted as SEQ ID NOS: 1; 2; 3; 4; 5; and 6 at a temperature of about 65°C in a solution containing 6 x SSC, 0.01 % SDS and 0.25% skimmed milk powder, followed by rinsing at the same temperature in a solution containing 0.2 x SSC and 0.1 % SDS wherein said polynucleotide sequence still comprises a region encoding a carotene desaturase and a further region encoding a phytoene synthase and when said polynucleotide sequence is inserted into plant material the seed of a plant regenerated from said material produce carotenoids amounting to at least 80% of the carotenoid content of a seed which comprises a polynucleotide selected from the group depicted as SEQ ID NOS: 1; 2; 3; 4; 5 and 6. In a further embodiment, when said polynucleotide sequence is inserted into plant material, the seed of a plant regenerated from said material produce carotenoids amounting to at least 85% of the carotenoid content of a seed which comprises a polynucleotide selected from the group depicted as SEQ ID NOS: 1; 2; 3; 4; 5 and 6. In a still further embodiment when said polynucleotide sequence is inserted into plant material, the seed of a plant regenerated from said material produce carotenoids amounting to at least 90% of the carotenoid content of a seed which comprises a polynucleotide selected from the group depicted as SEQ ID NOS: 1; 2; 3; 4; 5; and 6. In a still further embodiment when said polynucleotide sequence is inserted into plant material, the seed of a plant regenerated from said material produce carotenoids amounting to at least 95% of the carotenoid content of a seed which comprises a polynucleotide selected from the group depicted as SEQ ID NOS: 1; 2; 3; 4; 5 and 6. In a still further embodiment when said polynucleotide sequence is inserted into plant material, the seed of a plant regenerated from said material produce carotenoids amounting to at least 100% of the carotenoid content of a seed which comprises a polynucleotide selected from the group depicted as SEQ ID NOS: 1; 2; 3; 4; 5 and 6. In a particular embodiment the polynucleotide sequence provides for a percentage of the carotenoid content of seed as described above wherein the seed with which the comparison is made comprises the polynucleotide depicted as SEQ ID NO: 1. In a particular embodiment the polynucleotide sequence provides for a percentage of the carotenoid content of seed as described above wherein the seed with which the comparison is made comprises the polynucleotide depicted as SEQ ID NO: 2. In a particular embodiment the polynucleotide sequence provides for a percentage of the carotenoid content of seed as described above wherein the seed with which the comparison is made comprises the polynucleotide depicted as SEQ ID NO: 3. In a particular embodiment the polynucleotide sequence provides for a percentage of the carotenoid content of seed as described above wherein the seed with which the comparison is made comprises the polynucleotide depicted as SEQ ID NO: 4. In a particular embodiment the polynucleotide sequence provides for a percentage of the carotenoid content of seed as described above wherein the seed with which the comparison is made comprises the polynucleotide depicted as SEQ ID NO: 6.

It is preferred that when the carotenoid content of the seed comprising said polynucleotide sequence is compared with the seed comprising the polynucleotide selected from the group depicted as SEQ ID NOS: 1; 2; 3; 4; 5 and 6, the seed are from plants of substantially the same species. It is further preferred that when the carotenoid content of the seed comprising said polynucleotide sequence is compared with the seed comprising the polynucleotide selected from the group depicted as SEQ ID NOS: 1; 2; 3; 4; 5 and 6, the seed are from plants which are substantially genetically identical save for the presence of said polynucleotide or said polynucleotide sequence. It is further preferred that when the carotenoid content of the seed comprising said polynucleotide sequence is compared with the seed comprising the polynucleotide selected from the group depicted as SEQ ID NOS: 1; 2; 3; 4; 5 and 6, the seed are from plants which are grown subject to the same environmental growing conditions.

The present invention still further provides a polynucleotide sequence which is the complement of one which hybridises to a polynucleotide which consists of a sequence selected from the group depicted as SEQ ID NOS: 7; 8; and 9 at a temperature of about 65°C in a solution containing 6 x SSC, 0.01% SDS and 0.25% skimmed milk powder, followed by rinsing at the same temperature in a solution containing 0.2 x SSC and 0.1% SDS wherein said polynucleotide sequence still comprises a region encoding a carotene desaturase and a further region encoding a phytoene synthase and when said polynucleotide sequence is inserted into plant material the seed of a plant regenerated from said material produce carotenoids amounting to at least 80% of the carotenoid content of a seed which comprises a polynucleotide selected from the group depicted as SEQ ID NOS: 7; 8 and 9. It is preferred that when the carotenoid content of the seed comprising said polynucleotide sequence is compared with the seed comprising the polynucleotide selected from the group depicted as SEQ ID NOS: 7; 8 and 9, the seed are from plants of substantially the same species. It is further preferred that when the carotenoid content of the seed comprising said polynucleotide sequence is compared with the seed comprising the polynucleotide selected from the group depicted as SEQ ID NOS: 7; 8 and 9, the seed are from plants which are substantially genetically identical save for the presence of said polynucleotide or said polynucleotide sequence.

In a particular embodiment the polynucleotide sequences according to the invention which are identified based on their hybridisation (under the conditions provided) to the sequences described in the Sequence Listing, encode the same proteins as those provided by the sequences in the Sequence Listing. In a further embodiment, said polynucleotide sequences may encode proteins which have the same or a similar function as the proteins encoded by the sequences in the Sequence Listing. In a still further embodiment, the proteins encoded by the polynucleotide sequence according to the invention comprise amino acid substitutions and/or deletions when compared to the porteins encoded by the sequences in the Sequence Listing. In a still further embodiment, said amino acid substitutions are "conservative" substitutions. A "conservative" substitution is understood to mean that the amino acid is replaced with an amino acid with broadly similar chemical properties. In particular "conservative" substitutions may be made between amino acids within the following groups: (i) Alanine and Glycine; (ii) Threonine and Serine; (ii) Glutamic acid and Aspartic acid; (iii) Arginine and Lysine; (iv) Asparagine and Glutamine; (v) Isoleucine and Leucine; (vi) Valine and Methionine; and (vii) Phenylalanine and Tryptophan.

The present invention still further provides a polynucleotide or a polynucleotide sequence as described above which when inserted into plant material, the seed of a plant regenerated from said material produces carotenoids at levels which are higher than those present in native like-seeds. The present invention still further provides a polynucleotide or a polynucleotide sequence as described above which when inserted into plant material, the seed of a plant regenerated from said material produces carotenoids at levels which are higher than those present in untransformed like-seeds.

In a particular embodiment, the carotenoids which are increased are selected from the group consisting of: lycopene; alpha-carotene; lutein; beta-carotene; zeaxanthin; beta-cryptoxanthin; antheraxanthin; violaxanthin; and neoxanthin or a combination thereof In a further embodiment, the carotenoids which are increased are selected from the group consisting of: lycopene; alpha-carotene; lutein; beta-carotene; zeaxanthin; beta-cryptoxanthin; or a combination thereof. In a still further embodiment, the carotenoids which are increased are selected from the group consisting of: alpha-carotene; lutein; beta-carotene; zeaxanthin; beta-cryptoxanthin; or a combination thereof In a still further embodiment, the carotenoids which are increased include at least phytoene and beta-carotene. In a still further embodiment, the carotenoids which are increased include at least beta-carotene. In a still further embodiment, the carotenoid which is increased is beta-carotene. In a still further embodiment the carotenoids which are increased are coloured carotenoids.

The present invention still further provides a polynucleotide or a polynucleotide sequence as described above wherein said seed is a rice seed. In a particular embodiment of the invention, before the seeds are analysed for their carotenoid content, the seeds are prepared prior to the analysis. Such preparation may include, for example, with respect to rice seed, "dehusking" and "polishing". Furthermore, such preparation may involve the removal of those plant parts associated with the seed which plant parts are not normally intended for human consumption.

The amount of carotenoids in the seeds can be determined using techniques which are well known and available to the person skilled in the art. Such techniques include but are not necessarily limited to High Performance Liquid Chromatography (HPLC) analysis and spectrophotometry.

The present invention still further provides a polynucleotide or polynucleotide sequence as described above which further comprises a region which encodes a selectable marker. In a particular embodiment said selectable marker comprises a mannose-6-phosphate isomerase gene. In a further particular embodiment the selectable marker used is the mannose-6-phosphate isomerase gene according to the Positech^{™} selection system. In a specific embodiment said selectable marker is the one as described in European Patent/Application publication Number EP 0 896 063 and EP 0 601 092. Alternatively, the selectable marker used may, in particular, confer resistance to kanamycin, hygromycin or gentamycin. Further suitable selectable markers include genes that confer resistance to herbicides such as glyphosate-based herbicides (e.g. EPSPS genes such as in USP 5510471 or WO 00/66748) or resistance to toxins such as eutypine. Other forms of selection are also available such as hormone based selection systems such as the Multi Auto Transformation (MAT) system of Hiroyrasu Ebinuma et al. 1997. PNAS Vol. 94 pp2117-2121; visual selection systems which use fluorescent proteins, β glucoronidase and any other selection system such as xylose isomerase and 2-deoxyglucose (2-DOG).

The present invention still further provides a polynucleotide or a polynucleotide sequence according to the invention which is codon optimised for expression in a particular plant species. In a particular embodiment the polynucleotide or polynucleotide sequence is codon optimised for expression in rice (*Orzya sp.)* or maize *(Zea sp.).* Such codon optimisation is well known to the person skilled in the art and the table below provides an example of the plant-preferred codons for rice and maize.

| **Amino Acid** | **Rice preference** | **Maize preference** |
|---|---|---|
| Alanine | GCC | GCC |
| Arginine | CGC | AGG |
| Asparagine | AAC | ACC |
| Aspartic Acid | GAC | GAC |
| Cysteine | TGC | TGC |
| Glutamine | CAG | CAG |
| Glutamic Acid | GAG | GAG |
| Glycine | GGC | GGC |
| Histidine | CAC | CAC |
| Isoleucine | ATC | ATC |
| Leucine | CTC | CTG |
| Lysine | AAG | AAG |
| Methionine | ATG | ATG |
| Phenylalanine | TTC | TTC |
| Proline | CCG | CCG |
| Serine | TCC | AGC |
| Threonine | ACC | ACC |
| Tryptophan | TGG | TGG |
| Tyrosine | TAC | TAC |
| Valine | GTG | GTG |

The present invention further provides a vector comprising a polynucleotide or a polynucleotide sequence as described above. In a particular embodiment of the invention said vector comprises a polynucleotide selected from the group depicted as SEQ ID NO: 1; 2; 3; 4; 5 and 6. In a particular embodiment of the invention said vector comprises a polynucleotide selected from the group depicted as SEQ ID NOS: 7; 8 and 9. In a particular embodiment said vector allows for replication of said polynucleotide or polynucleotide sequence in a bacterium.

The present invention still further provides a vector as described above which is a plant expression vector.

In a particular embodiment of the invention the sequence around the translational start position(s) of the phytoene synthase and/or said carotene desaturase encoding sequences as described above may be modified such that it is "Kozak" preferred. What is meant by this is well known to the skilled artisan. Examples of Kozak consensus sequences which are well known to the person skilled in the art include cagcc(atg) or agcc(atg). The phytoene synthase and/or said carotene desaturase encoding sequences as described above may also further comprise a sequence which provides for retention in a particular intracellular organelle.

In a further aspect of the present invention there is provided a method for increasing the carotenoid content of seeds comprising inserting into plant material a polynucleotide or a polynucleotide sequence or a vector as described above; and regenerating a seed-containing plant from said material and identifying the seeds which contain carotenoids at levels greater that those of a control like-seed.

The present invention still further provides a method for increasing the carotenoid content of seeds comprising inserting into plant material a polynucleotide comprising a sequence selected from the group depicted as SEQ ID NO: 1; 2; 3; 4; 5 and 6 and regenerating a seed containing plant from said material and identifying the seeds which contain carotenoids at levels greater that those of control like-seeds. In a particular embodiment of the invention, the seeds obtained by said method contain at least a sixty fold increase in carotenoids when compared to a control like-seed. In a further embodiment the seeds obtained by said method contain at least a one hundred fold increase in carotenoids when compared to control like-seeds. In a further embodiment the seeds obtained by said method contain at least a one hundred and fifty fold increase in carotenoids when compared to control like-seeds. In a further embodiment the seeds obtained by said method contain at least a two hundred fold increase in carotenoids when compared to control like-seeds. In a further embodiment the seeds obtained by said method contain at least a two hundred and fifty fold increase in carotenoids when compared to control like-seeds. In a further embodiment the seeds obtained by said method contain at least a three hundred fold increase in carotenoids when compared to control like-seeds. In a further embodiment the seeds obtained by said method contain at least a three hundred and fifty fold increase in carotenoids when compared to control like-seeds. In a further embodiment the seeds obtained by said method contain at least a four hundred fold increase in carotenoids when compared to control like-seeds. In a further embodiment the seeds obtained by said method contain at least a five hundred fold increase in carotenoids when compared to control like-seeds.

The present invention still further provides a method as described above wherein said seed contains carotenoids at a level of at least 3µg/g of endosperm of said seed. In a particular embodiment said seed contains carotenoids at a level of at least 4µg/g of endosperm of said seed. In a particular embodiment said seed contains carotenoids at a level of at least 5µg/g of endosperm of said seed. In a particular embodiment said seed contains carotenoids at a level of at least 6µg/g of endosperm of said seed. In a particular embodiment said seed contains carotenoids at a level of at least 7µg/g of endosperm of said seed. In a particular embodiment said seed contains carotenoids at a level of at least 8µg/g of endosperm of said seed. In a particular embodiment said seed contains carotenoids at a level of at least 9µg/g of endosperm of said seed. In a particular embodiment said seed contains carotenoids at a level of at least 10µg/g of endosperm of said seed. In a particular embodiment said seed contains carotenoids at a level of at least 15µg/g of endosperm of said seed. In a particular embodiment said seed contains carotenoids at a level of at least 20µg/g of endosperm of said seed. In a particular embodiment said seed contains carotenoids at a level of at least 25µg/g of endosperm of said seed. In a particular embodiment said seed contains carotenoids at a level of at least 30µg/g of endosperm of said seed. In a particular embodiment said seed contains carotenoids at a level of at least 35µg/g of endosperm of said seed. In a particular embodiment said seed contains carotenoids at a level of at least 40µg/g of endosperm of said seed. In a particular embodiment said seed contains carotenoids at a level of at least 45µg/g of endosperm of said seed. In a particular embodiment said seed contains carotenoids at a level of at least 50µg/g of endosperm of said seed. In a particular embodiment said seed contains carotenoids at a level of at least 55µg/g of endosperm of said seed. In a particular embodiment said seed contains carotenoids at a level of at least 60µg/g of endosperm of said seed. In a particular embodiment said seed contains carotenoids at a level of at least 65µg/g of endosperm of said seed.

The present invention still further provides a method for increasing the carotenoid content of seeds comprising inserting into plant material a polynucleotide sequence as described above and regenerating a seed-containing plant from said material and identifying the seed which contains carotenoids amounting to at least 80% of the carotenoid content of a seed which comprises a polynucleotide selected from the group depicted as SEQ ID NOS: 1; 2; 3; 4; 5 and 6. In a further embodiment, the seed of a plant regenerated from said material produce carotenoids amounting to at least 85% of the carotenoid content of a seed which comprises a polynucleotide selected from the group depicted as SEQ ID NOS: 1; 2; 3; 4; 5 and 6. In a still further embodiment, the seed of a plant regenerated from said material produce carotenoids amounting to at least 90% of the carotenoid content of a seed which comprises a polynucleotide selected from the group depicted as SEQ ID NOS: 1; 2; 3; 4; 5 and 6. In a still further embodiment, the seed of a plant regenerated from said material produce carotenoids amounting to at least 95% of the carotenoid content of a seed which comprises a polynucleotide selected from the group depicted as SEQ ID NOS: 1; 2; 3; 4; 5 and 6. In a still further embodiment, the seed of a plant regenerated from said material produce carotenoids amounting to at least 100% of the carotenoid content of a seed which comprises a polynucleotide selected from the group depicted as SEQ ID NOS: 1; 2; 3; 4; 5 and 6. In a particular embodiment the polynucleotide sequence provides for a percentage of the carotenoid content of seed as described above wherein the seed with which the comparison is made comprises the polynucleotide depicted as SEQ ID NO: 1. In a particular embodiment the polynucleotide sequence provides for a percentage of the carotenoid content of seed as described above wherein the seed with which the comparison is made comprises the polynucleotide depicted as SEQ ID NO: 2. In a particular embodiment the polynucleotide sequence provides for a percentage of the carotenoid content of seed as described above wherein the seed with which the comparison is made comprises the polynucleotide depicted as SEQ ID NO: 3. In a particular embodiment the polynucleotide sequence provides for a percentage of the carotenoid content of seed as described above wherein the seed with which the comparison is made comprises the polynucleotide depicted as SEQ ID NO: 4. In a particular embodiment the polynucleotide sequence provides for a percentage of the carotenoid content of seed as described above wherein the seed with which the comparison is made comprises the polynucleotide depicted as SEQ ID NO: 6.

The present invention still further provides a method for increasing the carotenoid content of seeds comprising inserting into plant material a polynucleotide comprising a sequence selected from the group depicted as SEQ ID NOS: 7; 8 and 9 and regenerating a seed containing plant from said material and identifying the seeds which contain carotenoids at levels greater that those of control like-seeds. In a particular embodiment of the invention, the seeds obtained by said method contain at least a fifty fold increase in carotenoids when compared to a control like-seed.

The present invention still further provides a method as described above wherein said seed contains carotenoids at a level of at least 3µg/g of endosperm of said seed.

The present invention still further provides a method for increasing the carotenoid content of seeds comprising inserting into plant material a polynucleotide sequence as described above and regenerating a seed-containing plant from said material and identifying the seed which contains carotenoids amounting to at least 80% of the carotenoid content of a seed which comprises a polynucleotide selected from the group depicted as SEQ ID NOS: 7; 8 and 9.

The present invention still further provides a method as described above wherein the carotenoids which are increased are selected from the group consisting of: lycopene; alpha-carotene; lutein; beta-carotene; zeaxanthin; beta-cryptoxanthin; antheraxanthin; violaxanthin; and neoxanthin or a combination thereof. In a further embodiment, the carotenoids which are increased are selected from the group consisting of: lycopene; alpha-carotene; lutein; beta-carotene; zeaxanthin; beta-cryptoxanthin; or a combination thereof. In a still further embodiment, the carotenoids which are increased are selected from the group consisting of: alpha-carotene; lutein; beta-carotene; zeaxanthin; beta-cryptoxanthin; or a combination thereof. In a still further embodiment, the carotenoids which are increased include at least phytoene and beta-carotene. In a still further embodiment, the carotenoids which are increased include at least beta-carotene. In a still further embodiment, the carotenoid which is increased is beta-carotene. In a still further embodiment the carotenoids which are increased are coloured carotenoids.

The polynucleotide or polynucleotide sequence or vector as described above may be inserted into plant material by plant transformation techniques that are well known to the person skilled in the art. Such techniques include but are not limited to particle mediated biolistic transformation, *Agrobacterium-*mediated transformation, protoplast transformation (optionally in the presence of polyethylene glycols); sonication of plant tissues, cells or protoplasts in a medium comprising the polynucleotide or vector; micro-insertion of the polynucleotide or vector into totipotent plant material (optionally employing the known silicon carbide "whiskers" technique), electroporation and the like. In a particular embodiment of the invention rice plant material is transformed in accordance with the methods described in the Examples disclosed herein. In a particular embodiment the *Agrobacterium* that is used is a strain that has been modified to reduce the possibility of recombination between sequences having a high degree of similarity within the TDNA region of the *Agrobacterium.* Furthermore, techniques and elements such as those referred to in WO99/01563, US6,265,638, US5,731,179 and US5,591,616 may be employed as part of the transformation process.

The present invention still further provides seed obtained or obtainable by a method as described above. In a specific embodiment said seed are rice seed. In a further embodiment said seeds are maize seeds.

Througout this specification the terms "seed" and "seeds" may be interchanged with the terms "grain" or "grains". In particular, the terms "seed" and "seeds" refers to edible seeds or seed parts, in particular, seed endosperm.

The present invention still further comprises a plant which comprises a seed according to the preceding paragraph.

The present invention further provides a plant or plant material which comprises a polynucleotide or a polynucleotide sequence or a vector as described above. In a particular embodiment said plant or plant material is a rice plant or rice plant material or a maize plant or maize plant material. In a still further embodiment the plant or plant material of the present invention is selected from the group consisting watermelon, melon, mango, soybean, cotton, tobacco, sugar beet, oilseed rape, canola, flax, sunflower, potato, tomato, alfalfa, lettuce, maize, wheat, sorghum, rye, bananas, barley, oat, turf grass, forage grass, sugar cane, pepper, pea, field bean, rice, pine, poplar, apple, peach, grape, strawberry, carrot, cabbage, onion, citrus, cereal or nut plants or any other horticultural crops. In a specific embodiment said plant is a rice plant.

The present invention still further provides a plant or seed according to the invention which further comprises a gene which gene encodes an enzyme which is capable of converting carotene to a retinoid. An example of such a gene is the gene encoding β-carotene dioxygenase as described in WO01/48162 and/or WO01/48163.

The present invention still further provides a plant according to the invention which further comprises a polynucleotide which provides for a trait selected from the group consisting of: insect resistance and/or tolerance; nematode resistance and/or tolerance; herbicide resistance and/or tolerance; improved resistance and/or tolerance to stress; a substance having pharmaceutical activity and/or any other desired agronomic trait.

The present invention still further provides a plant according to the invention which further comprises a polynucleotide which provides for a further enhancement of isoprenoid biosynthesis and/or carotenoid accumulation in the plant. In a particular embodiment said polynucleotide provides for a transcription factor which provides for a further enhancement of isoprenoid biosynthesis and/or carotenoid accumulation in the plant.

The present invention still further provides a plant according to the invention which further comprises a polynucleotide which provides for an increase in plastids within the plant. In a particular embodiment said polynucleotide comprises the PhyA gene from oats or arabidopsis which genes are well known to th person skilled in the art. In a further embodiment said polynucleotide comprises the Hp1or Hp2 gene from tomato which are also well known the the person skilled in the art.

The present invention still further provides a molecular marker which marker is capable of identifying plant material which comprises a sequence selected from the group depitected as SEQ ID NOS: 1 to 9 wherein said molecular marker comprises at least about 25 contigous nucleotides of a sequence selected from the group consisting of SEQ ID NOS: 1 to 9.

The present invention still further provides a method for identifying plant material according to the invention via the use, for example, of the polymerase chain reaction (PCR). Suitable primers may be designed using parameters well known to those skilled in the art and based on the sequences listed in the Sequence Listing. The person skilled in the art is well versed in nucleic acid extraction techniques, and once a test sample has been isolated, it can be analysed for the presence of the sequence according to the invention using techniques that are well known in the art. These include, but are not limited to, PCR, RAPIDS, RFLPs and AFLPs.

The present invention still further provides a kit which kit comprises a means for obtaining a test sample and a means for detecting the presence of the sequences of the invention within said test sample. Kits may also be generated that are suitable for testing the carotenoid content of a test sample and this may optionally be combined with the features of a kit as described in the preceding sentence.

In a further aspect of the present invention there is provided the use of a polynucleotide, polynucleotide sequence or a vector as described above in a method for the production of seeds containing increased carotenoids. In a particular embodiment the present invention provides the use of a polynucleotide selected from the group depicted as SEQ ID NOS: 1; 2; 3; 4; 5 and 6 for the production of seeds which contain carotenoids at levels greater that those of a control like-seed.

In a further aspect of the present invention there is provided the use of a polynucleotide, polynucleotide sequence or a vector as described above in a method for the production of seeds containing increased carotenoids. In a particular embodiment the present invention provides the use of a polynucleotide selected from the group depicted as SEQ ID NOS: 7; 8 and 9 for the production of seeds which contain carotenoids at levels greater that those of a control like-seed.

In a further aspect of the invention there is provided the use of a polynucleotide, polynucleotide sequence or a vector as described above in a method of producing a plant which comprises said polynucleotide, said polynucleotide sequence or said vector.

In a further aspect of the invention there is provided the use of a polynucleotide selected from the group depicted as SEQ ID NOS: 1; 2; 3; 4; 5 and 6 in a method for the production of a plant comprising said polynucleotide.

In a further aspect of the invention there is provided the use of a polynucleotide selected from the group depicted as SEQ ID NOS: 7; 8 and 9 in a method for the production of a plant comprising said polynucleotide.

In a further aspect of the invention there is provided a method for increasing the carotenoid content of seeds comprising inserting into plant material (a) a first polynucleotide which comprises as operably linked components (i) a promoter which provides for seed preferred expression; and (ii) a nucleotide sequence derived from a bacterium which sequence encodes a carotene desaturase; and (iii) a transcription termination region; and (b) a second polynucleotide which comprises as operably linked components (i) a promoter which provides for seed preferred expression; and (ii) a nucleotide sequence encoding a phytoene synthase which sequence is derived from maize *(Zea sp.)* or rice (*Orzya sp.);* and (iii) a transcription termination region; and (c) regenerating a seed containing plant from said material and identifying the seeds which contain carotenoids at levels greater that those of control like-seeds.

Further disclosed is a method for increasing the carotenoid content of seeds comprising inserting into plant material (a) a first polynucleotide which comprises as operably linked components (i) a promoter which provides for seed preferred expression; and (ii) a nucleotide sequence derived from a bacterium which sequence encodes a carotene desaturase; and (iii) a transcription termination region; and (b) a second polynucleotide which comprises as operably linked components (i) a promoter which provides for seed preferred expression; and (ii) a nucleotide sequence encoding a phytoene synthase which sequence is derived from tomato (*Lycopersicon sp.*) or pepper *(Capsicum sp.*); or a bacterium; and (iii) a transcription termination region; and (c) regenerating a seed containing plant from said material and identifying the seeds which contain carotenoids at levels greater that those of control like-seeds.

In a particular embodiment, step (a) of the preceding paragraph is performed prior to step (b). In a further embodiment, step (b) of the preceding paragraph is performed prior to step (a). In a still further embodiment, the promoter, the sequence encoding said carotene desaturase, the sequence encoding phytoene synthase and the terminator region are derived from the sequences depicted in the Sequence Listing. In a still further embodiment said carotene desaturase is the CrtI gene from *Erwinia sp.* In a still further embodiment the carotene desaturase comprises or consists of the sequence depicted as SEQ ID NO: 18 or 19. In a further embodiment said phytoene synthase is derived from maize or rice. In a still further embodiment said phytoene synthase is from maize or rice. In a still further embodiment said phytoene synthase comprises or consists of a sequence selected from the group depicted as SEQ ID NOS: 10; 11; 12; and 13.

In a further aspect of the invention there is provided a method for increasing the carotenoid content of seeds comprising crossing (a) a first plant comprising a polynucleotide which comprises as operably linked components (i) a promoter which provides for seed preferred expression; and (ii) a nucleotide sequence derived from a bacterium which sequence encodes a carotene desaturase; and (iii) a transcription termination region; with (b) a further plant comprising a polynucleotide which comprises as operably linked components (i) a promoter which provides for seed preferred expression; and (ii) a nucleotide sequence encoding a phytoene synthase which sequence is derived from maize (*Zea sp.*) or rice (*Orzya sp.);* and (iii) a transcription termination region; and (c) harvesting seed from the female parent of the thus crossed plants; and (d) growing said seed to produce plants comprising further seeds and identifying said further seeds which contain carotenoids at levels greater that those of control like-seeds.

Further disclosed is a method for increasing the carotenoid content of seeds comprising crossing (a) a first plant comprising a polynucleotide which comprises as operably linked components (i) a promoter which provides for seed preferred expression; and (ii) a nucleotide sequence derived from a bacterium which sequence encodes a carotene desaturase; and (iii) a transcription termination region; with (b) a further plant comprising a polynucleotide which comprises as operably linked components (i) a promoter which provides for seed preferred expression; and (ii) a nucleotide sequence encoding a phytoene synthase which sequence is derived from tomato *(Lycopersicon sp.*) or pepper *(Capsicum sp.*); or a bacterium; and (iii) a transcription termination region; and (c) harvesting seed from the female parent of the thus crossed plants; and (d) growing said seed to produce plants comprising further seeds and identifying said further seeds which contain carotenoids at levels greater that those of control like-seeds.

In a still further embodiment, the promoter, the sequence encoding said carotene desaturase, the sequence encoding phytoene synthase and the terminator region are obtainable from the sequences depicted in the Sequence Listing. In a still further embodiment said carotene desaturase is the CrtI gene from *Erwinia sp.* In a still further embodiment the carotene desaturase comprises or consists of the sequence depicted as SEQ ID NO: 18 or 19. In a further embodiment said phytoene synthase is derived from maize or rice. In a still further embodiment said phytoene synthase is from maize or rice. In a still further embodiment said phytoene synthase comprises or consists of a sequence selected from the group depicted as SEQ ID NOS: 10; 11; 12 and 13.

In a further aspect of the invention there is provided a polynucleotide which comprises (a) a region which comprises as operably linked components (i) a promoter which provides for seed preferred expression; and (ii) a nucleotide sequence derived from a bacterium which sequence encodes a carotene desaturase or a nucleotide sequence which encodes a carotene desaturase derived from a plant selected from the group consisting of: tomato *(Lycopersicon sp.);* pepper *(Capsicum sp.);* maize *(Zea sp.);* rice *(Orzya sp.);* and (iii) a transcription termination region; and (b) a further region which comprises as operably linked components (i) a promoter which provides for seed preferred expression; and (ii) a nucleotide sequence encoding a phytoene synthase which sequence is derived from a bacterium, or from a plant selected from the group consisting of maize (*Zea sp*.) and rice *(Orzya sp.);* and (iii) a transcription termination region; and (c) a still further region which comprises as operably linked components (i) a promoter which provides for seed preferred expression; and (ii) a nucleotide sequence encoding a zeta-carotene desaturase (ZDS) derived from a bacterium, or from a plant selected from the group consisting of: tomato (*Lycopersicon sp.);* pepper (*Capsicum sp.);* maize (*Zea sp.);* rice (*Orzya sp.);* and (iii) a transcription termination region. In a particular embodiment the carotene desaturase and phytoene synthase is derived from maize *(Zea sp.)* and and zeta-carotene desaturase (ZDS) is derived from pepper *(Capsicum sp.).* In a further embodiment the carotene desaturase and phytoene synthase is from maize *(Zea sp.)* and the zeta-carotene desaturase (ZDS) is from pepper *(Capsicum sp.).*

The polynucleotides as described above may be used to identify polynucleotide sequences providing for a like-function, based on the hybrisation conditions described above. These polynucleotides and polynucleotide sequences which comprise the zeta-carotene desaturase may also be used in methods for increasing the carotenoid content of seeds in a manner analogous to the methods described above.

In a further aspect of the invention there is provided a polynucleotide which comprises as operably linked components (i) a promoter which provides for seed preferred expression; and (ii) a nucleotide sequence derived from a bacterium which sequence encodes a carotene desaturase or a nucleotide sequence which encodes a carotene desaturase derived from a plant selected from the group consisting of: tomato (*Lycopersicon sp.);* pepper (*Capsicum sp.*); maize (*Zea sp.*); rice (*Orzya sp.*); and (iii) a nucleotide sequence encoding a phytoene synthase which sequence is derived from a plant selected from the group consisting of: maize (*Zea sp*.) and rice (*Orzya sp.*) and (iv) a nucleotide sequence encoding a zeta-carotene desaturase (ZDS) derived from a bacterium or from a plant selected from the group consisting of: tomato (*Lycopersicon sp.*); pepper *(Capsicum sp.);* maize (*Zea sp.);* rice (*Orzya sp.);* and (v) a transcription termination region.

Any of the regions described in this specification may be separated by a region which provides for a self-processing polypeptide which is capable of separating the proteins such as the self-processing polypeptide described in USS,846,767 or any similarly functioning element. Alternatively the regions may be separated by a sequence which acts as a target site for an external element which is capable of separating the protein sequences. Alternatively the polynucleotide may provide for a polyprotein which comprises a plurality of protein functions. In a further embodiment of the present invention the proteins of the polyprotein may be arranged in tandem. The person skilled in the art will appreciate that when a polynucleotide is generated which encodes such a polyprotein, expression of such a polyprotein may be achieved via the use of a single promoter which promoter is described herein.

All of the polynucleotides and polynucleotide sequences described throughout this specification can be isolated and constructed using techniques that are well known to the person skilled in the art. For example, the polynucleotides can be synthesised using standard polynucleotide synthesisers. Such synthetic polynucleotides can be synthesised and then ligated to form the longer polynucleotides according to the invention. The polynucleotides and polynucleotide sequences can also be isolated from other constructs/vectors which contain said sequences and then be inserted into further constructs/vectors to produce the ones according to the invention. The sequences can also be isolated from libraries, for example cDNA and gDNA, using the sequence information provided in the Sequence Listing for the creation of suitable probes/primers for the purpose of identifying said sequences from said libraries. Once isolated, the sequences can be assembled to create the polynucleotides and polynucleotide sequences according to the invention. The sequences according to the invention can also be used to identify like-sequences in accordance with the hybridisation conditions described above. In identifying such like-sequences the person skilled in the art may wish to identify like-sequences of one or more of the component parts of the sequences depicted in the Sequence Listing and then subsequently assemble the like-sequences in a manner similar to the arrangement of the sequences depicted in the Sequence Listing. For example, it may be desired to modify the region encoding phytoene synthase gene only. Once the modified phytoene synthase encoding sequence had been identified, it could be used to replace the phytoene synthase encoding sequence in one of the sequences depicted in the Sequence Listing. Alternatively, the modified phytoene synthase may be used in the creation of a new sequence wherein all the component parts are the same as a sequence in the Sequence Listing save for the phytoene synthase. In a further example, all of the components may be modified and then each of the modified components is arranged in the same manner as the sequences of the Sequence Listing, for example, promoter-intron-target sequence-carotene desaturase-terminator- promoter-intron-(target sequence) - phytoene synthase-terminator. The degree of modification will affect the ability of the thus modified sequence to hybridise to a sequence in the Sequence Listing under the conditions described above.

The present invention further provides a plant which comprises a polynucleotide or polynucleotide sequence as described above. In a particular embodiment said plant is a rice or a maize plant.

In a further aspect of the present invention there is provided a polynucleotide or a polynucleotide sequence as described above wherein the promoter(s) are tissue preferred and/or organ preferred. In a particular embodiment the promoter(s) provide for prefferential expression in fruit. In a further embodiment said promoter(s) provide for high expression in fruit. In a still further embodiment said fruit is a banana fruit. In a further aspect of the present invention there is provided a polynucleotide comprising:
(a) a region which comprises as operably linked components (i) a promoter which provides for fruit preferred expression; and (ii) a nucleotide sequence derived from a bacterium which sequence encodes a carotene desaturase; and (iii) a transcription termination region; and (b) a further region which comprises as operably linked components (i) a promoter which provides for fruit preferred expression; and (ii) a nucleotide sequence encoding a phytoene synthase which sequence is derived from maize (*Zea sp.)* or rice (*Orzya sp.);* and (iii) a transcription termination region. In a still further embodiment there is provided a method for increasing the carotenoid content of fruit comprising inserting into plant material a polynucleotide comprising:
   (a) a region which comprises as operably linked components (i) a promoter which provides for fruit preferred expression; and (ii) a nucleotide sequence derived from a bacterium which sequence encodes a carotene desaturase; and (iii) a transcription termination region; and (b) a further region which comprises as operably linked components (i) a promoter which provides for fruit preferred expression; and (ii) a nucleotide sequence encoding a phytoene synthase which sequence is derived from maize (*Zea sp.)* or rice (*Orzya sp.);* and (iii) a transcription termination region and regenerating a fruit-containing plant from said material and identifying the fruit which contain carotenoids at levels greater than those of control like-fruit. The present invention still further provides polynucleotides which comprise the phytoene synthase encoding sequences and carotene desaturase encoding sequences mentioned above which sequences are operably linked to promoters which provide for fruit preferred or tissue or organ preferred expression. Such suitable promoters may be identified by the person skilled in the art.

In a further aspect of the present invention there is provided the use of a polynucleotide or a polynucleotide sequence as described above in a method for the production of plants which are resistant and/or tolerant to a herbicide.

In a still further aspect of the present invention there is provided a method for the production of a plant that is resistant and/or tolerant to a herbicide comprising inserting into plant material a polynucleotide or a polynucleotide sequence as described above and regenerating a morphologically normal plant from said material. The herbicide resistance and/or tolerance of the plant containing the polynucleotide or polynucleotide sequence of the invention can be compared to a control like-plant. The term control like-plant relates to plants which are substantially similar to those according to the invention but which control like-plant does not contain the polynucleotides or polynucleotide sequences according to the invention. Typically, a control like-plant will comprise a plant of the same or similar plant species which control like-plant is a native plant or which has not been transformed.

In a further aspect of the present invention there is provided a polynucleotide comprising the sequence depicted as SEQ ID NO: 13. In a particular embodiment there is provided a polynucelotide which consists of the sequence depicted as SEQ ID NO: 13. The present invention still further provides a polynucleotide which encodes the protein depicted as SEQ ID NO: 14. The present invention still further provides a polynucleotide sequence which has at least 87% identity to the sequence depicted as SEQ ID NO: 13 wherein said sequence still encodes a phytoene synthase. The present invention still further provides a polynucleotide sequence which has at least 90% identity to the sequence depicted as SEQ ID NO: 13 wherein said sequence still encodes a phytoene synthase. The present invention still further provides a polynucleotide sequence which has at least 91% identity to the sequence depicted as SEQ ID NO: 13 wherein said sequence still encodes a phytoene synthase. The present invention still further provides a polynucleotide sequence which has at least 92% identity to the sequence depicted as SEQ ID NO: 13 wherein said sequence still encodes a phytoene synthase. The present invention still further provides a polynucleotide sequence which has at least 93% identity to the sequence depicted as SEQ ID NO: 13 wherein said sequence still encodes a phytoene synthase. The present invention still further provides a polynucleotide sequence which has at least 94% identity to the sequence depicted as SEQ ID NO: 13 wherein said sequence still encodes a phytoene synthase. The present invention still further provides a polynucleotide sequence which has at least 95% identity to the sequence depicted as SEQ ID NO: 13 wherein said sequence still encodes a phytoene synthase. The present invention still further provides a polynucleotide sequence which has at least 96% identity to the sequence depicted as SEQ ID NO: 13 wherein said sequence still encodes a phytoene synthase. The present invention still further provides a polynucleotide sequence which has at least 97% identity to the sequence depicted as SEQ ID NO: 13 wherein said sequence still encodes a phytoene synthase. The present invention still further provides a polynucleotide sequence which has at least 98% identity to the sequence depicted as SEQ ID NO: 13 wherein said sequence still encodes a phytoene synthase. The present invention still further provides a polynucleotide sequence which has at least 99% identity to the sequence depicted as SEQ ID NO: 13 wherein said sequence still encodes a phytoene synthase.

The present invention still further provides a protein having the sequence deipicted as SEQ ID NO: 14 or a variant which has at least 82% identity to SEQ ID NO: 14 wherein said variant still provides for phytoene synthase activity. In a further embodiment said variant has at least 85% identity to SEQ ID NO: 14 wherein said variant still provides for phytoene synthase activity. In a still further embodiment said variant has at least 90% identity to SEQ ID NO: 14 wherein said variant still provides for phytoene synthase activity. In a still further embodiment said variant has at least 91 % identity to SEQ ID NO: 14 wherein said variant still provides for phytoene synthase activity. In a still further embodiment said variant has at least 92% identity to SEQ ID NO: 14 wherein said variant still provides for phytoene synthase activity. In a still further embodiment said variant has at least 93% identity to SEQ ID NO: 14 wherein said variant still provides for phytoene synthase activity. In a still further embodiment said variant has at least 94% identity to SEQ ID NO: 14 wherein said variant still provides for phytoene synthase activity. In a still further embodiment said variant has at least 95% identity to SEQ ID NO: 14 wherein said variant still provides for phytoene synthase activity. In a still further embodiment said variant has at least 96% identity to SEQ ID NO: 14 wherein said variant still provides for phytoene synthase activity. In a still further embodiment said variant has at least 97% identity to SEQ ID NO: 14 wherein said variant still provides for phytoene synthase activity. In a still further embodiment said variant has at least 98% identity to SEQ ID NO: 14 wherein said variant still provides for phytoene synthase activity. In a still further embodiment said variant has at least 99% identity to SEQ ID NO: 14 wherein said variant still provides for phytoene synthase activity. In a still further embodiment there is provided a polynucleotide which encodes said variant. By phytoene synthase activity it is meant that the variant protein has the same or a similar function to the protein depicted as SEQ ID NO: 14. The percentage of sequence identity for proteins is determined by comparing two optimally aligned sequences over a comparison window, wherein the portion of the amino acid sequence in the comparison window may comprise additions or deletions (i.e. gaps) as compared to the initial reference sequence (which does not comprise additions or deletions) for optimal alignment of the two sequences. The percentage is calculated by determining the number of positions at which the identical amino acid residue occurs in both sequences to yield the number of match positions, dividing the number of match positions by the total number of positions in the window of comparison and multiplying the result by 100 to yield the percentage of sequence identity. Optimal alignment of sequences for comparison may also be conducted by computerised implementations of known algorithms such as Altschul, Stephen F., Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402. There are also algorithms available to the person skilled in the art that enable a calculation of the percentage sequence identity between polynucleotide sequences. The variant may differ from the protein depicted as SEQ ID NO: 14 in particular by conservative substitutions. Such conservative substitutions are described above.

The present invention will now be described by way of the following nonlimiting examples with reference to the following Figures and Sequence Listing of which:
SEQ ID NO: 1 = 12423 = Glu-Cat-SSU-crtI-Nos-Glu-Cat-Psy (Maize-gb)-nos
SEQ ID NO: 2 = 12421 = Glu-Cat-SSU-crtI-Nos-Glu-Cat-Psy (Maize-E1B)-nos
SEQ ID NO: 3 = 12422 = Glu-SSU-crtI-Nos-Glu-Psy (Maize-E1B)-nos
SEQ ID NO: 4 = 12424 = Glu-SSU-crtI-Nos-Glu-Psy (Maize-gb)-nos
SEQ ID NO: 5 = Glu-Cat-SSU-crtI-Nos-Glu-Cat-Psy (Maize)-nos
SEQ ID NO: 6 =11586 = Glu-Cat-SSU-crtI-Nos-Glu-Cat-Psy (Rice)-nos
SEQ ID NO: 7 = 7651= Glu-Cat-SSU-crtI-Nos-Glu-Cat-Psy (Pepper)-nos
SEQ ID NO: 8 = 7650= Glu-Cat-SSU-crtI-Nos-Glu-Cat-Psy (Tomato)-nos
SEQ ID NO: 9 = Glu-Cat-SSU-crtI-Nos-Glu-Cat-SSU-Psy (crtB)-nos
SEQ ID NO: 10 = Phytoene synthase gb (Maize)
SEQ ID NO: 11 = Phytoene synthase (Maize) from SEQ ID NO 5 above
SEQ ID NO: 12 = Phytoene synthase E1B (Maize)
SEQ ID NO: 13 = Phytoene synthase (Rice)
SEQ ID NO: 14 = Phytoene synthase (Rice) PROTEIN
SEQ ID NO: 15 = Phytoene synthase (Pepper)
SEQ ID NO: 16 = Phytoene synthase (Tomato)
SEQ ID NO: 17 = Phytoene synthase (*Erwinia* crtB)
SEQ ID NO: 18 = Carotene desaturase (*Erwinia* crtI) used in SEQ ID NOS: 1-4
SEQ ID NO: 19 = Carotene desaturase (*Erwinia* crtI)
SEQ ID NO: 20 = Glutelin seed preferred promoter
SEQ ID NO: 21 = Prolamin seed preferred promoter
SEQ ID NO: 22 = Intron from catalase gene
SEQ ID NO: 23 = Transit peptide (Small sub-unit Rubisco)
SEQ ID NO: 24 = Transcription termination region from nopaline synthase gene
SEQ ID NO: 25 = Transcription termination region from 35S CaMV
SEQ ID NO: 26 = Transcription termination region from proteinase inhibitor form potato
SEQ ID NO: 27 = Carotene desaturase (Tomato)
SEQ ID NO: 28 = Carotene desaturase (Pepper)
SEQ ID NO: 29= Carotene desaturase (Maize)
SEQ ID NO: 30 = Carotene desaturase (Rice)
SEQ ID NO: 31 = Zeta-carotene desaturase (Tomato)
SEQ ID NO: 32 = Zeta-carotene desaturase (Pepper)
SEQ ID NO: 33 = Zeta-carotene desaturase (Maize)
SEQ ID NO: 34 = Zeta-carotene desaturase (Rice)
SEQ ID NOS: 35 to 38 = Primers

Glu = Glutelin seed preferred promoter
Pro = Prolamin seed preferred promoter
Cat = Intron from catalase gene
SSU = Transit peptide (Small sub-unit Rubisco)
CrtI = Carotene desaturase from Erwinia
Pds = Carotene desaturase (source indicated in parenthesis)
Psy = Phytoene synthase (source indicated in parenthesis)
Zds = Zeta-carotene desaturase
Pds = Phytoene desaturase
Nos = Transcription termination region from nopaline synthase gene
35S term = Transcription termination region from 35S CaMV
PotP1-II term = Transcription termination region from proteinase inhibitor form potato

FIGURE 1 = Part of carotenoid biosynthesis pathway starting from GGPP (Geranylgeranyl diphosphate).
FIGURE 2 = Construct pRP0117.

### EXAMPLES

General molecular biology methods are carried out according to Sambrook et al (1989) 'Molecular cloning: A laboratory Manual, 2nd Edition. Cold Spring Harbour Lab. Press.

### 1.0 Construction of plant binary vectors

References for the gene sequences below are given as they are listed on the EMBL database. This database is maintained and distributed by the European Bioinformatics Institute (Patricia Rodriguez-Tomé, Peter J. Stoehr, Graham N. Cameron and Tomas P. Flores, "The European Bioinformatics Institute (EBI) databases", Nucleic Acids Res. 24:(6-13), 1996, www.ebi.ac.uk.)

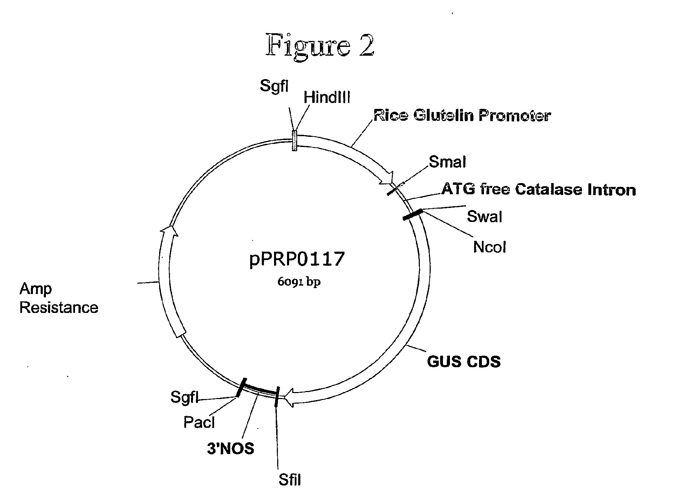

A pUC based vector pPRP0117 (Figure 2) was used for cloning of all the plant transformation vectors. This contains the nucleotides -806 to +33 of the rice glutelin gene as a promoter (Y00687), the first intron of the catalase-1 gene from castor bean altered to remove the ATG sequences, a *gus* coding region and a *nos* terminator. The *gus* coding sequence was removed by digestion of pPRP0117 with Ncol and Sfil and replaced by the coding regions of the carotenoid phytoene synthase genes or phytoene desaturase genes, or removed as a *gus: :nos* cassette by digestion of pPRP0117 with Nco1 and Pac1 and replaced by a carotenoid coding region/ nos terminator fusion as described below.

### 1.1 Construction of the pPRP0117 +crtI vector

A cassette of the signal peptide of the small subunit of pea ribulose bisphosphate carboxylase (*SSU*)(X00806) fused to the bacterial phytoene desaturase *CrtI* (D90087) and a *nos* terminator was cloned into the Nco1 and Pac1 sites of pPRP0117. The *crtI* sequence in constructs 7651, 7650 and 11586 had 9 nucleotides extra (3 alanines) inserted after the first ATG in order to incorporate a NotI restriction site for cloning purposes.

### 1.2 Construction of the pJH0104HygCrtI binary vector

The SgfI Gt::intron::SSUcrtI::nos cassette was cloned into the PacI site of the binary vector pJH0104+Hyg (which contains a hygromycin resistance gene for antibiotic selection) to give the construct pJH0104HygSSUCrtI.

### 1.3 Pepper psy + crtI construct 7651

The catalase intron and pepper *psy* (X68017) were separately amplified by PCR using primers that overlap both sequences, and then fused by recombinant PCR, and cloned into the EcoRI and SfiI sites of pPRP0117. The Gt::intron::pepper psy::nos cassette was recovered with SgfI digestion and cloned into the PacI site of pJH0104HygCrtI to give the construct 7651.

### 1.4 Tomato psy + crtI construct 7650

The catalase intron and tomato *psy* (Y00521) were separately amplified by PCR using primers that overlap both sequences, and then fused by recombinant PCR and cloned into the EcoRI and SfiI sites of pPRP0117. The Gt::intron::tomato psy::nos cassette was recovered by Sgfl and cloned into the PacI site of pJH0104HygCrtI to give the construct 7650.

### 1.5 Rice psy + crtI construct 11586

PolyA mRNA was extracted from rice leaves (Asanohikari). First strand synthesis of Rice *psy* cDNA was synthesized using the antisense primer 5' cgtcggcctgcatggccctacttctggctatttctcagtg 3' (SEQ ID NO: 35) and cDNA was then obtained by PCR amplification with this antisense primer and the sense primer 5'ctgtccatggcggccatcacgctcct 3' (SEQ ID NO: 36). This was digested with NcoI and SfiI and cloned into pFRP0117. The Gt::intron::rice psy::nos fragment was transferred to the binary vector pJH0104Hyg. A Hindni/PacI Gt::intron::SSUcrtI::nos cassette was blunt ended and cloned into the Pmel site of the pJH0104HygRicepsy vector to give the construct 11586.

### 1.6 Maize psy (E1B) + crtI construct 12421

PolyA mRNA was extracted from maize leaves. First strand synthesis of maize *psy* (sequence designated "E1B") cDNA was synthesized using the antisense primer 5' cgatggcctgcatggccctaggtctggccatttctcaatg 3' (SEQ ID NO: 37) and cDNA was then obtained by PCR amplification with this antisense primer and the sense primer 5' taggataagatagcaaatccatggccatcata 3' (SEQ ID NO: 38). This was digested with NcoI and SfiI and cloned into the Ncol and Sfil sites of a pPRP0117 based vector. The Gt::intron::maize psy::nos cassette was recovered with HindIII/Pacl digestion and cloned into a binary vector containing a Gt::intron::SSUcrtI::nos cassette to give the construct 12421.

### 1.7 Maize psy (E1B) + crtI construct 12422

The vector with the Gt::intron::maize psy::nos cassette in the pPRP0117 backbone (from construction of 12421 above) was digested with the restriction enzymes flanking the intron followed by religation of the vector in order to remove the catalase intron. The Gt::maize psy::nos cassette was recovered with HindIII/Pac1 digestion and cloned into a binary vector containing a Gt::SSUcrtI::nos cassette to give the construct 12422.

### 1.8 Maize psy + crtI construct 12423

The Y1 maize psy (U32636) cds sequence was synthesised with Nco1 and Sfi1 restriction sites added at the 5' and 3' end respectively. This was cloned into the Nco1 and Sfi1 sites of a pPRP0117 based vector. The Gt::intron::maize psy::nos cassette was recovered with HindIII/Pac1 digestion and cloned into a binary vector containing a Gt::intron::SSUcrtI::nos cassette to give the construct 12423.

### 1.9 Maize psy + crtI construct 12424

The vector with the Gt::intron::maize psy::nos cassette in the pPRP0117 backbone (from construction of 12423 above) was digested with the restriction enzymes flanking the intron followed by religation of the vector in order to remove the catalase intron. The Gt::maize psy::nos cassette was recovered with HindIII/Pad 1 digestion and cloned into a binary vector containing a Gt::SSUcrtI::nos cassette to give the construct 12424.

### 2.0 Construction of vectors for plant transformation.

When providing vectors for plant transformation which utilise *Agrobacterium,* it is preferred that the sequences according to the invention are inserted between the border regions of a single TDNA region. *Agrobacterium* may be transformed in accordance with methods which are well known to the person skilled in the art, and/or via the methods disclosed herein.

### 3.0 Transformation of Rice

Based on the protocol published by Hiei et al (1994 The Plant Journal, 6 (2), 271-282). The key modification involves use of a supervirulent strain in combination with a standard binary vector.
The basic procedure is as follows:
Mature rice seed (Asanohikari) are de-husked and surface sterilised by 70% ethanol for one minute followed by 4% Sodium hypochlorite + Tween for 30 minutes. Seed are then sown on a callus induction media (CIM) (N6 salts, N6 vitamins, 30 g/l sucrose, 1 g/l casein hydrolysate, 2 mg/12,4-D, pH 5.8, 4 g/l Gelrite) and placed in the dark at 30°C.
After 3 weeks embryogenic calli are isolated and plated on CIM and placed under the same conditions. At the same time *Agrobacterium* cultures (AGL1 strain plus binary) are established by spreading inoculum onto LB plates plus Kanamycin (50 mg/l). After three days, *Agrobacterium* is scraped from the plate and re-suspended in AA1 + AS (AA salts, B5 vitamins, AA Amino Acids, 68.5 g/l sucrose, 36 g/l glucose, 0.5 g/l casein hydrolysate, 100 µM Acetosyringone, pH 5.2) to an optical density of 0.1 at 600 nm. The embryogenic calli is inoculated with the *Agrobacterium* solution for 10 minutes after which the calli are spread on to plates of R2COMAS (R2 Micro salts, ½ R2 Macro salts, B5 Vitamins, 20 g/l sucrose, 10 g/l glucose, 1 g/l casein hydrolysate, 2 mg/12,4-D, 100 µM Acetosyringone, pH 5.2) and placed in the dark at 26°C. After three days calli are transferred on to selection media (N6 salts, N6 vitamins, 30 g/l sucrose, 1 g/l casein hydrolysate, 2 mg/l2,4-D, 300 mg/l Timentin, 50 mg/l Hygromycin, 4 g/l Gelrite, pH 5.8) and place in the light at 30°C. All of the following steps occur under the same growth conditions (Light, 30°C). After three weeks the putative transgenic calli are transferred on a pre-regeneration media (N6 salts and vitamins, 30 g/l sucrose, 1 g/l casein hydrolysate, 1 mg/12,4-D, 300 mg/l Timentin, 50 mg/l Hygromycin, 6 g/l Gelrite, pH 5.8). After two weeks the good quality embryogenic calli is transferred to regeneration media (N6 Micro, ½ N6 Macro, N6 vitamins, AA amino acids, 20 g/l sucrose, 1 g/l casein hydrolysate, 0.2 mg/l NAA, 1 mg/l Kinetin, 50 mg/l Hygromycin, pH 5.8, Gelrite 6 g/l). After three weeks, plantlets that regenerate are subcultured to rooting media (½ MS salts, ½ B5 Vitamins, 10 g/l sucrose, 25 mg/l Hygromycin, pH 5.8, 8 g/l microagar. After two weeks the plantlets that have robust root systems are transferred to soil (50% John Innes #3, 50% peat, 26°C, 16 hour photoperiod) and covered with a fleece until the plants are established.

### 4.0 Analysis of carotenoids in rice transformants

Carotenoids are extracted from seeds harvested at maturity. Seed is dehusked using a TR-200 Electromotion Rice Husker and then polished for 1 min. with a Pearlest polisher (Kett). Any white or discoloured seed are removed and 0.5g of the sample is ground for 2 minutes using a Glen Creston 8000 Mixer/Mill. A total of 1g of ground material/plant may be obtained by this method. This can be thoroughly mixed together prior to extraction of 2 x 0.5g portions of the powder. A standard compound can then be added to the samples for quantification of recoveries. Astaxanthin and echinenone are examples of standards which can be used. Samples are hydrated with 1ml of water and mixed using a vortex for a few seconds. 6ml of acetone is then added and the samples sonicated for 2 minutes. The samples are centrifuged for 5 min. at 3500 rpm. The supernatants are decanted, and the samples re-extracted twice more with 3 ml acetone, repeating the sonication/centrifugation steps between extractions. One extraction with 2 ml of tert-methylbutylether is then performed including the sonication/centrifugation steps and all supernatants for one sample pooled together. The total volume for each extract is adjusted to 14 ml with acetone and the centrifugation step repeated. A 2ml aliquot of each sample is evaporated to dryness under a stream of nitrogen gas. These aliquots are re-dissolved in 75 µl of ethyl acetate, vortexed for 5-10 s and then transferred to amber HPLC insert vials. The vials are sealed immediately and centrifuged again at 3500 rpm prior to HPLC analysis.
The HPLC quantification is based on the response factor determined for each reference standards. The overall concentration of the prepared and dissolved reference standards is measured spectrophotometrically based on the published molar extinction coefficients and/or absorbance measured for solution of 1 % concentration using 1cm path length (A1%1cm) (Ref: Britton G., Liaanen-Jensen S. and Pfander H. P. (1995) Carotenoids: Spectroscopy Vol 1B pp57-62, Birkhauser Verlag, Basel, ISBN 3-7643-2909-2). For each standard stock solution, the purity of the principal component is determined by HPLC. For components where no reference standard is available e.g. various cis isomers quantitative results are expressed using the response factor for that of β-carotene.

### 4.1 HPLC Equipment & Conditions

| | | |
|---|---|---|
| Pump | : | Agilent 1100 Quaternary or Binary Pump; model number G1311A or G1312A, respectively |
| Degasser | : | Agilent 1100 Degasser; model number G1322A |
| Temperature Controlled | : | Agilent 1100 Automatic Liquid Sampler; model number G1313A equipped with autosampler |
| Autosampler | | temperature controller model number G1330A |
| Detector | : | Agilent 1100 Diode array detector; model number G1315A or G1315B |
| | | Agilent 1100 fluorescence detector; model number G1321A (for tocopherol analysis only). |
| Column Oven | : | Agilent 1100 Column Compartment; model number G1316A |

| Instrument Conditions | | |
|---|---|---|
| Column | : | YMC C30 3 µm particles in 25 cm x 4.6 mm id stainless steel column + 1cm x 4 mm 5 µm |
| | | YMC C30 guard column |
| Column temperature | : | 25°C |
| Sample temperature | : | 4°C |
| Mobile phase | : | Solvent A = MeOH/H2O/tert-butylmethylether (TBME) + 1.3mM NH4acetate (70/25/5 v/v) |
| | | Solvent B = MeOH/H2O/TBME + 1.3mM |
| | | NH4acetate (7/3/90 v/v) |
| Stop time | | 30 min |
| Post time | | 0 min |
| Flow rate | | 1 ml min-1 |
| Injection volume | | 25 µl in ethyl acetate |

### Gradient conditions (6%/min):

| Time | A% | B% |
|---|---|---|
| (min) | MeOH/H2O/TB ME + 1.3mM | MeOH/H2O/TB ME + 1.3mM |
| | NH4acetate | NH4acetate |
| | (70/25/5 v/v) | (7/3/90 v/v) |
| 0 | 95 | 5 |
| 15.83 | 0 | 100 |
| 22 | 0 | 100 |
| 24 | 95 | 5 |
| 30 | 95 | 5 |

### 5.0 Results of HPLC quantification of carotenoids:

Results for rice plants transformed with construct 11586 (construct described in 1.5 above)

| **Sample identity** | **µg/g dry weight (dwt) endosperm** |
|---|---|
| wild type | 0.05 |
| 11586-10 | 4.19 |
| 11586-7 | 6.11 |
| 11586-25 | 6.32 |
| 11586-15 | 7.55 |
| 11586-30 | 7.69 |
| 11586-28 | 9.05 |
| 11586-14 | 11.82 |
| 11586-20 | 12.82 |
| 11586-1 | 13.29 |
| 11586-12 | 18.59 |

| **Sequence ID Number** | **Components** | **µg/g dwt endosperm** |
|---|---|---|
| 7 | crtI + pepper psy | >3 |
| 8 | crtI + tomato psy | >3 |
| 9 | crtI + crtB | >5 |
| - | Untransformed Control | 0 |

### 6.0 Transformation of rice - method used for transformation of rice withAgrobacterium comprising constructs 12421, 12422, 12423 and 12424 (constructs described in examples 1.6, 1.7, 1.8 and 1.9 respectively).

For this example, rice (*Oryza sativa*) is used for generating transgenic plants. Various rice cultivars can be used (Hiei et al., 1994, Plant Journal 6:271-282; Dong et al., 1996, Molecular Breeding 2:267-276; Hiei et al., 1997, Plant Molecular Biology, 35:205-218). Also, the various media constituents described below may be either varied in concentration or substituted. Embryogenic responses are initiated and/or cultures are established from mature embryos by culturing on MS-CIM medium (MS basal salts, 4.3 g/liter; B5 vitamins (200 x), 5 ml/liter; Sucrose, 30 g/liter; proline, 500 mg/liter; glutamine, 500 mg/liter; casein hydrolysate, 300 mg/liter; 2,4-D (1 mg/ml), 2 ml/liter; adjust pH to 5.8 with 1 N KOH; Phytagel, 3 g/liter). Either mature embryos at the initial stages of culture response or established culture lines are inoculated and co-cultivated with the *Agrobacterium* strain LBA4404 containing the desired vector construction. *Agrobacterium* is cultured from glycerol stocks on solid YPC medium (100 mg/L spectinomycin and any other appropriate antibiotic) for ~2 days at 28 °C. *Agrobacterium* is re-suspended in liquid MS-CIM medium. The *Agrobacterium* culture is diluted to an OD600 of 0.2-0.3 and acetosyringone is added to a final concentration of 200 uM. *Agrobacterium* is induced with acetosyringone before mixing the solution with the rice cultures. For inoculation, the cultures are immersed in the bacterial suspension. The liquid bacterial suspension is removed and the inoculated cultures are placed on cocultivation medium and incubated at 22°C for two days. The cultures are then transferred to MS-CIM medium with Ticarcillin (400 mg/liter) to inhibit the growth of *Agrobacterium.* For constructs utilizing the PMI selectable marker gene (Reed et al., In Vitro Cell. Dev. Biol.-Plant 37:127-132), cultures are transferred to selection medium containing Mannose as a carbohydrate source (MS with 2%Mannose, 300 mg/liter Ticarcillin) after 7 days, and cultured for 3-4 weeks in the dark. Resistant colonies are then transferred to regeneration induction medium (MS with no 2,4-D, 0.5 mg/liter IAA, 1 mg/liter zeatin, 200 mg/liter timentin 2% Mannose and 3% Sorbitol) and grown in the dark for 14 days. Proliferating colonies are then transferred to another round of regeneration induction media and moved to the light growth room. Regenerated shoots are transferred to GA7-1 medium (MS with no hormones and 2% Sorbitol) for 2 weeks and then moved to the greenhouse when they are large enough and have adequate roots. Plants are transplanted to soil in the greenhouse and grown to maturity.

### 7.0 Method of extraction/quantification of carotenoids - for plants transformed in accordance with Example 6.0

(a) Sample is grinded in a Geno/Grinder at 1600 rpm for 40 seconds.
(b) Representative amounts of homogenised sample (0.1 g) are weighed into an appropriate extraction vessel (e.g. 2 ml microcentrifuge tube). Sample weight is recorded.
(c) Samples are hydrated with water (200 µl) and vortexed for about 2-3 seconds then left to stand for about 10 minutes.
(d) Acetone (1.2 ml) is added to the sample.
(e) Ultrasonicate sample for 5 minutes.
(f) Centrifuge sample for 3 min at 6000 rpm, then transfer the supernatant to another appropriately sized tube.
(g) Steps (d) to (f) are repeated and combined with the previous extract.
(h) Steps (d) to (f) are repeated with tert-butylmethylether (400 µl) and combine with the acetone extracts.
(i) All of the combined extracts are transferred into an appropriate size tube and evaporate it to dryness under a stream of nitrogen gas.
(j) Samples are redissolved in 2 ml of ethyl acetate + 0.5% BHT, vortex or ultrasonicate for 5-10 seconds and then centrifuged at 3500 rpm for 5 minutes before HPLC analysis.
(k) Absorption at wavelength 450 nm is measured on a spectrophotometer for all samples and total carotenoid concentration is calculated based on an ε value of 124865.

### 8.0 Results of the method of Example 7.0

| **Construct** | **Sample ID** | **Calculated Carotenoids (µg/g)** |
|---|---|---|
| 12421 | RIGQ2003001046A4A | 48.7 |
| 12421 | RIGQ2003001063A43A | 42.7 |
| 12421 | RIGQ2003001063A4A | 36.9 |
| 12421 | RIGQ2003001063A99A | 31.4 |
| 12421 | RIGQ2003001063A59A | 30 |
| 12421 | RIGQ2003001063A75A | 28.7 |
| 12421 | RIGQ2003001048A3A | 28.5 |
| 12421 | RIGQ2003001050A23A | 28 |
| 12421 | RIGQ2003001050A13A | 26.8 |
| 12421 | RIGQ2003001048A25A | 26.8 |
| 12421 | RIGQ2003001048A61A | 26.1 |
| 12421 | RIGQ2003001049A46A | 23.4 |
| 12421 | RIGQ2003000993A63A | 21.7 |
| 12421 | RIGQ2003001049A43A | 20.6 |
| 12421 | RIGQ2003001049A26A | 10.5 |
| 12421 | RIGQ2003001050A18A | 5.7 |

| **Construct** | **Sample ID** | **Calculated Carotenoids (µg/g)** |
|---|---|---|
| 12422 | RIGQ2003001045A30A | 58.8 |
| 12422 | RIGQ2003001045A51A | 54 |
| 12422 | RIGQ2003000995A29A | 43.6 |
| 12422 | RIGQ2003000995A31A | 43.1 |
| 12422 | RIGQ2003001043A10A | 42.4 |
| 12422 | RIGQ2003001043A8A | 42 |
| 12422 | RIGQ2003000995A19A | 41.9 |
| 12422 | RIGQ2003000995A17A | 40.9 |
| 12422 | RIGQ2003001060A23A | 37.2 |
| 12422 | RIGQ2003001052A56A | 35 |
| 12422 | RIGQ2003001051A75A | 32 |
| 12422 | RIGQ2003001045A68A | 30.6 |
| 12422 | RIGQ2003001045A86A | 29.8 |
| 12422 | RIGQ2003001045A49A | 29.2 |
| 12422 | RIGQ2003001060A25A | 28.9 |
| 12422 | RIGQ2003001051A64A | 26.4 |
| 12422 | RIGQ2003001051A34A | 25.2 |
| 12422 | RIGQ2003000994A7A | 22.8 |
| 12422 | RIGQ2003001045A74A | 20.3 |
| 12422 | RIGQ2003001051A46A | 16.6 |
| 12422 | RIGQ2003000995A7A | 13.8 |
| 12422 | RIGQ2003000995A26A | 8.1 |
| 12422 | RIGQ2003000995A41A | 6.8 |
| 12422 | RIGQ2003000995A8A | 3.8 |

| **Construct** | **Sample ID** | **Calculated Carotenoids (µg/g)** |
|---|---|---|
| 12423 | RIGQ2003001097A42A | 52.80 |
| 12423 | RIGQ2003001097A15A | 50.40 |
| 12423 | RIGQ2003001097A41A | 48.00 |
| 12423 | RIGQ2003001114A10A | 44.20 |
| 12423 | RIGQ2003001099A8A | 40.00 |
| 12423 | RIGQ2003001098A2A | 28.80 |
| 12423 | RIGQ2003001099A42A | 26.00 |
| 12423 | RIGQ2003001097A30A | 24.00 |
| 12423 | RIGQ2003001186A80A | 23.30 |
| 12423 | RIGQ2003001097A18A | 21.60 |
| 12423 | RIGQ2003001099A60A | 17.60 |
| 12423 | RIGQ2003001098A5A | 17.40 |
| 12423 | RIGQ2003001097A44A | 12.00 |

| **Construct** | **Sample ID** | **Calculated Carotenoids (µg/g)** |
|---|---|---|
| 12424 | RIGQ2003001121A60A | 51.9 |
| 12424 | RIGQ2003001121A20A | 51.8 |
| 12424 | RIGQ2003001093A11A | 40 |
| 12424 | RIGQ2003001094A85A | 37.8 |
| 12424 | RIGQ2003001093A58A | 27.3 |
| 12424 | RIGQ2003001118A84A | 26 |

Although the invention has been described by way of the above-referenced examples and the Sequence Listing and Figures as provided herein, it will be apparent that modifications and changes may be practiced which remain within the ambit of the present invention.

### References:

| Name | Database Accession No. | Description | Reference |
|---|---|---|---|
| Glutelin1 | NCBI D00584, EMBL Y00867 | Rice, promoter of a rice seed storage protein | Takaiwa,F.; Ebinuma,H.; Kikuchi, S.; Oono,K.; Nucleotide sequence of a rice glutelin gene, FEBS Lett. 221:43 (1987) |
| Prolamin | D73383 D73384 | Rice, promoter of a rice seed storage protein | Nakase,M.; Yamada, T.; Kira, T.; Yamaguchi,J.; Aoki,N.; Nakamura,R.; Matsuda, T.; Adachi, T.. The same nuclear proteins bind to the 5'-flanking regions of genes for the rice seed storage protein: 16kDa albumin, 13 kDa prolamin and type II glutelin. Plant Mol. Biol. 32:621-630 (1996) |
| Prolamin | M23746 | Rice, promoter of a rice seed storage protein | Kim,W.T.; Okita,T.W.; Structure, expression, and heterogeneity of the rice seed prolamines Plant Physiol. 88:649 (1988) |
| 1^{st} intron of catalase | D21161 | Castor bean | Suzuki,M.; Ario,T.; Hattori,T.; Nakamura,K.; Isolation and characterization of two tightly gene linked catalase genes from castor bean that are differentially regulated Plant Mol. Biol. 25:507 (1994 |
| SSU | X00806 | Pea, signal peptide of small subunit of rubisco | Coruzzi,G.; Broglie,R.; Edwards,C.; Chua,N.H.; Tissue-specific and light-regulated expression of a pea nuclear gene encoding the small subunit of ribulose-1,5- bisphosphate carboxylase EMBO J. 3:1671 (1984 |
| SSU | X04334 | Pea, signal peptide of small subunit of ribulose bisphosphate carboxylase | Fluhr,R.; Moses,P.; Morelli,G.; Coruzzi,G.; Chua,N.H.; Expression dynamics of the pea rbcS multigene family and organ distribution of the transcripts EMBO J. 5:2063 (1986 |
| Maize psy | U32636 | Maize phytoene synthase gene | Buckner,B.; Miguel,P.S.; Janick-Buckner,D.; Bennetzen,J.L.; The yl gene of maize codes for phytoene synthase Genetics 143(1):479 (1996) |
| Pepper psy | X68017 | Pepper phytoene synthase gene | Romer,S.; Hugueney,P.; Bouvier,F.; Camara,B.; Kuntz,M.; Expression of the genes encoding the early carotenoid biosynthetic enzymes in Capsicum annuum Biochem. Biophys. Res. Commun. 196:1414 (1993 |
| Tomato psy | Y00521 | tomato phytoene synthase gene | Ray,J.; Bird,C.R.; Maunders,M.; Grierson,D.; Schuch,W.; Sequence of ptom 5 a ripening related cDNA from tomato Nucleic Acids Res. 15:10587 (1987) |
| Daffodil psy | X78814 | daffodil phytoene synthase gene | Schledz,M.; Ali Babili,S.; Lintig,J.; Haubruck,H.; Rabbani,S.; Kleing,H.; Beyer,P.; Phytoene synthase from Narcissus pseudonarcissus: functional, expression, galactolipid requirement, topological distribution in chromoplasts and induction during flowering Plant J. 10:781 (1996 |
| CrtB | D90087 | Erwinia uredovora phytoene synthase | Misawa,N.; Nakagawa,M.; Kobayashi,K.; Yamano,S.; Izawa,Y.; Nakamura,K.; Harashima,K.; Elucidation of the Erwinia uredovora carotenoid biosynthetic pathway by functional analysis of gene products expressed in Escherichia coli J. Bacteriol. 172:6704 (1990 |
| CrtI | D90087 | Erwinia uredovora phytoene desaturase | Misawa,N.; Nakagawa,M.; Kobayashi, K.; Yamano, S.; Izawa,Y.; Nakamura, K.; Harashima,K.; Elucidation of the Erwinia uredovora carotenoid biosynthetic pathway by functional analysis of gene products expressed in Escherichia coli J. Bacteriol. 172:6704 (1990 |
| Nos | AJ237588 | Agrobacterium tumefaciens Ti plasmid | |
| Phytoene Desaturase | M88683 | Tomato | Giuliano,G.; Bartley,G.E.; Scolnik,P.A.; Regulation of carotenoid biosynthesis during tomato development Plant Cell 5(4):379 (1993) |
| Phytoene Desaturase | X68058 | Pepper | Hugueney,P.; Roemer,S.; Kuntz,M.; Camara,B.; Characterization and molecular cloning of a flavoprotein catalyzing the synthesis of phytofluenen and zeta-carotene in Capsicum chromoplasts Eur. J. Biochem. 209:399 (1992 |
| Phytoene Desaturase | U37285 | Maize | Li,Z.H.; Matthews,P.D.; Burr,B.; Wurtzel,E.T.; Cloning and characterization of a maize cDNA encoding phytoene desaturase, an enzyme of the carotenoid biosynthetic pathway Plant Mol. Biol. 30(2):269 (1996 |
| Phytoene Desaturase | AF049356 | Rice | Vigneswaran,A.; Wurtzel,E.T.; A Rice cDNA Encoding Phytoene Desaturase (Accession No. AF049356) (PGR99-131) Plant Physiol. 121(1):312 (1999) |
| ZDS | AF195507 | Tomato | Bartley,G.E.; Ishida,B.K.; Zeta-carotene desaturase (Accession No. AF195507) from tomato (PGR99-181) Plant Physiol. 121(4):1383 (1999) |
| ZDS | X89897 | Pepper | Albrecht,M.; Klein,A.; Hugueney,P.; Sandmann,G.; Kuntz,M.; Molecular cloning and functional expression in E. coli of a novel plant enzyme mediating zeta-carotene desaturation FEBS Lett. 372:199 (1995) |
| ZDS | AF047490 | Maize | Luo,R.; Wurtzel,E.T.; A Maize cDNA Encoding Zeta Carotene Desaturase (Accession No. AF047490). (PGR99-118) Plant Physiol. 120(4):1206 (1999) |
| ZDS | AF054629 | Rice | |

## Claims

1. A polynucleotide comprising:
(a) a region which comprises as operably linked components (i) a promoter which provides for seed preferred expression; and (ii) a nucleotide sequence derived from a bacterium which sequence encodes a carotene desaturase; and (iii) a transcription termination region; and
(b) a further region which comprises as operably linked components (i) a promoter which provides for seed preferred expression; and (ii) a nucleotide sequence encoding a phytoene synthase which sequence is derived from maize (*Zea sp.*) or rice (*Orzya sp.*)*;* and (iii) a transcription termination region.

2. A polynucleotide according to claim 1 wherein the sequence which encodes the carotene desaturase is derived from *Erwinia sp.*

3. A polynucleotide according to claim 1 or claim 2 wherein said promoter is selected from the Glutelin 1 promoter and the Prolamin promoter and said transcription termination region is selected from the Nos; CaMV 35S and PotP1-II transcription termination regions.

4. A polynucleotide according to any one of claims 1 to 3 wherein the sequence which encodes carotene desaturase and the sequence which encodes phytoene synthase further comprises a sequence encoding a plastid targeting sequence.

5. A polynucleotide according to any one of claims 1 to 4 which comprises a sequence selected from the group depicted as SEQ ID NO: 1; 2; 3; 4; and 6.

6. A polynucleotide sequence which is the complement of one which hybridises to a polynucleotide according to claim 5 at a temperature of about 65°C in a solution containing 6 x SSC, 0.01% SDS and 0.25% skimmed milk powder, followed by rinsing at the same temperature in a solution containing 0.2 x SSC and 0.1% SDS wherein said polynucleotide sequence still comprises a region encoding a carotene desaturase and a further region encoding a phytoene synthase and wherein when said polynucleotide sequence is inserted into plant material the seed of a plant regenerated from said material produces carotenoids at a level of at least 10µg/g of endosperm of said seed.

7. A polynucleotide sequence which is the complement of one which hybridises to a polynucleotide according to claim 5 at a temperature of about 65°C in a solution containing 6 x SSC, 0.01% SDS and 0.25% skimmed milk powder, followed by rinsing at the same temperature in a solution containing 0.2 x SSC and 0.1% SDS wherein said polynucleotide sequence still comprises a region encoding a carotene desaturase and a further region encoding a phytoene synthase and when said polynucleotide sequence is inserted into plant material the seed of a plant regenerated from said material produce carotenoids amounting to at least 80% of the carotenoid content of a seed which comprises a polynucleotide selected from the group depicted as SEQ ID NO: 1; 2; 3; 4; 5 and 6.

8. A polynucleotide sequence according to any one of claims 6 to 7 wherein said seed is a rice seed.

9. A polynucleotide or a polynucleotide sequence according to any one of claims 1 to 8 which further comprises a region which encodes a selectable marker.

10. A polynucleotide or a polynucleotide sequence according to claim 9 wherein said selectable marker comprises a mannose-6-phosphate isomerase gene.

11. A polynucleotide or a polynucleotide sequence according to any one of claims 1 to 10 which is codon optimised for expression in a particular plant species.

12. A polynucleotide or a polynucleotide sequence according to claim 11 wherein said plant species is rice (*Orzya sp.*)*.*

13. A vector comprising a polynucleotide or a polynucleotide sequence according to any one of claims 1 to 12.

14. A method for increasing the carotenoid content of seeds comprising inserting into plant material a polynucleotide or a polynucleotide sequence according to any one of claims 1 to 12 or a vector according to claim 13; and regenerating a seed-containing plant from said material and identifying the seeds which contain carotenoids at levels greater than those of control like-seeds.

15. A method for increasing the carotenoid content of a seed comprising inserting into plant material a polynucleotide comprising a sequence selected from the group depicted as SEQ ID NO: 1; 2; 3; 4; 5 and 6 and regenerating a seed-containing plant from said material and identifying the seed which contains carotenoids at levels greater than those of a control like-seed.

16. A method according to claim 14 or claim 15 wherein said seed contains at least a sixty fold increase in carotenoids when compared to a control like-seed.

17. A method according to claim 14 or claim 15 wherein said seed contains carotenoids at a level of at least 10µg/g of endosperm of said seed.

18. A method according to any one of claims 14 to 17 wherein said carotenoids are selected from the group consisting of: lycopene; alpha-carotene; lutein; beta-carotene; zeanthin; antheraxanthin; violaxanthin; and neoxanthin or a combination thereof.

19. A plant or plant material which comprises a polynucleotide or a polynucleotide sequence according to any one of claims 1 to 12 or a vector according to claim 13.

20. A plant or plant material according to claim 19 which is a rice plant or is rice plant material.

21. A plant or plant material according to claim 19 which is a maize plant or is maize plant material.

22. Use of a polynucleotide or a polynucleotide sequence according to any one of claims 1 to 12 or a vector according to claim 13 in a method for the production of seeds containing increased carotenoids.

23. Use of a polynucleotide selected from the group depicted as SEQ ID NO: 1; 2; 3; 4; 5 and 6 for the production of a seed which contains carotenoids at levels greater that those of a control like-seed.

## Patentansprüche

1. Polynucleotid umfassend:
(a) einen Bereich, welcher als operativ verbundene Bestandteile (i) einen Promotor, welcher eine in Samen bevorzugte Expression ermöglicht; und (ii) eine Nucleotidsequenz, die von einem Bakterium stammt, wobei die Sequenz eine Carotin-Desaturase kodiert; und (iii) einen Transkriptions-Beendigungsbereich umfasst; und
(b) einen weiteren Bereich, welcher als operativ verbundene Bestandteile (i) einen Promotor, der eine in Samen bevorzugte Expression ermöglicht ; und (ii) eine Nucleotidsequenz, die eine Phytoensynthase kodiert, wobei die Sequenz aus Mais (Zea sp.) oder Reis (Orzya sp.) stammt; und (iii) einen Transkriptions-Beendigungsbereich umfasst.

2. Polynucleotid gemäß Anspruch 1, wobei die Sequenz, die die Carotin-Desaturase kodiert, aus Erwinia sp. stammt.

3. Polynucleotid gemäß Anspruch 1 oder Anspruch 2, wobei der Promotor ausgewählt ist aus dem Glutelin 1-Promotor und dem Prolamin-Promotor, und der Transkriptions-Beendigungsbereich ausgewählt ist aus Nos; CaMV 35S und dem PotP1-II-Transkriptions-Beendigungsbereich.

4. Polynucleotid gemäß irgend einem der Ansprüche 1 bis 3, wobei die Sequenz, welche die Carotin-Desaturase kodiert, und die Sequenz, welche die Phytoen-Synthase kodiert, weiter eine Sequenz umfasst, die eine Plastid-Zielsequenz kodiert.

5. Polynucleotid gemäß irgendeinem der Ansprüche 1 bis 4, welches eine Sequenz umfasst, die aus der Gruppe ausgewählt ist, die in den SEQ ID Nr. 1, 2, 3, 4 und 6 wiedergegeben ist.

6. Polynucleotidsequenz, welche das Komplement einer ist, die mit einem Polynucleotid gemäß Anspruch 5 bei einer Temperatur von ungefähr 65°C in einer Lösung hybridisiert, die 6 x SSC, 0,01 % SDS und 0,25 % Magermilchpulver enthält, gefolgt vom Spülen bei der gleichen Temperatur in einer Lösung, die 0,2 x SSC und 0,1 % SDS enthält, wobei die Polynucleotidsequenz noch einen Bereich umfasst, der eine Carotin-Desaturase kodiert und einen weiteren Bereich, welcher eine Phytoen-Synthase kodiert, und wobei, wenn die Polynucleotidsequenz in Pflanzenmaterial inseriert ist, der Samen einer Pflanze, der aus diesem Material regeneriert wird, Carotinoide in einer Konzentration von mindestens 10 µg/g des Endosperms dieses Samens produziert.

7. Polynucleotidsequenz, welche das Komplement einer ist, die mit einem Polynucleotid gemäß Anspruch 5 bei einer Temperatur von ungefähr 65°C in einer Lösung hybridisiert, die 6 x SSC, 0,01 % SDS und 0,25 % Magermilchpulver enthält, gefolgt vom Spülen bei der gleichen Temperatur in einer Lösung, die 0,2 x SSC und 0,1 % SDS enthält, wobei die Polynucleotidsequenz noch einen Bereich umfasst, welcher eine Carotin-Desaturase kodiert, und einen weiteren Bereich, welcher eine Phytoen-Synthase kodiert, und wenn die Polynucleotidsequenz in Pflanzenmaterial inseriert ist, Samen einer Pflanze, die aus diesem Material regeneriert ist, Carotinoide herstellen, die bis zu 80 % des Carotinoidgehalts eines Samens ausmachen, welcher ein Polynucleotid umfasst, das aus der Gruppe ausgewählt ist, die in SEQ ID Nr. 1, 2, 3, 4, 5 und 6 wiedergegeben ist.

8. Polynucleotidsequenz gemäß irgendeinem der Ansprüche 6 bis 7, wobei der Samen ein Reissamen ist.

9. Polynucleotid oder eine Polynucleotidsequenz gemäß irgendeinem der Ansprüche 1 bis 8, welche ferner einen Bereich umfasst, welcher einen selektierbaren Marker kodiert.

10. Polynucleotid oder eine Polynucleotidsequenz gemäß Anspruch 9, wobei der selektierbare Marker das Mannose-6-phosphatisomerase-Gen umfasst.

11. Polynucleotid oder Polynucleotidsequenz gemäß irgendeinem der Ansprüche 1 bis 10, welche für die Expression in einer besonderen Pflanzenspezies Kodon-optimiert sind.

12. Polynucleotid oder Polynucleotidsequenz gemäß Anspruch 11, wobei die Pflanzenart Reis ist (Orzya sp.).

13. Vektor umfassend ein Polynucleotid oder eine Polynucleotidsequenz gemäß irgendeinem der Ansprüche 1 bis 12.

14. Verfahren zur Erhöhung des Carotinoidgehalts von Samen, welches das Inserieren eines Polynucleotids oder einer Polynucleotidsquenz gemäß irgendeinem der Ansprüche 1 bis 12 oder eines Vektors gemäß Anspruch 13 umfasst; und Regenerieren einer samenhaltigen Pflanze aus diesem Material sowie das Identifizieren der Samen, die Carotinoide in größeren Konzentrationen als die der Kontrollsamen enthalten.

15. Verfahren zum Erhöhen des Carotinoidgehalts eines Samens, welches das Inserieren eines Polynucleotids in Pflanzenmaterial umfasst, welches eine Sequenz umfasst, die aus der Gruppe ausgewählt ist, die in SEQ ID Nr. 1, 2, 3, 4, 5 und 6 wiedergegeben ist, und Regenerieren einer samenhaltigen Pflanze aus diesem Material und Identifizieren der Samen, welche Carotinoide in Konzentrationen enthalten, die größer sind als die der Kontrollsamen.

16. Verfahren gemäß Anspruch 14 oder Anspruch 15, wobei diese Samen mindestens einen 60-fachen Anstieg an Carotinoiden enthalten, wenn sie mit einem Kontrollsamen verglichen werden.

17. Verfahren gemäß Anspruch 14 oder Anspruch 15, wobei dieser Samen Carotinoide in einer Konzentration von mindestens 10 µg/g des Endosperms dieses Samens enthält.

18. Verfahren gemäß irgendeinem der Ansprüche 14 bis 17, wobei diese Carotinoide ausgewählt sind aus der Gruppe bestehend aus: Lycopen, Alpha-Carotin, Lutein, Beta-Carotin, Zeanthin, Antheraxanthin, Violaxanthin und Neoxanthin oder einer Kombination daraus.

19. Eine Pflanze oder Pflanzenmaterial, welche ein Polynucleotid oder eine Polynucleotidsequenz gemäß irgendeinem der Ansprüche 1 bis 12 oder einen Vektor gemäß Anspruch 13 umfassen.

20. Eine Pflanze oder Pflanzenmaterial gemäß Anspruch 19, welche eine Reispflanze oder Reispflanzenmaterial sind.

21. Eine Pflanze oder Pflanzenmaterial gemäß Anspruch 19, welche eine Maispflanze oder Maispflanzenmaterial sind.

22. Verwendung eines Polynucleotids oder einer Polynucleotidsequenz gemäß irgendeinem der Ansprüche 1 bis 12 oder eines Vektors gemäß Anspruch 13 in einem Verfahren zur Herstellung von Samen, welche mehr Carotinoide enthalten.

23. Verwendung eines Polynucleotids ausgewählt aus der Gruppe, die in SEQ ID Nr. 1, 2, 3, 4, 5 und 6 wiedergegeben ist, zur Herstellung eines Samens, welcher Carotinoide in größeren Konzentrationen als die in Kontrollsamen enthält.

## Revendications

1. Polynucléotide comprenant:
(a) une région qui comprend en tant que constituants fonctionnellement liés (i) un promoteur qui assure une expression préférentielle dans les graines; et (ii) une séquence de nucléotides provenant d'une bactérie, cette séquence codant pour une carotène-désaturase; et (iii) une région de terminaison de la transcription; et
(b) une autre région qui comprend en tant que constituants fonctionnellement liés (i) un promoteur qui assure une expression préférentielle dans les graines; et (ii) une séquence de nucléotides codant pour une phytoène-synthase, cette séquence provenant de maïs (*Zea sp.* ) ou de riz (*Oryza sp.* )*;* et (iii) une région de terminaison de la transcription.

2. Polynucléotide selon la revendication 1, dans lequel la séquence qui code pour la carotène-désaturase provient de *Erwinia sp.*

3. Polynucléotide selon la revendication 1 ou la revendication 2, dans lequel ledit promoteur est choisi parmi le promoteur de glutéline 1 et le promoteur de prolamine, et ladite région de terminaison de la transcription est choisie parmi les régions de terminaison de la transcription de Nos; 35S de CAMV et PotP1-II.

4. Polynucléotide selon l'une quelconque des revendications 1 à 3, dans lequel la séquence qui code pour la carotène-désaturase et la séquence qui code pour la phytoène-synthase comprennent en outre une séquence codant pour une séquence de ciblage des plastides.

5. Polynucléotide selon l'une quelconque des revendications 1 à 4, qui comprend une séquence choisi dans le groupe représenté par SEQ ID NO: 1: 2; 3; 4 et 6.

6. Séquence de polynucléotide qui est le complément d'une séquence qui s'hybride à un polynucléotide selon la revendication 5 à une température d'environ 65°C dans une solution contenant du SSC 6x, 0,01 % de SDS, et 0,25 % de lait écrémé en poudre, puis rinçage à la même température dans une solution contenant du SSC 0,2× et 0,1 % de SDS, où ladite séquence de polynucléotide comprend encore une région codant pour une carotène-désaturase et une autre région codant pour une phytoène-synthase, et où, lorsque ladite séquence de polynucléotide est insérée dans du matériel végétal, la graine d'une plante régénérée à partir dudit matériel produit des caroténoïdes en une quantité d'au moins 10 µg/g d'endosperme de ladite graine.

7. Séquence de polynucléotide qui est le complément d'une séquence qui s'hybride à un polynucléotide selon la revendication 5 à une température d'environ 65°C dans une solution contenant du SSC 6× 0,01 % de SDS, et 0,25 % de lait écrémé en poudre, puis rinçage à la même température dans une solution contenant SSC 0,2× et 0,1 % de SDS, où ladite séquence de polynucléotide comprend encore une région codant pour une carotène-désaturase et une autre région codant pour une phytoène-synthase, et où, lorsque ladite séquence de polynucléotide est insérée dans du matériel végétal, la graine d'une plante régénérée à partir dudit matériel produit des caroténoïdes en une quantité d'au moins 80 % de la teneur en caroténoïdes d'une graine qui comprend un polynucléotide choisi dans le groupe représenté par SEQ ID NO: 1; 2; 3; 4; 5 et 6.

8. Séquence de polynucléotide selon l'une quelconque des revendications 6 à 7, où ladite graine est une graine de riz.

9. Polynucléotide ou séquence de polynucléotide selon l'une quelconque des revendications 1 à 8, qui comprend en outre une région qui code pour un marqueur sélectionnable.

10. Polynucléotide ou séquence de polynucléotide selon la revendication 9, dans lequel ledit marqueur sélectionnable comprend un gène de mannose-6-phosphate-isomérase.

11. Polynucléotide ou séquence de polynucléotide selon l'une quelconque des revendications 1 à 10, dont les codons sont optimisés pour l'expression dans une espèce végétale particulière.

12. Polynucléotide ou séquence de polynucléotide selon la revendication 11, où ladite espèce végétale est le riz (*Oryza sp.*)*.*

13. Vecteur comprenant un polynucléotide ou une séquence de polynucléotide selon l'une quelconque des revendications 1 à 12.

14. Procédé pour augmenter la teneur en caroténoïdes de graines, comprenant l'insertion dans du matériel végétal d'un polynucléotide ou d'une séquence de polynucléotide selon l'une quelconque des revendications 1 à 12 ou d'un vecteur selon la revendication 13; et la régénération d'une plante contenant des graines à partir dudit matériel végétal et l'identification des graines qui contiennent des caroténoïdes à des niveaux supérieurs à ceux de graines témoins.

15. Procédé pour augmenter la teneur en caroténoïdes d'une graine, comprenant l'insertion dans du matériel végétal d'un polynucléotide comprenant une séquence choisie dans le groupe représenté par SEQ ID NO: 1; 2; 3; 4; 5 et 6, et la régénération d'une plante contenant des graines à partir dudit matériel, et l'identification des graines qui contiennent des caroténoïdes à des niveaux supérieurs à ceux d'une graine témoin.

16. Procédé selon la revendication 14 ou la revendication 15, dans lequel ladite graine contient au moins 60 fois plus de caroténoïdes par comparaison à une graine témoin.

17. Procédé selon la revendication 14 ou la revendication 15, dans lequel ladite graine contient des caroténoïdes en une quantité d'au moins 10 µg/g d'endosperme de ladite graine.

18. Procédé selon l'une quelconque des revendications 14 à 17, dans lequel lesdits caroténoïdes sont choisis dans le groupe constitué par le lycopène; l'α-carotène; la lutéine; le β-carotène; la zéanthine; l'anthéraxanthine; la violaxanthine; et la néoxanthine ou une de leurs combinaisons.

19. Plante ou matériel végétal qui comprend un polynucléotide ou une séquence de polynucléotide selon l'une quelconque des revendications 1 à 12 ou un vecteur selon la revendication 13.

20. Plante ou matériel végétal selon la revendication 19, qui est une plante de riz ou du matériel végétal de riz.

21. Plante ou matériel végétal selon la revendication 19, qui est une plante de maïs ou du matériel végétal de maïs.

22. Utilisation d'un polynucléotide ou d'une séquence de polynucléotide selon l'une quelconque des revendications 1 à 12 ou d'un vecteur selon la revendication 13 dans un procédé de production de graines contenant des quantités accrues de caroténoïdes.

23. Utilisation d'un polynucléotide choisi dans le groupe représenté par SEQ ID NO: 1; 2; 3; 4; 5 et 6 pour la production d'une graine qui contient des caroténoïdes à des niveaux plus élevés que ceux d'une graine témoin.


### SEQUENCE LISTING

<110> SYNGENTA LIMITED
<120> ENHANCED ACCUMULATION OF CAROTENOIDS IN PLANTS
<130> 70237/WO
<150> US60/457,053
   <151> 2003-03-24
<160> 38
<170> PatentIn version 3.1
<210> 1
   <211> 5630
   <212> DNA
   <213> artificial sequence
<220>
   <223> SYNTHETIC - 12423
<400> 1
   gttaatcatg gtgtaggcaa cccaaataaa acaccaaaat atgcacaagg cagtttgttg      60
   tattctgtag tacagacaaa actaaaagta atgaaagaag atgtggtgtt agaaaaggaa     120
   acaatatcat gagtaatgtg tgagcattat gggaccacga aataaaaaga acattttgat     180
   gagtcgtgta tcctcgatga gcctcaaaag ttctctcacc ccggataaga aacccttaag     240
   caatgtgcaa agtttgcatt ctccactgac ataatgcaaa ataagatatc atcgatgaca     300
   tagcaactca tgcatcatat catgcctctc tcaacctatt cattcctact catctacata     360
   agtatcttca gctaaatgtt agaacataaa cccataagtc acgtttgatg agtattaggc     420
   gtgacacatg acaaatcaca gactcaagca agataaagca aaatgatgtg tacataaaac     480
   tccagagcta tatgtcatat tgcaaaaaga ggagagctta taagacaagg catgactcac 540
   aaaaattcat ttgcctttcg tgtcaaaaag aggagggctt tacattatcc atgtcatatt 600
   gcaaaagaaa gagagaaaga acaacacaat gctgcgtcaa ttatacatat ctgtatgtcc 660
   atcattattc atccaccttt cgtgtaccac acttcatata tcatgagtca cttcatgtct 720
   ggacattaac aaactctatc ttaacattta gatgcaagag cctttatctc actataaatg 780
   cacgatgatt tctcattgtt tctcacaaaa agcattcagt tcattagtcc tacaacaacg 840
   aattcggctt cccgggtaca gggtaaattt ctagtttttc tccttcattt tcttggttag 900
   gacccttttc tctttttatt tttttgagct ttgatctttc tttaaactga tctatttttt 960
   aattgattgg ttatcgtgta aatattacat agctttaact gataatctga ttactttatt 1020
   tcgtgtgtct ttgatcatct tgatagttac agaaccgtcg actctagaga agccatttaa 1080
   atcgccgcca ccatggcttc tatgatatcc tcttccgctg tgacaacagt cagccgtgcc 1140
   tctagggggc aatccgccgc agtggctcca ttcggcggcc tcaaatccat gactggattc 1200
   ccagtgaaga aggtcaacac tgacattact tccattacaa gcaatggtgg aagagtaaag 1260
   tgcatgaaac caactacggt aattggtgca ggcttcggtg gcctggcact ggcaattcgt 1320
   ctacaagctg cggggatccc cgtcttactg cttgaacaac gtgataaacc cggcggtcgg 1380
   gcttatgtct acgaggatca ggggtttacc tttgatgcag gcccgacggt tatcaccgat 1440
   cccagtgcca ttgaagaact gtttgcactg gcaggaaaac agttaaaaga gtatgtcgaa 1500
   ctgctgccgg ttacgccgtt ttaccgcctg tgttgggagt cagggaaggt ctttaattac 1560
   gataacgatc aaacccggct cgaagcgcag attcagcagt ttaatccccg cgatgtcgaa 1620
   ggttatcgtc agtttctgga ctattcacgc gcggtgttta aagaaggcta tctgaagctc 1680
   ggtactgtcc cttttttatc gttcagagac atgcttcgcg ccgcacctca actggcgaaa 1740
   ctgcaggcat ggagaagcgt ttacagtaag gttgccagtt acatcgaaga tgaacatctg 1800
   cgccaggcgt tttctttcca ctcgctgttg gtgggcggca atcccttcgc cacctcatcc 1860
   atttatacgt tgatacacgc gctggagcgt gagtggggcg tctggtttcc gcgtggcggc 1920
   accggcgcat tagttcaggg gatgataaag ctgtttcagg atctgggtgg cgaagtcgtg 1980
   ttaaacgcca gagtcagcca tatggaaacg acaggaaaca agattgaagc cgtgcattta 2040
   gaggacggtc gcaggttcct gacgcaagcc gtcgcgtcaa atgcagatgt ggttcatacc 2100
   tatcgcgacc tgttaagcca gcaccctgcc gcggttaagc agtccaacaa actgcagact 2160
   aagcgcatga gtaactctct gtttgtgctc tattttggtt tgaatcacca tcatgatcag 2220
   ctcgcgcatc acacggtttg tttcggcccg cgttaccgcg agctgattga cgaaattttt 2280
   aatcatgatg gcctcgcaga ggacttctca ctttatctgc acgcgccctg tgtcacggat 2340
   tcgtcactgg cgcctgaagg ttgcggcagt tactatgtgt tggcgccggt gccgcattta 2400
   ggcaccgcga acctcgactg gacggttgag gggccaaaac tacgcgaccg tatttttgcg 2460
   taccttgagc agcattacat gcctggctta cggagtcagc tggtcacgca ccggatgttt 2520
   acgccgtttg attttcgcga ccagcttaat gcctatcatg gctcagcctt ttctgtggag 2580
   cccgttctta cccagagcgc ctggtttcgg ccgcataacc gcgataaaac cattactaat 2640
   ctctacctgg tcggcgcagg cacgcatccc ggcgcaggca ttcctggcgt catcggctcg 2700
   gcaaaagcga cagcaggttt gatgctggag gatctgattt gaggccatgc aggccgatcc 2760
   ccgatcgttc aaacatttgg caataaagtt tcttaagatt gaatcctgtt gccggtcttg 2820
   cgatgattat catataattt ctgttgaatt acgttaagca tgtaataatt aacatgtaat 2880
   gcatgacgtt atttatgaga tgggttttta tgattagagt cccgcaatta tacatttaat 2940
   acgcgataga aaacaaaata tagcgcgcaa actaggataa attatcgcgc gcggtgtcat 3000
   ctatgttact agatcgggcc ttaataagct tgttaatcat ggtgtaggca acccaaataa 3060
   aacaccaaaa tatgcacaag gcagtttgtt gtattctgta gtacagacaa aactaaaagt 3120
   aatgaaagaa gatgtggtgt tagaaaagga aacaatatca tgagtaatgt gtgagcatta 3180
   tgggaccacg aaataaaaag aacattttga tgagtcgtgt atcctcgatg agcctcaaaa 3240
   gttctctcac cccggataag aaacccttaa gcaatgtgca aagtttgcat tctccactga 3300
   cataatgcaa aataagatat catcgatgac atagcaactc atgcatcata tcatgcctct 3360
   ctcaacctat tcattcctac tcatctacat aagtatcttc agctaaatgt tagaacataa 3420
   acccataagt cacgtttgat gagtattagg cgtgacacat gacaaatcac agactcaagc 3480
   aagataaagc aaaatgatgt gtacataaaa ctccagagct atatgtcata ttgcaaaaag 3540
   aggagagctt ataagacaag gcatgactca caaaaattca tttgcctttc gtgtcaaaaa 3600
   gaggagggct ttacattatc catgtcatat tgcaaaagaa agagagaaag aacaacacaa 3660
   tgctgcgtca attatacata tctgtatgtc catcattatt catccacctt tcgtgtacca 3720
   cacttcatat atcatgagtc acttcatgtc tggacattaa caaactctat cttaacattt 3780
   agatgcaaga gcctttatct cactataaat gcacgatgat ttctcattgt ttctcacaaa 3840
   aagcattcag ttcattagtc ctacaacaac gaattcggct tcccgggtac agggtaaatt 3900
   tctagttttt ctccttcatt ttcttggtta ggaccctttt ctctttttat ttttttgagc 3960
   tttgatcttt ctttaaactg atctattttt taattgattg gttatcgtgt aaatattaca 4020
   tagctttaac tgataatctg attactttat ttcgtgtgtc tttgatcatc ttgatagtta 4080
   cagaaccgtc gactctagag aagccattta aatcgccgcc accatggcca tcatactcgt 4140
   acgagcagcg tcgccggggc tctccgccgc cgacagcatc agccaccagg ggactctcca    4200
   gtgctccacc ctgctcaaga cgaagaggcc ggcggcgcgg cggtggatgc cctgctcgct    4260
   ccttggcctc cacccgtggg aggctggccg tccctccccc gccgtctact ccagcctgcc    4320
   cgtcaacccg gcgggagagg ccgtcgtctc gtccgagcag aaggtctacg acgtcgtgct    4380
   caagcaggcc gcattgctca aacgccagct gcgcacgccg gtcctcgacg ccaggcccca    4440
   ggacatggac atgccacgca acgggctcaa ggaagcctac gaccgctgcg gcgagatctg    4500
   tgaggagtat gccaagacgt tttacctcgg aactatgttg atgacagagg agcggcgccg    4560
   cgccatatgg gccatctatg tgtggtgtag gaggacagat gagcttgtag atgggccaaa    4620
   cgccaactac attacaccaa cagctttgga ccggtgggag aagagacttg aggatctgtt    4680
   cacgggacgt ccttacgaca tgcttgatgc cgctctctct gataccatct caaggttccc    4740
   catagacatt cagccattca gggacatgat tgaagggatg aggagtgatc ttaggaagac    4800
   aaggtataac aacttcgacg agctctacat gtactgctac tatgttgctg gaactgtcgg    4860
   gttaatgagc gtacctgtga tgggcatcgc aaccgagtct aaagcaacaa ctgaaagcgt    4920
   atacagtgct gccttggctc tgggaattgc gaaccaactc acgaacatac tccgggatgt    4980
   tggagaggat gctagaagag gaaggatata tttaccacaa gatgagcttg cacaggcagg    5040
   gctctctgat gaggacatct tcaaaggggt cgtcacgaac cggtggagaa acttcatgaa    5100
   gaggcagatc aagagggcca ggatgttttt tgaggaggca gagagagggg taactgagct    5160
   ctcacaggct agcagatggc cagtatgggc ttccctgttg ttgtacaggc agatcctgga    5220
   tgagatcgaa gccaacgact acaacaactt cacgaagagg gcgtatgttg gtaaagggaa    5280
   gaagttgcta gcacttcctg tggcatatgg aaaatcgcta ctgctcccat gttcattgag    5340
   aaatggccag acctagggcc atgcaggccg atccccgatc gttcaaacat ttggcaataa    5400
   agtttcttaa gattgaatcc tgttgccggt cttgcgatga ttatcatata atttctgttg    5460
   aattacgtta agcatgtaat aattaacatg taatgcatga cgttatttat gagatgggtt    5520
   tttatgatta gagtcccgca attatacatt taatacgcga tagaaaacaa aatatagcgc    5580
   gcaaactagg ataaattatc gcgcgcggtg tcatctatgt tactagatcg                5630
<210> 2
   <211> 5630
   <212> DNA
   <213> artificial sequence
<220>
<223> SYNTHETIC - 12421
<400> 2
   gttaatcatg gtgtaggcaa cccaaataaa acaccaaaat atgcacaagg cagtttgttg      60
   tattctgtag tacagacaaa actaaaagta atgaaagaag atgtggtgtt agaaaaggaa     120
   acaatatcat gagtaatgtg tgagcattat gggaccacga aataaaaaga acattttgat     180
   gagtcgtgta tcctcgatga gcctcaaaag ttctctcacc ccggataaga aacccttaag     240
   caatgtgcaa agtttgcatt ctccactgac ataatgcaaa ataagatatc atcgatgaca     300
   tagcaactca tgcatcatat catgcctctc tcaacctatt cattcctact catctacata     360
   agtatcttca gctaaatgtt agaacataaa cccataagtc acgtttgatg agtattaggc     420
   gtgacacatg acaaatcaca gactcaagca agataaagca aaatgatgtg tacataaaac     480
   tccagagcta tatgtcatat tgcaaaaaga ggagagctta taagacaagg catgactcac     540
   aaaaattcat ttgcctttcg tgtcaaaaag aggagggctt tacattatcc atgtcatatt     600
   gcaaaagaaa gagagaaaga acaacacaat gctgcgtcaa ttatacatat ctgtatgtcc     660
   atcattattc atccaccttt cgtgtaccac acttcatata tcatgagtca cttcatgtct     720
   ggacattaac aaactctatc ttaacattta gatgcaagag cctttatctc actataaatg     780
   cacgatgatt tctcattgtt tctcacaaaa agcattcagt tcattagtcc tacaacaacg     840
   aattcggctt cccgggtaca gggtaaattt ctagtttttc tccttcattt tcttggttag     900
   gacccttttc tctttttatt tttttgagct ttgatctttc tttaaactga tctatttttt     960
   aattgattgg ttatcgtgta aatattacat agctttaact gataatctga ttactttatt    1020
   tcgtgtgtct ttgatcatct tgatagttac agaaccgtcg actctagaga agccatttaa    1080
   atcgccgcca ccatggcttc tatgatatcc tcttccgctg tgacaacagt cagccgtgcc    1140
   tctagggggc aatccgccgc agtggctcca ttcggcggcc tcaaatccat gactggattc    1200
   ccagtgaaga aggtcaacac tgacattact tccattacaa gcaatggtgg aagagtaaag    1260
   tgcatgaaac caactacggt aattggtgca ggcttcggtg gcctggcact ggcaattcgt    1320
   ctacaagctg cggggatccc cgtcttactg cttgaacaac gtgataaacc cggcggtcgg    1380
   gcttatgtct acgaggatca ggggtttacc tttgatgcag gcccgacggt tatcaccgat    1440
   cccagtgcca ttgaagaact gtttgcactg gcaggaaaac agttaaaaga gtatgtcgaa    1500
   ctgctgccgg ttacgccgtt ttaccgcctg tgttgggagt cagggaaggt ctttaattac    1560
   gataacgatc aaacccggct cgaagcgcag attcagcagt ttaatccccg cgatgtcgaa    1620
   ggttatcgtc agtttctgga ctattcacgc gcggtgttta aagaaggcta tctgaagctc    1680
   ggtactgtcc cttttttatc gttcagagac atgcttcgcg ccgcacctca actggcgaaa 1740
   ctgcaggcat ggagaagcgt ttacagtaag gttgccagtt acatcgaaga tgaacatctg 1800
   cgccaggcgt tttctttcca ctcgctgttg gtgggcggca atcccttcgc cacctcatcc 1860
   atttatacgt tgatacacgc gctggagcgt gagtggggcg tctggtttcc gcgtggcggc 1920
   accggcgcat tagttcaggg gatgataaag ctgtttcagg atctgggtgg cgaagtcgtg 1980
   ttaaacgcca gagtcagcca tatggaaacg acaggaaaca agattgaagc cgtgcattta 2040
   gaggacggtc gcaggttcct gacgcaagcc gtcgcgtcaa atgcagatgt ggttcatacc 2100
   tatcgcgacc tgttaagcca gcaccctgcc gcggttaagc agtccaacaa actgcagact 2160
   aagcgcatga gtaactctct gtttgtgctc tattttggtt tgaatcacca tcatgatcag 2220
   ctcgcgcatc acacggtttg tttcggcccg cgttaccgcg agctgattga cgaaattttt 2280
   aatcatgatg gcctcgcaga ggacttctca ctttatctgc acgcgccctg tgtcacggat 2340
   tcgtcactgg cgcctgaagg ttgcggcagt tactatgtgt tggcgccggt gccgcattta 2400
   ggcaccgcga acctcgactg gacggttgag gggccaaaac tacgcgaccg tatttttgcg 2460
   taccttgagc agcattacat gcctggctta cggagtcagc tggtcacgca ccggatgttt 2520
   acgccgtttg attttcgcga ccagcttaat gcctatcatg gctcagcctt ttctgtggag 2580
   cccgttctta cccagagcgc ctggtttcgg ccgcataacc gcgataaaac cattactaat 2640
   ctctacctgg tcggcgcagg cacgcatccc ggcgcaggca ttcctggcgt catcggctcg 2700
   gcaaaagcga cagcaggttt gatgctggag gatctgattt gaggccatgc aggccgatcc 2760
   ccgatcgttc aaacatttgg caataaagtt tcttaagatt gaatcctgtt gccggtcttg 2820
   cgatgattat catataattt ctgttgaatt acgttaagca tgtaataatt aacatgtaat 2880
   gcatgacgtt atttatgaga tgggttttta tgattagagt cccgcaatta tacatttaat    2940
   acgcgataga aaacaaaata tagcgcgcaa actaggataa attatcgcgc gcggtgtcat 3000
   ctatgttact agatcgggcc ttaataagct tgttaatcat ggtgtaggca acccaaataa 3060
   aacaccaaaa tatgcacaag gcagtttgtt gtattctgta gtacagacaa aactaaaagt 3120
   aatgaaagaa gatgtggtgt tagaaaagga aacaatatca tgagtaatgt gtgagcatta 3180
   tgggaccacg aaataaaaag aacattttga tgagtcgtgt atcctcgatg agcctcaaaa 3240
   gttctctcac cccggataag aaacccttaa gcaatgtgca aagtttgcat tctccactga 3300
   cataatgcaa aataagatat catcgatgac atagcaactc atgcatcata tcatgcctct 3360
   ctcaacctat tcattcctac tcatctacat aagtatcttc agctaaatgt tagaacataa 3420
   acccataagt cacgtttgat gagtattagg cgtgacacat gacaaatcac agactcaagc 3480
   aagataaagc aaaatgatgt gtacataaaa ctccagagct atatgtcata ttgcaaaaag 3540
   aggagagctt ataagacaag gcatgactca caaaaattca tttgcctttc gtgtcaaaaa 3600
   gaggagggct ttacattatc catgtcatat tgcaaaagaa agagagaaag aacaacacaa 3660
   tgctgcgtca attatacata tctgtatgtc catcattatt catccacctt tcgtgtacca 3720
   cacttcatat atcatgagtc acttcatgtc tggacattaa caaactctat cttaacattt 3780
   agatgcaaga gcctttatct cactataaat gcacgatgat ttctcattgt ttctcacaaa 3840
   aagcattcag ttcattagtc ctacaacaac gaattcggct tcccgggtac agggtaaatt 3900
   tctagttttt ctccttcatt ttcttggtta ggaccctttt ctctttttat ttttttgagc 3960
   tttgatcttt ctttaaactg atctattttt taattgattg gttatcgtgt aaatattaca 4020
   tagctttaac tgataatctg attactttat ttcgtgtgtc tttgatcatc ttgatagtta 4080
   cagaaccgtc gactctagag aagccattta aatcgccgcc accatggcca tcatactcgt 4140
   acgagcagcg tcgccggggc tctccgccgc cgacagcatc agccaccagg ggactctcca 4200
   gtgctccacc ctgctcaaga cgaagaggcc ggcggcgcgc cggtggatgc cctgctcgct 4260
   ccttggcctc cacccgtggg aggctggccg tccctccccc gccgtctact ccagcctcgc 4320
   cgtcaacccg gcgggagagg ccgtcgtctc gtccgagcag aaggtctacg acgtcgtgct 4380
   caagcaggcc gcattgctca aacgccagct gcgcacgccg gtcctcgacg ccaggcccca 4440
   ggacatggac atgccacgca acgggctcaa ggaagcctac gaccgctgcg gcgagatctg 4500
   tgaggagtat gccaagacgt tttacctcgg aactatgttg atgacagagg agcggcgccg 4560
   cgccatatgg gccatctatg tgtggtgtag gaggacagat gagcttgtag atgggccaaa 4620
   cgccaactac attacaccaa cagctttgga ccggtgggag aagagacttg aggatctgtt 4680
   cacgggacgt ccttacgaca tgcttgatgc cgctctctct gataccatct caaggttccc 4740
   catagacatt cagccattca gggacatgat tgaagggatg aggagtgatc ttaggaagac 4800
   aaggtataac aacttcgacg agctctacat gtactgctac tatgttgctg gaactgtcgg 4860
   gttaatgagc gtaccagtga tgggcatcgc atccgagtct aaagcaacaa ctgaaagcgt 4920
   gtacagtgct gccttggctc tcggaattgc gaaccaactc acgaacatac tccgggatgt 4980
   tggagaggat gctagacgag gaaggatata tttaccacaa gatgagcttg cacaggcagg 5040
   gctctctgat gaggacatct tcaaaggggt cgtcacgaac cggtggagaa acttcatgaa 5100
   gaggcagatc aagagggcca ggatgttttt tgaggaggca gagagagggg taactgagct 5160
   ctcacaggct agcagatggc cagtatgggc ttccctgttg ttgtacaggc agatcctgga 5220
   tgagatcgaa gccaacgact acaacaactt cacgaagagg gcgtatgttg gtaaagggaa 5280
   gaagttgcta gcacttcctg tggcatatgg aaaatcgcta ctgctcccat gttcattgag 5340
   aaatggccag acctagggcc atgcaggccg atccccgatc gttcaaacat ttggcaataa 5400
   agtttcttaa gattgaatcc tgttgccggt cttgcgatga ttatcatata atttctgttg 5460
   aattacgtta agcatgtaat aattaacatg taatgcatga cgttatttat gagatgggtt 5520
   tttatgatta gagtcccgca attatacatt taatacgcga tagaaaacaa aatatagcgc 5580
   gcaaactagg ataaattatc gcgcgcggtg tcatctatgt tactagatcg 5630
<210> 3
   <211> 5180
   <212> DNA
   <213> artificial sequence
<220>
   <223> SYNTHETIC - 12422
<400> 3
   gttaatcatg gtgtaggcaa cccaaataaa acaccaaaat atgcacaagg cagtttgttg 60
   tattctgtag tacagacaaa actaaaagta atgaaagaag atgtggtgtt agaaaaggaa 120
   acaatatcat gagtaatgtg tgagcattat gggaccacga aataaaaaga acattttgat 180
   gagtcgtgta tcctcgatga gcctcaaaag ttctctcacc ccggataaga aacccttaag 240
   caatgtgcaa agtttgcatt ctccactgac ataatgcaaa ataagatatc atcgatgaca 300
   tagcaactca tgcatcatat catgcctctc tcaacctatt cattcctact catctacata 360
   agtatcttca gctaaatgtt agaacataaa cccataagtc acgtttgatg agtattaggc 420
   gtgacacatg acaaatcaca gactcaagca agataaagca aaatgatgtg tacataaaac 480
   tccagagcta tatgtcatat tgcaaaaaga ggagagctta taagacaagg catgactcac 540
   aaaaattcat ttgcctttcg tgtcaaaaag aggagggctt tacattatcc atgtcatatt 600
   gcaaaagaaa gagagaaaga acaacacaat gctgcgtcaa ttatacatat ctgtatgtcc 660
   atcattattc atccaccttt cgtgtaccac acttcatata tcatgagtca cttcatgtct 720
   ggacattaac aaactctatc ttaacattta gatgcaagag cctttatctc actataaatg 780
   cacgatgatt tctcattgtt tctcacaaaa agcattcagt tcattagtcc tacaacaacg 840
   aattcggctt cccaaatcgc cgccaccatg gcttctatga tatcctcttc cgctgtgaca 900
   acagtcagcc gtgcctctag ggggcaatcc gccgcagtgg ctccattcgg cggcctcaaa 960
   tccatgactg gattcccagt gaagaaggtc aacactgaca ttacttccat tacaagcaat 1020
   ggtggaagag taaagtgcat gaaaccaact acggtaattg gtgcaggctt cggtggcctg 1080
   gcactggcaa ttcgtctaca agctgcgggg atccccgtct tactgcttga acaacgtgat 1140
   aaacccggcg gtcgggctta tgtctacgag gatcaggggt ttacctttga tgcaggcccg 1200
   acggttatca ccgatcccag tgccattgaa gaactgtttg cactggcagg aaaacagtta 1260
   aaagagtatg tcgaactgct gccggttacg ccgttttacc gcctgtgttg ggagtcaggg 1320
   aaggtcttta attacgataa cgatcaaacc cggctcgaag cgcagattca gcagtttaat 1380
   ccccgcgatg tcgaaggtta tcgtcagttt ctggactatt cacgcgcggt gtttaaagaa 1440
   ggctatctga agctcggtac tgtccctttt ttatcgttca gagacatgct tcgcgccgca 1500
   cctcaactgg cgaaactgca ggcatggaga agcgtttaca gtaaggttgc cagttacatc 1560
   gaagatgaac atctgcgcca ggcgttttct ttccactcgc tgttggtggg cggcaatccc 1620
   ttcgccacct catccattta tacgttgata cacgcgctgg agcgtgagtg gggcgtctgg 1680
   tttccgcgtg gcggcaccgg cgcattagtt caggggatga taaagctgtt tcaggatctg 1740
   ggtggcgaag tcgtgttaaa cgccagagtc agccatatgg aaacgacagg aaacaagatt 1800
   gaagccgtgc atttagagga cggtcgcagg ttcctgacgc aagccgtcgc gtcaaatgca 1860
   gatgtggttc atacctatcg cgacctgtta agccagcacc ctgccgcggt taagcagtcc 1920
   aacaaactgc agactaagcg catgagtaac tctctgtttg tgctctattt tggtttgaat 1980
   caccatcatg atcagctcgc gcatcacacg gtttgtttcg gcccgcgtta ccgcgagctg 2040
   attgacgaaa tttttaatca tgatggcctc gcagaggact tctcacttta tctgcacgcg 2100
   ccctgtgtca cggattcgtc actggcgcct gaaggttgcg gcagttacta tgtgttggcg 2160
   ccggtgccgc atttaggcac cgcgaacctc gactggacgg ttgaggggcc aaaactacgc 2220
   gaccgtattt ttgcgtacct tgagcagcat tacatgcctg gcttacggag tcagctggtc 2280
   acgcaccgga tgtttacgcc gtttgatttt cgcgaccagc ttaatgccta tcatggctca 2340
   gccttttctg tggagcccgt tcttacccag agcgcctggt ttcggccgca taaccgcgat 2400
   aaaaccatta ctaatctcta cctggtcggc gcaggcacgc atcccggcgc aggcattcct 2460
   ggcgtcatcg gctcggcaaa agcgacagca ggtttgatgc tggaggatct gatttgaggc 2520
   catgcaggcc gatccccgat cgttcaaaca tttggcaata aagtttctta agattgaatc 2580
   ctgttgccgg tcttgcgatg attatcatat aatttctgtt gaattacgtt aagcatgtaa 2640
   taattaacat gtaatgcatg acgttattta tgagatgggt ttttatgatt agagtcccgc 2700
   aattatacat ttaatacgcg atagaaaaca aaatatagcg cgcaaactag gataaattat 2760
   cgcgcgcggt gtcatctatg ttactagatc gggccttaat aagcttgtta atcatggtgt 2820
   aggcaaccca aataaaacac caaaatatgc acaaggcagt ttgttgtatt ctgtagtaca 2880
   gacaaaacta aaagtaatga aagaagatgt ggtgttagaa aaggaaacaa tatcatgagt 2940
   aatgtgtgag cattatggga ccacgaaata aaaagaacat tttgatgagt cgtgtatcct 3000
   cgatgagcct caaaagttct ctcaccccgg ataagaaacc cttaagcaat gtgcaaagtt 3060
   tgcattctcc actgacataa tgcaaaataa gatatcatcg atgacatagc aactcatgca 3120
   tcatatcatg cctctctcaa cctattcatt cctactcatc tacataagta tcttcagcta 3180
   aatgttagaa cataaaccca taagtcacgt ttgatgagta ttaggcgtga cacatgacaa 3240
   atcacagact caagcaagat aaagcaaaat gatgtgtaca taaaactcca gagctatatg 3300
   tcatattgca aaaagaggag agcttataag acaaggcatg actcacaaaa attcatttgc 3360
   ctttcgtgtc aaaaagagga gggctttaca ttatccatgt catattgcaa aagaaagaga 3420
   gaaagaacaa cacaatgctg cgtcaattat acatatctgt atgtccatca ttattcatcc 3480
   acctttcgtg taccacactt catatatcat gagtcacttc atgtctggac attaacaaac 3540
   tctatcttaa catttagatg caagagcctt tatctcacta taaatgcacg atgatttctc 3600
   attgtttctc acaaaaagca ttcagttcat tagtcctaca acaacgaatt cggcttccca 3660
   aatcgccgcc accatggcca tcatactcgt acgagcagcg tcgccggggc tctccgccgc 3720
   cgacagcatc agccaccagg ggactctcca gtgctccacc ctgctcaaga cgaagaggcc 3780
   ggcggcgcgc cggtggatgc cctgctcgct ccttggcctc cacccgtggg aggctggccg 3840
   tccctccccc gccgtctact ccagcctcgc cgtcaacccg gcgggagagg ccgtcgtctc 3900
   gtccgagcag aaggtctacg acgtcgtgct caagcaggcc gcattgctca aacgccagct 3960
   gcgcacgccg gtcctcgacg ccaggcccca ggacatggac atgccacgca acgggctcaa 4020
   ggaagcctac gaccgctgcg gcgagatctg tgaggagtat gccaagacgt tttacctcgg 4080
   aactatgttg atgacagagg agcggcgccg cgccatatgg gccatctatg tgtggtgtag 4140
   gaggacagat gagcttgtag atgggccaaa cgccaactac attacaccaa cagctttgga 4200
   ccggtgggag aagagacttg aggatctgtt cacgggacgt ccttacgaca tgcttgatgc 4260
   cgctctctct gataccatct caaggttccc catagacatt cagccattca gggacatgat 4320
   tgaagggatg aggagtgatc ttaggaagac aaggtataac aacttcgacg agctctacat 4380
   gtactgctac tatgttgctg gaactgtcgg gttaatgagc gtaccagtga tgggcatcgc 4440
   atccgagtct aaagcaacaa ctgaaagcgt gtacagtgct gccttggctc tcggaattgc 4500
   gaaccaactc acgaacatac tccgggatgt tggagaggat gctagacgag gaaggatata 4560
   tttaccacaa gatgagcttg cacaggcagg gctctctgat gaggacatct tcaaaggggt 4620
   cgtcacgaac cggtggagaa acttcatgaa gaggcagatc aagagggcca ggatgttttt 4680
   tgaggaggca gagagagggg taactgagct ctcacaggct agcagatggc cagtatgggc 4740
   ttccctgttg ttgtacaggc agatcctgga tgagatcgaa gccaacgact acaacaactt 4800
   cacgaagagg gcgtatgttg gtaaagggaa gaagttgcta gcacttcctg tggcatatgg 4860
   aaaatcgcta ctgctcccat gttcattgag aaatggccag acctagggcc atgcaggccg 4920
   atccccgatc gttcaaacat ttggcaataa agtttcttaa gattgaatcc tgttgccggt 4980
   cttgcgatga ttatcatata atttctgttg aattacgtta agcatgtaat aattaacatg 5040
   taatgcatga cgttatttat gagatgggtt tttatgatta gagtcccgca attatacatt 5100
   taatacgcga tagaaaacaa aatatagcgc gcaaactagg ataaattatc gcgcgcggtg 5160
   tcatctatgt tactagatcg 5180
<210> 4
   <211> 5180
   <212> DNA
   <213> artificial sequence
<220>
   <223> SYNTHETIC - 12424
<400> 4
   gttaatcatg gtgtaggcaa cccaaataaa acaccaaaat atgcacaagg cagtttgttg 60
   tattctgtag tacagacaaa actaaaagta atgaaagaag atgtggtgtt agaaaaggaa 120
   acaatatcat gagtaatgtg tgagcattat gggaccacga aataaaaaga acattttgat 180
   gagtcgtgta tcctcgatga gcctcaaaag ttctctcacc ccggataaga aacccttaag 240
   caatgtgcaa agtttgcatt ctccactgac ataatgcaaa ataagatatc atcgatgaca 300
   tagcaactca tgcatcatat catgcctctc tcaacctatt cattcctact catctacata 360
   agtatcttca gctaaatgtt agaacataaa cccataagtc acgtttgatg agtattaggc 420
   gtgacacatg acaaatcaca gactcaagca agataaagca aaatgatgtg tacataaaac 480
   tccagagcta tatgtcatat tgcaaaaaga ggagagctta taagacaagg catgactcac 540
   aaaaattcat ttgcctttcg tgtcaaaaag aggagggctt tacattatcc atgtcatatt 600
   gcaaaagaaa gagagaaaga acaacacaat gctgcgtcaa ttatacatat ctgtatgtcc 660
   atcattattc atccaccttt cgtgtaccac acttcatata tcatgagtca cttcatgtct 720
   ggacattaac aaactctatc ttaacattta gatgcaagag cctttatctc actataaatg 780
   cacgatgatt tctcattgtt tctcacaaaa agcattcagt tcattagtcc tacaacaacg 840
   aattcggctt cccaaatcgc cgccaccatg gcttctatga tatcctcttc cgctgtgaca 900
   acagtcagcc gtgcctctag ggggcaatcc gccgcagtgg ctccattcgg cggcctcaaa 960
   tccatgactg gattcccagt gaagaaggtc aacactgaca ttacttccat tacaagcaat 1020
   ggtggaagag taaagtgcat gaaaccaact acggtaattg gtgcaggctt cggtggcctg 1080
   gcactggcaa ttcgtctaca agctgcgggg atccccgtct tactgcttga acaacgtgat 1140
   aaacccggcg gtcgggctta tgtctacgag gatcaggggt ttacctttga tgcaggcccg 1200
   acggttatca ccgatcccag tgccattgaa gaactgtttg cactggcagg aaaacagtta 1260
   aaagagtatg tcgaactgct gccggttacg ccgttttacc gcctgtgttg ggagtcaggg 1320
   aaggtcttta attacgataa cgatcaaacc cggctcgaag cgcagattca gcagtttaat 1380
   ccccgcgatg tcgaaggtta tcgtcagttt ctggactatt cacgcgcggt gtttaaagaa 1440
   ggctatctga agctcggtac tgtccctttt ttatcgttca gagacatgct tcgcgccgca 1500
   cctcaactgg cgaaactgca ggcatggaga agcgtttaca gtaaggttgc cagttacatc 1560
   gaagatgaac atctgcgcca ggcgttttct ttccactcgc tgttggtggg cggcaatccc 1620
   ttcgccacct catccattta tacgttgata cacgcgctgg agcgtgagtg gggcgtctgg 1680
   tttccgcgtg gcggcaccgg cgcattagtt caggggatga taaagctgtt tcaggatctg 1740
   ggtggcgaag tcgtgttaaa cgccagagtc agccatatgg aaacgacagg aaacaagatt 1800
   gaagccgtgc atttagagga cggtcgcagg ttcctgacgc aagccgtcgc gtcaaatgca 1860
   gatgtggttc atacctatcg cgacctgtta agccagcacc ctgccgcggt taagcagtcc 1920
   aacaaactgc agactaagcg catgagtaac tctctgtttg tgctctattt tggtttgaat 1980
   caccatcatg atcagctcgc gcatcacacg gtttgtttcg gcccgcgtta ccgcgagctg 2040
   attgacgaaa tttttaatca tgatggcctc gcagaggact tctcacttta tctgcacgcg 2100
   ccctgtgtca cggattcgtc actggcgcct gaaggttgcg gcagttacta tgtgttggcg 2160
   ccggtgccgc atttaggcac cgcgaacctc gactggacgg ttgaggggcc aaaactacgc 2220
   gaccgtattt ttgcgtacct tgagcagcat tacatgcctg gcttacggag tcagctggtc 2280
   acgcaccgga tgtttacgcc gtttgatttt cgcgaccagc ttaatgccta tcatggctca 2340
   gccttttctg tggagcccgt tcttacccag agcgcctggt ttcggccgca taaccgcgat 2400
   aaaaccatta ctaatctcta cctggtcggc gcaggcacgc atcccggcgc aggcattcct 2460
   ggcgtcatcg gctcggcaaa agcgacagca ggtttgatgc tggaggatct gatttgaggc 2520
   catgcaggcc gatccccgat cgttcaaaca tttggcaata aagtttctta agattgaatc 2580
   ctgttgccgg tcttgcgatg attatcatat aatttctgtt gaattacgtt aagcatgtaa 2640
   taattaacat gtaatgcatg acgttattta tgagatgggt ttttatgatt agagtcccgc 2700
   aattatacat ttaatacgcg atagaaaaca aaatatagcg cgcaaactag gataaattat 2760
   cgcgcgcggt gtcatctatg ttactagatc gggccttaat aagcttgtta atcatggtgt 2820
   aggcaaccca aataaaacac caaaatatgc acaaggcagt ttgttgtatt ctgtagtaca 2880
   gacaaaacta aaagtaatga aagaagatgt ggtgttagaa aaggaaacaa tatcatgagt 2940
   aatgtgtgag cattatggga ccacgaaata aaaagaacat tttgatgagt cgtgtatcct 3000
   cgatgagcct caaaagttct ctcaccccgg ataagaaacc cttaagcaat gtgcaaagtt 3060
   tgcattctcc actgacataa tgcaaaataa gatatcatcg atgacatagc aactcatgca 3120
   tcatatcatg cctctctcaa cctattcatt cctactcatc tacataagta tcttcagcta 3180
   aatgttagaa cataaaccca taagtcacgt ttgatgagta ttaggcgtga cacatgacaa 3240
   atcacagact caagcaagat aaagcaaaat gatgtgtaca taaaactcca gagctatatg 3300
   tcatattgca aaaagaggag agcttataag acaaggcatg actcacaaaa attcatttgc 3360
   ctttcgtgtc aaaaagagga gggctttaca ttatccatgt catattgcaa aagaaagaga 3420
   gaaagaacaa cacaatgctg cgtcaattat acatatctgt atgtccatca ttattcatcc 3480
   acctttcgtg taccacactt catatatcat gagtcacttc atgtctggac attaacaaac 3540
   tctatcttaa catttagatg caagagcctt tatctcacta taaatgcacg atgatttctc 3600
   attgtttctc acaaaaagca ttcagttcat tagtcctaca acaacgaatt cggcttccca 3660
   aatcgccgcc accatggcca tcatactcgt acgagcagcg tcgccggggc tctccgccgc 3720
   cgacagcatc agccaccagg ggactctcca gtgctccacc ctgctcaaga cgaagaggcc 3780
   ggcggcgcgg cggtggatgc cctgctcgct ccttggcctc cacccgtggg aggctggccg 3840
   tccctccccc gccgtctact ccagcctgcc cgtcaacccg gcgggagagg ccgtcgtctc 3900
   gtccgagcag aaggtctacg acgtcgtgct caagcaggcc gcattgctca aacgccagct 3960
   gcgcacgccg gtcctcgacg ccaggcccca ggacatggac atgccacgca acgggctcaa 4020
   ggaagcctac gaccgctgcg gcgagatctg tgaggagtat gccaagacgt tttacctcgg 4080
   aactatgttg atgacagagg agcggcgccg cgccatatgg gccatctatg tgtggtgtag 4140
   gaggacagat gagcttgtag atgggccaaa cgccaactac attacaccaa cagctttgga 4200
   ccggtgggag aagagacttg aggatctgtt cacgggacgt ccttacgaca tgcttgatgc 4260
   cgctctctct gataccatct caaggttccc catagacatt cagccattca gggacatgat 4320
   tgaagggatg aggagtgatc ttaggaagac aaggtataac aacttcgacg agctctacat 4380
   gtactgctac tatgttgctg gaactgtcgg gttaatgagc gtacctgtga tgggcatcgc 4440
   aaccgagtct aaagcaacaa ctgaaagcgt atacagtgct gccttggctc tgggaattgc 4500
   gaaccaactc acgaacatac tccgggatgt tggagaggat gctagaagag gaaggatata 4560
   tttaccacaa gatgagcttg cacaggcagg gctctctgat gaggacatct tcaaaggggt 4620
   cgtcacgaac cggtggagaa acttcatgaa gaggcagatc aagagggcca ggatgttttt 4680
   tgaggaggca gagagagggg taactgagct ctcacaggct agcagatggc cagtatgggc 4740
   ttccctgttg ttgtacaggc agatcctgga tgagatcgaa gccaacgact acaacaactt 4800
   cacgaagagg gcgtatgttg gtaaagggaa gaagttgcta gcacttcctg tggcatatgg 4860
   aaaatcgcta ctgctcccat gttcattgag aaatggccag acctagggcc atgcaggccg 4920
   atccccgatc gttcaaacat ttggcaataa agtttcttaa gattgaatcc tgttgccggt 4980
   cttgcgatga ttatcatata atttctgttg aattacgtta agcatgtaat aattaacatg 5040
   taatgcatga cgttatttat gagatgggtt tttatgatta gagtcccgca attatacatt 5100
   taatacgcga tagaaaacaa aatatagcgc gcaaactagg ataaattatc gcgcgcggtg 5160
   tcatctatgt tactagatcg 5180
<210> 5
   <211> 5653
   <212> DNA
   <213> artificial sequence
<220>
   <223> Synthetic
<400> 5
<210> 6
   <211> 5714
   <212> DNA
   <213> artificial sequence
<220>
   <223> SYNTHETIC - 11586
<400> 6
   gttaatcatg gtgtaggcaa cccaaataaa acaccaaaat atgcacaagg cagtttgttg      60
   tattctgtag tacagacaaa actaaaagta atgaaagaag atgtggtgtt agaaaaggaa     120
   acaatatcat gagtaatgtg tgagcattat gggaccacga aataaaaaga acattttgat     180
   gagtcgtgta tcctcgatga gcctcaaaag ttctctcacc ccggataaga aacccttaag     240
   caatgtgcaa agtttgcatt ctccactgac ataatgcaaa ataagatatc atcgatgaca     300
   tagcaactca tgcatcatat catgcctctc tcaacctatt cattcctact catctacata     360
   agtatcttca gctaaatgtt agaacataaa cccataagtc acgtttgatg agtattaggc     420
   gtgacacatg acaaatcaca gactcaagca agataaagca aaatgatgtg tacataaaac     480
   tccagagcta tatgtcatat tgcaaaaaga ggagagctta taagacaagg catgactcac     540
   aaaaattcat ttgcctttcg tgtcaaaaag aggagggctt tacattatcc atgtcatatt     600
   gcaaaagaaa gagagaaaga acaacacaat gctgcgtcaa ttatacatat ctgtatgtcc     660
   atcattattc atccaccttt cgtgtaccac acttcatata tcatgagtca cttcatgtct     720
   ggacattaac aaactctatc ttaacattta gatgcaagag cctttatctc actataaatg     780
   cacgatgatt tctcattgtt tctcacaaaa agcattcagt tcattagtcc tacaacaacg     840
   aattcggctt cccgggtaca gggtaaattt ctagtttttc tccttcattt tcttggttag     900
   gacccttttc tctttttatt tttttgagct ttgatctttc tttaaactga tctatttttt     960
   aattgattgg ttatcgtgta aatattacat agctttaact gataatctga ttactttatt    1020
   tcgtgtgtct ttgatcatct tgatagttac agaaccgtcg actctagaga agccatttaa    1080
   atcgccgcca ccatggcttc tatgatatcc tcttccgctg tgacaacagt cagccgtgcc    1140
   tctagggggc aatccgccgc agtggctcca ttcggcggcc tcaaatccat gactggattc    1200
   ccagtgaaga aggtcaacac tgacattact tccattacaa gcaatggtgg aagagtaaag    1260
   tgcatggcgg ccgccaaacc aactacggta attggtgcag gcttcggtgg cctggcactg    1320
   gcaattcgtc tacaagctgc ggggatcccc gtcttactgc ttgaacaacg tgataaaccc    1380
   ggcggtcggg cttatgtcta cgaggatcag gggtttacct ttgatgcagg cccgacggtt    1440
   atcaccgatc ccagtgccat tgaagaactg tttgcactgg caggaaaaca gttaaaagag    1500
   tatgtcgaac tgctgccggt tacgccgttt taccgcctgt gttgggagtc agggaaggtc    1560
   tttaattacg ataacgatca aacccggctc gaagcgcaga ttcagcagtt taatccccgc    1620
   gatgtcgaag gttatcgtca gtttctggac tattcacgcg cggtgtttaa agaaggctat    1680
   ctgaagctcg gtactgtccc ttttttatcg ttcagagaca tgcttcgcgc cgcacctcaa    1740
   ctggcgaaac tgcaggcatg gagaagcgtt tacagtaagg ttgccagtta catcgaagat    1800
   gaacatctgc gccaggcgtt ttctttccac tcgctgttgg tgggcggcaa tcccttcgcc 1860
   acctcatcca tttatacgtt gatacacgcg ctggagcgtg agtggggcgt ctggtttccg 1920
   cgtggcggca ccggcgcatt agttcagggg atgataaagc tgtttcagga tctgggtggc 1980
   gaagtcgtgt taaacgccag agtcagccat atggaaacga caggaaacaa gattgaagcc 2040
   gtgcatttag aggacggtcg caggttcctg acgcaagccg tcgcgtcaaa tgcagatgtg 2100
   gttcatacct atcgcgacct gttaagccag caccctgccg cggttaagca gtccaacaaa 2160
   ctgcagacta agcgcatgag taactctctg tttgtgctct attttggttt gaatcaccat 2220
   catgatcagc tcgcgcatca cacggtttgt ttcggcccgc gttaccgcga gctgattgac 2280
   gaaattttta atcatgatgg cctcgcagag gacttctcac tttatctgca cgcgccctgt 2340
   gtcacggatt cgtcactggc gcctgaaggt tgcggcagtt actatgtgtt ggcgccggtg 2400
   ccgcatttag gcaccgcgaa cctcgactgg acggttgagg ggccaaaact acgcgaccgt 2460
   atttttgcgt accttgagca gcattacatg cctggcttac ggagtcagct ggtcacgcac 2520
   cggatgttta cgccgtttga ttttcgcgac cagcttaatg cctatcatgg ctcagccttt 2580
   tctgtggagc ccgttcttac ccagagcgcc tggtttcggc cgcataaccg cgataaaacc 2640
   attactaatc tctacctggt cggcgcaggc acgcatcccg gcgcaggcat tcctggcgtc 2700
   atcggctcgg caaaagcgac agcaggtttg atgctggagg atctgatttg aggtacctcg 2760
   acggccatgc aggccgatcc ccgatcgttc aaacatttgg caataaagtt tcttaagatt 2820
   gaatcctgtt gccggtcttg cgatgattat catataattt ctgttgaatt acgttaagca 2880
   tgtaataatt aacatgtaat gcatgacgtt atttatgaga tgggttttta tgattagagt 2940
   cccgcaatta tacatttaat acgcgataga aaacaaaata tagcgcgcaa actaggataa 3000
   attatcgcgc gcggtgtcat ctatgttact agatcgggcc ttaaaactga aggcgggaaa 3060
   cgacaatctg atctctagga agcttgttaa tcatggtgta ggcaacccaa ataaaacacc 3120
   aaaatatgca caaggcagtt tgttgtattc tgtagtacag acaaaactaa aagtaatgaa 3180
   agaagatgtg gtgttagaaa aggaaacaat atcatgagta atgtgtgagc attatgggac 3240
   cacgaaataa aaagaacatt ttgatgagtc gtgtatcctc gatgagcctc aaaagttctc 3300
   tcaccccgga taagaaaccc ttaagcaatg tgcaaagttt gcattctcca ctgacataat 3360
   gcaaaataag atatcatcga tgacatagca actcatgcat catatcatgc ctctctcaac 3420
   ctattcattc ctactcatct acataagtat cttcagctaa atgttagaac ataaacccat 3480
   aagtcacgtt tgatgagtat taggcgtgac acatgacaaa tcacagactc aagcaagata 3540
   aagcaaaatg atgtgtacat aaaactccag agctatatgt catattgcaa aaagaggaga 3600
   gcttataaga caaggcatga ctcacaaaaa ttcatttgcc tttcgtgtca aaaagaggag 3660
   ggctttacat tatccatgtc atattgcaaa agaaagagag aaagaacaac acaatgctgc 3720
   gtcaattata catatctgta tgtccatcat tattcatcca cctttcgtgt accacacttc 3780
   atatatcatg agtcacttca tgtctggaca ttaacaaact ctatcttaac atttagatgc 3840
   aagagccttt atctcactat aaatgcacga tgatttctca ttgtttctca caaaaagcat 3900
   tcagttcatt agtcctacaa caacgaattc ggcttcccgg gtacagggta aatttctagt 3960
   ttttctcctt cattttcttg gttaggaccc ttttctcttt ttattttttt gagctttgat 4020
   ctttctttaa actgatctat tttttaattg attggttatc gtgtaaatat tacatagctt 4080
   taactgataa tctgattact ttatttcgtg tgtctttgat catcttgata gttacagaac 4140
   cgtcgactct agagaagcca tttaaatcgc cgccaccatg gcggccatca cgctcctacg 4200
   ttcagcgtct cttccgggcc tctccgacgc cctcgcccgg gacgctgctg ccgtccaaca 4260
   tgtctgctcc tcctacctgc ccaacaacaa ggagaagaag aggaggtgga tcctctgctc 4320
   gctcaagtac gcctgccttg gcgtcgaccc tgccccgggc gagattgccc ggacctcgcc 4380
   ggtgtactcc agcctcaccg tcacccctgc tggagaggcc gtcatctcct cggagcagaa 4440
   ggtgtacgac gtcgtcctca agcaggcagc attgctcaaa cgccacctgc gcccacaacc 4500
   acacaccatt cccatcgttc ccaaggacct ggacctgcca agaaacggcc tcaagcaggc 4560
   ctatcatcgc tgcggagaga tctgcgagga gtatgccaag accttttacc ttggaactat 4620
   gctcatgacg gaggaccgac ggcgcgccat atgggccatc tatgtgtggt gtaggaggac 4680
   agatgagctt gtagatggac caaatgcctc gcacatcaca ccgtcagccc tggaccggtg 4740
   ggagaagagg cttgatgatc tcttcaccgg acgcccctac gacatgcttg atgctgcact 4800
   ttctgatacc atctccaagt ttcctataga tattcagcct ttcagggaca tgatagaagg 4860
   gatgcggtca gacctcagaa agactagata caagaacttc gacgagctct acatgtactg 4920
   ctactatgtt gctggaactg tggggctaat gagtgttcct gtgatgggta ttgcacccga 4980
   gtcgaaggca acaactgaaa gtgtgtacag tgctgctttg gctctcggca ttgcaaacca 5040
   gctcacaaat atactccgtg acgttggaga ggacgcgaga agagggagga tatatttacc 5100
   acaagatgaa cttgcagagg cagggctctc tgatgaggac atcttcaatg gcgttgtgac 5160
   taacaaatgg agaagcttca tgaagagaca gatcaagaga gctaggatgt tttttgagga 5220
   ggcagagaga ggggtgaccg agctcagcca ggcaagccgg tggccggtct gggcgtctct 5280
   gttgttatac cggcaaatcc ttgacgagat agaagcaaac gattacaaca acttcacaaa 5340
   gagggcgtac gttgggaagg cgaagaaatt gctagcgctt ccagttgcat atggtagatc 5400
   attgctgatg ccctactcac tgagaaatag ccagaagtag ggccatgcag gccgatcccc 5460
   gatcgttcaa acatttggca ataaagtttc ttaagattga atcctgttgc cggtcttgcg 5520
   atgattatca tataatttct gttgaattac gttaagcatg taataattaa catgtaatgc 5580
   atgacgttat ttatgagatg ggtttttatg attagagtcc cgcaattata catttaatac 5640
   gcgatagaaa acaaaatata gcgcgcaaac taggataaat tatcgcgcgc ggtgtcatct 5700
   atgttactag atcg 5714
<210> 7
   <211> 5974
   <212> DNA
   <213> artificial sequence
<220>
   <223> SYNTHETIC - 7651
<400> 7
   gttaatcatg gtgtaggcaa cccaaataaa acaccaaaat atgcacaagg cagtttgttg 60
   tattctgtag tacagacaaa actaaaagta atgaaagaag atgtggtgtt agaaaaggaa 120
   acaatatcat gagtaatgtg tgagcattat gggaccacga aataaaaaga acattttgat 180
   gagtcgtgta tcctcgatga gcctcaaaag ttctctcacc ccggataaga aacccttaag 240
   caatgtgcaa agtttgcatt ctccactgac ataatgcaaa ataagatatc atcgatgaca 300
   tagcaactca tgcatcatat catgcctctc tcaacctatt cattcctact catctacata 360
   agtatcttca gctaaatgtt agaacataaa cccataagtc acgtttgatg agtattaggc 420
   gtgacacatg acaaatcaca gactcaagca agataaagca aaatgatgtg tacataaaac 480
   tccagagcta tatgtcatat tgcaaaaaga ggagagctta taagacaagg catgactcac 540
   aaaaattcat ttgcctttcg tgtcaaaaag aggagggctt tacattatcc atgtcatatt 600
   gcaaaagaaa gagagaaaga acaacacaat gctgcgtcaa ttatacatat ctgtatgtcc 660
   atcattattc atccaccttt cgtgtaccac acttcatata tcatgagtca cttcatgtct 720
   ggacattaac aaactctatc ttaacattta gatgcaagag cctttatctc actataaatg 780
   cacgatgatt tctcattgtt tctcacaaaa agcattcagt tcattagtcc tacaacaacg 840
   aattcggctt cccgggtaca gggtaaattt ctagtttttc tccttcattt tcttggttag 900
   gacccttttc tctttttatt tttttgagct ttgatctttc tttaaactga tctatttttt 960
   aattgattgg ttatcgtgta aatattacat agctttaact gataatctga ttactttatt 1020
   tcgtgtgtct ttgatcatct tgatagttac agaaccgtcg actctagaga agccatttaa 1080
   atcgccgcca ccatggcttc tatgatatcc tcttccgctg tgacaacagt cagccgtgcc 1140
   tctagggggc aatccgccgc agtggctcca ttcggcggcc tcaaatccat gactggattc 1200
   ccagtgaaga aggtcaacac tgacattact tccattacaa gcaatggtgg aagagtaaag 1260
   tgcatggcgg ccgccaaacc aactacggta attggtgcag gcttcggtgg cctggcactg 1320
   gcaattcgtc tacaagctgc ggggatcccc gtcttactgc ttgaacaacg tgataaaccc 1380
   ggcggtcggg cttatgtcta cgaggatcag gggtttacct ttgatgcagg cccgacggtt 1440
   atcaccgatc ccagtgccat tgaagaactg tttgcactgg caggaaaaca gttaaaagag 1500
   tatgtcgaac tgctgccggt tacgccgttt taccgcctgt gttgggagtc agggaaggtc 1560
   tttaattacg ataacgatca aacccggctc gaagcgcaga ttcagcagtt taatccccgc 1620
   gatgtcgaag gttatcgtca gtttctggac tattcacgcg cggtgtttaa agaaggctat 1680
   ctgaagctcg gtactgtccc ttttttatcg ttcagagaca tgcttcgcgc cgcacctcaa 1740
   ctggcgaaac tgcaggcatg gagaagcgtt tacagtaagg ttgccagtta catcgaagat 1800
   gaacatctgc gccaggcgtt ttctttccac tcgctgttgg tgggcggcaa tcccttcgcc 1860
   acctcatcca tttatacgtt gatacacgcg ctggagcgtg agtggggcgt ctggtttccg 1920
   cgtggcggca ccggcgcatt agttcagggg atgataaagc tgtttcagga tctgggtggc 1980
   gaagtcgtgt taaacgccag agtcagccat atggaaacga caggaaacaa gattgaagcc 2040
   gtgcatttag aggacggtcg caggttcctg acgcaagccg tcgcgtcaaa tgcagatgtg 2100
   gttcatacct atcgcgacct gttaagccag caccctgccg cggttaagca gtccaacaaa 2160
   ctgcagacta agcgcatgag taactctctg tttgtgctct attttggttt gaatcaccat 2220
   catgatcagc tcgcgcatca cacggtttgt ttcggcccgc gttaccgcga gctgattgac 2280
   gaaattttta atcatgatgg cctcgcagag gacttctcac tttatctgca cgcgccctgt 2340
   gtcacggatt cgtcactggc gcctgaaggt tgcggcagtt actatgtgtt ggcgccggtg 2400
   ccgcatttag gcaccgcgaa cctcgactgg acggttgagg ggccaaaact acgcgaccgt 2460
   atttttgcgt accttgagca gcattacatg cctggcttac ggagtcagct ggtcacgcac 2520
   cggatgttta cgccgtttga ttttcgcgac cagcttaatg cctatcatgg ctcagccttt 2580
   tctgtggagc ccgttcttac ccagagcgcc tggtttcggc cgcataaccg cgataaaacc 2640
   attactaatc tctacctggt cggcgcaggc acgcatcccg gcgcaggcat tcctggcgtc 2700
   atcggctcgg caaaagcgac agcaggtttg atgctggagg atctgatttg aggtacctcg 2760
   acggccatgc aggccgatcc ccgatcgttc aaacatttgg caataaagtt tcttaagatt 2820
   gaatcctgtt gccggtcttg cgatgattat catataattt ctgttgaatt acgttaagca 2880
   tgtaataatt aacatgtaat gcatgacgtt atttatgaga tgggttttta tgattagagt 2940
   cccgcaatta tacatttaat acgcgataga aaacaaaata tagcgcgcaa actaggataa 3000
   attatcgcgc gcggtgtcat ctatgttact agatcgggcc ttaatgttcg gggcgaacat 3060
   cgcaagcttg ttaatcatgg tgtaggcaac ccaaataaaa caccaaaata tgcacaaggc 3120
   agtttgttgt attctgtagt acagacaaaa ctaaaagtaa tgaaagaaga tgtggtgtta 3180
   gaaaaggaaa caatatcatg agtaatgtgt gagcattatg ggaccacgaa ataaaaagaa 3240
   cattttgatg agtcgtgtat cctcgatgag cctcaaaagt tctctcaccc cggataagaa 3300
   acccttaagc aatgtgcaaa gtttgcattc tccactgaca taatgcaaaa taagatatca 3360
   tcgatgacat agcaactcat gcatcatatc atgcctctct caacctattc attcctactc 3420
   atctacataa gtatcttcag ctaaatgtta gaacataaac ccataagtca cgtttgatga 3480
   gtattaggcg tgacacatga caaatcacag actcaagcaa gataaagcaa aatgatgtgt 3540
   acataaaact ccagagctat atgtcatatt gcaaaaagag gagagcttat aagacaaggc 3600
   atgactcaca aaaattcatt tgcctttcgt gtcaaaaaga ggagggcttt acattatcca 3660
   tgtcatattg caaaagaaag agagaaagaa caacacaatg ctgcgtcaat tatacatatc 3720
   tgtatgtcca tcattattca tccacctttc gtgtaccaca cttcatatat catgagtcac 3780
   ttcatgtctg gacattaaca aactctatct taacatttag atgcaagagc ctttatctca 3840
   ctataaatgc acgatgattt ctcattgttt ctcacaaaaa gcattcagtt cattagtcct 3900
   acaacaacga attcggcttc ccgggtacag ggtaaatttc tagtttttct ccttcatttt 3960
   cttggttagg acccttttct ctttttattt ttttgagctt tgatctttct ttaaactgat 4020
   ctatttttta attgattggt tatcgtgtaa atattacata gctttaactg ataatctgat 4080
   tactttattt cgtgtgtctt tgatcatctt gatagttaca gaaccgtcga ctctagagaa 4140
   gccatttaaa tcgccgccac catgtctgtt gccttgttat gggttgtttc tccttgtgac 4200
   gtctcaaacg ggacaggatt cttggtatcc gttcgtgagg gaaaccggat ttttgattcg 4260
   tcggggcgta ggaatttggc gtgcaatgag agaatcaaga gaggaggtgg aaaacaaagg 4320
   tggagttttg gttcttactt gggaggagca caaactggaa gtggacggaa attttctgta 4380
   cgttctgcta tcgtggctac tccggctgga gaaatgacga tgtcatcaga acggatggta 4440
   tatgatgtgg ttttgaggca ggcagccttg gtgaagagac agctgagatc gaccgatgag 4500
   ttagatgtga agaaggatat acctattccg gggactttgg gcttgttgag tgaagcatat 4560
   gataggtgta gtgaagtatg tgcagagtac gcaaagacgt tttacttagg aacgatgcta 4620
   atgactccgg agagaagaaa ggctatctgg gcaatatacg tatggtgcag gagaacagac 4680
   gaacttgttg atggtccgaa tgcatcacac attactccgg cggccttaga taggtgggaa 4740
   gacaggctag aagatgtttt cagtggacgg ccatttgaca tgctcgatgc tgctttgtcc 4800
   gacacagttt ccaaatttcc agttgatatt cagccattca gagatatgat tgaaggaatg 4860
   cgtatggact tgaggaagtc aagatacaga aactttgacg aactatacct atattgttat 4920
   tacgttgctg gtacggttgg gttgatgagt gttccaatta tgggcatcgc acctgaatca 4980
   aaggcaacaa cggagagcgt atataatgct gctttggctt tggggatcgc aaatcagctg 5040
   accaacatac ttagagatgt tggagaagat gccagaagag gaagagtcta tttgcctcaa 5100
   gatgaattag cacaggcagg tctatccgac gaagacatat ttgctggaag agtgaccgat 5160
   aaatggagaa tcttcatgaa gaaacaaatt cagagggcaa gaaagttctt tgacgaggca 5220
   gagaaaggag tgaccgaatt gagcgcagct agtagatggc ctgtgttggc atctctgctg 5280
   ttgtaccgca ggatactgga cgagatcgaa gccaatgact acaacaactt cacaaagaga 5340
   gcttatgtga gcaaaccaaa gaagttgatt gcattaccta ttgcatatgc aaaatctctt 5400
   gtgccttcta caagaacatg aaatcaggat tttatataaa tcaaggccaa tgaagccaat 5460
   atacatttag aagaaaaaaa acaagtgttt ataaagtaga attattgaag gggaggcttg 5520
   gagtaactgg taaagttgtt gtcatgtgac tgggaagtca cgggttcaag ccttggaaac 5580
   agcctctggc agaaatgcaa ggtaaggttg cgtacaatat accgttaagg tggggtcctt 5640
   cccagtacac cgcgcatagc gatagattta gtgcaccggg tcgccttttt tctaaagtag 5700
   ggccatgcag gccgatcccc gatcgttcaa acatttggca ataaagtttc ttaagattga 5760
   atcctgttgc cggtcttgcg atgattatca tataatttct gttgaattac gttaagcatg 5820
   taataattaa catgtaatgc atgacgttat ttatgagatg ggtttttatg attagagtcc 5880
   cgcaattata catttaatac gcgatagaaa acaaaatata gcgcgcaaac taggataaat 5940
   tatcgcgcgc ggtgtcatct atgttactag atcg 5974
<210> 8
   <211> 5782
   <212> DNA
   <213> artificial sequence
<220>
   <223> SYNTHETIC - 7650
<400> 8
   gttaatcatg gtgtaggcaa cccaaataaa acaccaaaat atgcacaagg cagtttgttg 60
   tattctgtag tacagacaaa actaaaagta atgaaagaag atgtggtgtt agaaaaggaa 120
   acaatatcat gagtaatgtg tgagcattat gggaccacga aataaaaaga acattttgat 180
   gagtcgtgta tcctcgatga gcctcaaaag ttctctcacc ccggataaga aacccttaag 240
   caatgtgcaa agtttgcatt ctccactgac ataatgcaaa ataagatatc atcgatgaca 300
   tagcaactca tgcatcatat catgcctctc tcaacctatt cattcctact catctacata 360
   agtatcttca gctaaatgtt agaacataaa cccataagtc acgtttgatg agtattaggc 420
   gtgacacatg acaaatcaca gactcaagca agataaagca aaatgatgtg tacataaaac 480
   tccagagcta tatgtcatat tgcaaaaaga ggagagctta taagacaagg catgactcac 540
   aaaaattcat ttgcctttcg tgtcaaaaag aggagggctt tacattatcc atgtcatatt 600
   gcaaaagaaa gagagaaaga acaacacaat gctgcgtcaa ttatacatat ctgtatgtcc 660
   atcattattc atccaccttt cgtgtaccac acttcatata tcatgagtca cttcatgtct 720
   ggacattaac aaactctatc ttaacattta gatgcaagag cctttatctc actataaatg 780
   cacgatgatt tctcattgtt tctcacaaaa agcattcagt tcattagtcc tacaacaacg 840
   aattcggctt cccgggtaca gggtaaattt ctagtttttc tccttcattt tcttggttag 900
   gacccttttc tctttttatt tttttgagct ttgatctttc tttaaactga tctatttttt 960
   aattgattgg ttatcgtgta aatattacat agctttaact gataatctga ttactttatt 1020
   tcgtgtgtct ttgatcatct tgatagttac agaaccgtcg actctagaga agccatttaa 1080
   atcgccgcca ccatggcttc tatgatatcc tcttccgctg tgacaacagt cagccgtgcc 1140
   tctagggggc aatccgccgc agtggctcca ttcggcggcc tcaaatccat gactggattc 1200
   ccagtgaaga aggtcaacac tgacattact tccattacaa gcaatggtgg aagagtaaag 1260
   tgcatggcgg ccgccaaacc aactacggta attggtgcag gcttcggtgg cctggcactg 1320
   gcaattcgtc tacaagctgc ggggatcccc gtcttactgc ttgaacaacg tgataaaccc 1380
   ggcggtcggg cttatgtcta cgaggatcag gggtttacct ttgatgcagg cccgacggtt 1440
   atcaccgatc ccagtgccat tgaagaactg tttgcactgg caggaaaaca gttaaaagag 1500
   tatgtcgaac tgctgccggt tacgccgttt taccgcctgt gttgggagtc agggaaggtc 1560
   tttaattacg ataacgatca aacccggctc gaagcgcaga ttcagcagtt taatccccgc 1620
   gatgtcgaag gttatcgtca gtttctggac tattcacgcg cggtgtttaa agaaggctat 1680
   ctgaagctcg gtactgtccc ttttttatcg ttcagagaca tgcttcgcgc cgcacctcaa 1740
   ctggcgaaac tgcaggcatg gagaagcgtt tacagtaagg ttgccagtta catcgaagat 1800
   gaacatctgc gccaggcgtt ttctttccac tcgctgttgg tgggcggcaa tcccttcgcc 1860
   acctcatcca tttatacgtt gatacacgcg ctggagcgtg agtggggcgt ctggtttccg 1920
   cgtggcggca ccggcgcatt agttcagggg atgataaagc tgtttcagga tctgggtggc 1980
   gaagtcgtgt taaacgccag agtcagccat atggaaacga caggaaacaa gattgaagcc 2040
   gtgcatttag aggacggtcg caggttcctg acgcaagccg tcgcgtcaaa tgcagatgtg 2100
   gttcatacct atcgcgacct gttaagccag caccctgccg cggttaagca gtccaacaaa 2160
   ctgcagacta agcgcatgag taactctctg tttgtgctct attttggttt gaatcaccat 2220
   catgatcagc tcgcgcatca cacggtttgt ttcggcccgc gttaccgcga gctgattgac 2280
   gaaattttta atcatgatgg cctcgcagag gacttctcac tttatctgca cgcgccctgt 2340
   gtcacggatt cgtcactggc gcctgaaggt tgcggcagtt actatgtgtt ggcgccggtg 2400
   ccgcatttag gcaccgcgaa cctcgactgg acggttgagg ggccaaaact acgcgaccgt 2460
   atttttgcgt accttgagca gcattacatg cctggcttac ggagtcagct ggtcacgcac 2520
   cggatgttta cgccgtttga ttttcgcgac cagcttaatg cctatcatgg ctcagccttt 2580
   tctgtggagc ccgttcttac ccagagcgcc tggtttcggc cgcataaccg cgataaaacc 2640
   attactaatc tctacctggt cggcgcaggc acgcatcccg gcgcaggcat tcctggcgtc 2700
   atcggctcgg caaaagcgac agcaggtttg atgctggagg atctgatttg aggtacctcg 2760
   acggccatgc aggccgatcc ccgatcgttc aaacatttgg caataaagtt tcttaagatt 2820
   gaatcctgtt gccggtcttg cgatgattat catataattt ctgttgaatt acgttaagca 2880
   tgtaataatt aacatgtaat gcatgacgtt atttatgaga tgggttttta tgattagagt 2940
   cccgcaatta tacatttaat acgcgataga aaacaaaata tagcgcgcaa actaggataa 3000
   attatcgcgc gcggtgtcat ctatgttact agatcgggcc ttaatcgcaa gcttgttaat 3060
   catggtgtag gcaacccaaa taaaacacca aaatatgcac aaggcagttt gttgtattct 3120
   gtagtacaga caaaactaaa agtaatgaaa gaagatgtgg tgttagaaaa ggaaacaata 3180
   tcatgagtaa tgtgtgagca ttatgggacc acgaaataaa aagaacattt tgatgagtcg 3240
   tgtatcctcg atgagcctca aaagttctct caccccggat aagaaaccct taagcaatgt 3300
   gcaaagtttg cattctccac tgacataatg caaaataaga tatcatcgat gacatagcaa 3360
   ctcatgcatc atatcatgcc tctctcaacc tattcattcc tactcatcta cataagtatc 3420
   ttcagctaaa tgttagaaca taaacccata agtcacgttt gatgagtatt aggcgtgaca 3480
   catgacaaat cacagactca agcaagataa agcaaaatga tgtgtacata aaactccaga 3540
   gctatatgtc atattgcaaa aagaggagag cttataagac aaggcatgac tcacaaaaat 3600
   tcatttgcct ttcgtgtcaa aaagaggagg gctttacatt atccatgtca tattgcaaaa 3660
   gaaagagaga aagaacaaca caatgctgcg tcaattatac atatctgtat gtccatcatt 3720
   attcatccac ctttcgtgta ccacacttca tatatcatga gtcacttcat gtctggacat 3780
   taacaaactc tatcttaaca tttagatgca agagccttta tctcactata aatgcacgat 3840
   gatttctcat tgtttctcac aaaaagcatt cagttcatta gtcctacaac aacgaattcg 3900
   gcttcccggg tacagggtaa atttctagtt tttctccttc attttcttgg ttaggaccct 3960
   tttctctttt tatttttttg agctttgatc tttctttaaa ctgatctatt ttttaattga 4020
   ttggttatcg tgtaaatatt acatagcttt aactgataat ctgattactt tatttcgtgt 4080
   gtctttgatc atcttgatag ttacagaacc gtcgactcta gagaagccat ttaaatcgcc 4140
   gccaccatgt ctgttgcctt gttatgggtt gtttctcctt gtgacgtctc aaatgggaca 4200
   agtttcatgg aatcagtccg ggagggaaac cgtttttttg attcatcgag gcataggaat 4260
   ttggtgtcca atgagagaat caatagaggt ggtggaaagc aaactaataa tggacggaaa 4320
   ttttctgtac ggtctgctat tttggctact ccatctggag aacggacgat gacatcggaa 4380
   cagatggtct atgatgtggt tttgaggcag gcagccttgg tgaagaggca actgagatct 4440
   accaatgagt tagaagtgaa gccggatata cctattccgg ggaatttggg cttgttgagt 4500
   gaagcatatg ataggtgtgg tgaagtatgt gcagagtatg caaagacgtt taacttagga 4560
   actatgctaa tgactcccga gagaagaagg gctatctggg caatatatgt atggtgcaga 4620
   agaacagatg aacttgttga tggcccaaac gcatcatata ttaccccggc agccttagat 4680
   aggtgggaaa ataggctaga agatgttttc aatgggcggc catttgacat gctcgatggt 4740
   gctttgtccg atacagtttc taactttcca gttgatattc agccattcag agatatgatt 4800
   gaaggaatgc gtatggactt gagaaaatcg agatacaaaa acttcgacga actatacctt 4860
   tattgttatt atgttgctgg tacggttggg ttgatgagtg ttccaattat gggtatcgcc 4920
   cctgaatcaa aggcaacaac agagagcgta tataatgctg ctttggctct ggggatcgca 4980
   aatcaattaa ctaacatact cagagatgtt ggagaagatg ccagaagagg aagagtctac 5040
   ttgcctcaag atgaattagc acaggcaggt ctatccgatg aagatatatt tgctggaagg 5100
   gtgaccgata aatggagaat ctttatgaag aaacaaatac atagggcaag aaagttcttt 5160
   gatgaggcag agaaaggcgt gacagaattg agctcagcta gtagattccc tgtatgggca 5220
   tctttggtct tgtaccgcaa aatactagat gagattgaag ccaatgacta caacaacttc 5280
   acaaagagag catatgtgag caaatcaaag aagttgattg cattacctat tgcatatgca 5340
   aaatctcttg tgcctcctac aaaaactgcc tctcttcaaa gataaagcat gaaatgaaga 5400
   tatatatata tatatatata gcaatataca ttagaagaaa aaaaggaaga agaaatgttg 5460
   ttgtattgat ataaatgtat atcataaata ttaggttgta gtaacattgg ccatgcaggc 5520
   cgatccccga tcgttcaaac atttggcaat aaagtttctt aagattgaat cctgttgccg 5580
   gtcttgcgat gattatcata taatttctgt tgaattacgt taagcatgta ataattaaca 5640
   tgtaatgcat gacgttattt atgagatggg tttttatgat tagagtcccg caattataca 5700
   tttaatacgc gatagaaaac aaaatatagc gcgcaaacta ggataaatta tcgcgcgcgg 5760
   tgtcatctat gttactagat cg 5782
<210> 9
   <211> 5551
   <212> DNA
   <213> artificial sequence
<220>
   <223> SYNTHETIC
<400> 9
   gttaatcatg gtgtaggcaa cccaaataaa acaccaaaat atgcacaagg cagtttgttg 60
   tattctgtag tacagacaaa actaaaagta atgaaagaag atgtggtgtt agaaaaggaa 120
   acaatatcat gagtaatgtg tgagcattat gggaccacga aataaaaaga acattttgat 180
   gagtcgtgta tcctcgatga gcctcaaaag ttctctcacc ccggataaga aacccttaag 240
   caatgtgcaa agtttgcatt ctccactgac ataatgcaaa ataagatatc atcgatgaca 300
   tagcaactca tgcatcatat catgcctctc tcaacctatt cattcctact catctacata 360
   agtatcttca gctaaatgtt agaacataaa cccataagtc acgtttgatg agtattaggc 420
   gtgacacatg acaaatcaca gactcaagca agataaagca aaatgatgtg tacataaaac 480
   tccagagcta tatgtcatat tgcaaaaaga ggagagctta taagacaagg catgactcac 540
   aaaaattcat ttgcctttcg tgtcaaaaag aggagggctt tacattatcc atgtcatatt 600
   gcaaaagaaa gagagaaaga acaacacaat gctgcgtcaa ttatacatat ctgtatgtcc 660
   atcattattc atccaccttt cgtgtaccac acttcatata tcatgagtca cttcatgtct 720
   ggacattaac aaactctatc ttaacattta gatgcaagag cctttatctc actataaatg 780
   cacgatgatt tctcattgtt tctcacaaaa agcattcagt tcattagtcc tacaacaacg 840
   aattcggctt cccgggtaca gggtaaattt ctagtttttc tccttcattt tcttggttag 900
   gacccttttc tctttttatt tttttgagct ttgatctttc tttaaactga tctatttttt 960
   aattgattgg ttatcgtgta aatattacat agctttaact gataatctga ttactttatt 1020
   tcgtgtgtct ttgatcatct tgatagttac agaaccgtcg actctagaga agccatttaa 1080
   atcgccgcca ccatggcttc tatgatatcc tcttccgctg tgacaacagt cagccgtgcc 1140
   tctagggggc aatccgccgc agtggctcca ttcggcggcc tcaaatccat gactggattc 1200
   ccagtgaaga aggtcaacac tgacattact tccattacaa gcaatggtgg aagagtaaag 1260
   tgcatggcgg ccgccaaacc aactacggta attggtgcag gcttcggtgg cctggcactg 1320
   gcaattcgtc tacaagctgc ggggatcccc gtcttactgc ttgaacaacg tgataaaccc 1380
   ggcggtcggg cttatgtcta cgaggatcag gggtttacct ttgatgcagg cccgacggtt 1440
   atcaccgatc ccagtgccat tgaagaactg tttgcactgg caggaaaaca gttaaaagag 1500
   tatgtcgaac tgctgccggt tacgccgttt taccgcctgt gttgggagtc agggaaggtc 1560
   tttaattacg ataacgatca aacccggctc gaagcgcaga ttcagcagtt taatccccgc 1620
   gatgtcgaag gttatcgtca gtttctggac tattcacgcg cggtgtttaa agaaggctat 1680
   ctgaagctcg gtactgtccc ttttttatcg ttcagagaca tgcttcgcgc cgcacctcaa 1740
   ctggcgaaac tgcaggcatg gagaagcgtt tacagtaagg ttgccagtta catcgaagat 1800
   gaacatctgc gccaggcgtt ttctttccac tcgctgttgg tgggcggcaa tcccttcgcc 1860
   acctcatcca tttatacgtt gatacacgcg ctggagcgtg agtggggcgt ctggtttccg 1920
   cgtggcggca ccggcgcatt agttcagggg atgataaagc tgtttcagga tctgggtggc 1980
   gaagtcgtgt taaacgccag agtcagccat atggaaacga caggaaacaa gattgaagcc 2040
   gtgcatttag aggacggtcg caggttcctg acgcaagccg tcgcgtcaaa tgcagatgtg 2100
   gttcatacct atcgcgacct gttaagccag caccctgccg cggttaagca gtccaacaaa 2160
   ctgcagacta agcgcatgag taactctctg tttgtgctct attttggttt gaatcaccat 2220
   catgatcagc tcgcgcatca cacggtttgt ttcggcccgc gttaccgcga gctgattgac 2280
   gaaattttta atcatgatgg cctcgcagag gacttctcac tttatctgca cgcgccctgt 2340
   gtcacggatt cgtcactggc gcctgaaggt tgcggcagtt actatgtgtt ggcgccggtg 2400
   ccgcatttag gcaccgcgaa cctcgactgg acggttgagg ggccaaaact acgcgaccgt 2460
   atttttgcgt accttgagca gcattacatg cctggcttac ggagtcagct ggtcacgcac 2520
   cggatgttta cgccgtttga ttttcgcgac cagcttaatg cctatcatgg ctcagccttt 2580
   tctgtggagc ccgttcttac ccagagcgcc tggtttcggc cgcataaccg cgataaaacc 2640
   attactaatc tctacctggt cggcgcaggc acgcatcccg gcgcaggcat tcctggcgtc 2700
   atcggctcgg caaaagcgac agcaggtttg atgctggagg atctgatttg aggtacctcg 2760
   acggccatgc aggccgatcc ccgatcgttc aaacatttgg caataaagtt tcttaagatt 2820
   gaatcctgtt gccggtcttg cgatgattat catataattt ctgttgaatt acgttaagca 2880
   tgtaataatt aacatgtaat gcatgacgtt atttatgaga tgggttttta tgattagagt 2940
   cccgcaatta tacatttaat acgcgataga aaacaaaata tagcgcgcaa actaggataa 3000
   attatcgcgc gcggtgtcat ctatgttact agatcgggcc ttaatcgcaa gcttgttaat 3060
   catggtgtag gcaacccaaa taaaacacca aaatatgcac aaggcagttt gttgtattct 3120
   gtagtacaga caaaactaaa agtaatgaaa gaagatgtgg tgttagaaaa ggaaacaata 3180
   tcatgagtaa tgtgtgagca ttatgggacc acgaaataaa aagaacattt tgatgagtcg 3240
   tgtatcctcg atgagcctca aaagttctct caccccggat aagaaaccct taagcaatgt 3300
   gcaaagtttg cattctccac tgacataatg caaaataaga tatcatcgat gacatagcaa 3360
   ctcatgcatc atatcatgcc tctctcaacc tattcattcc tactcatcta cataagtatc 3420
   ttcagctaaa tgttagaaca taaacccata agtcacgttt gatgagtatt aggcgtgaca 3480
   catgacaaat cacagactca agcaagataa agcaaaatga tgtgtacata aaactccaga 3540
   gctatatgtc atattgcaaa aagaggagag cttataagac aaggcatgac tcacaaaaat 3600
   tcatttgcct ttcgtgtcaa aaagaggagg gctttacatt atccatgtca tattgcaaaa 3660
   gaaagagaga aagaacaaca caatgctgcg tcaattatac atatctgtat gtccatcatt 3720
   attcatccac ctttcgtgta ccacacttca tatatcatga gtcacttcat gtctggacat 3780
   taacaaactc tatcttaaca tttagatgca agagccttta tctcactata aatgcacgat 3840
   gatttctcat tgtttctcac aaaaagcatt cagttcatta gtcctacaac aacgaattcg 3900
   gcttcccggg tacagggtaa atttctagtt tttctccttc attttcttgg ttaggaccct 3960
   tttctctttt tatttttttg agctttgatc tttctttaaa ctgatctatt ttttaattga 4020
   ttggttatcg tgtaaatatt acatagcttt aactgataat ctgattactt tatttcgtgt 4080
   gtctttgatc atcttgatag ttacagaacc gtcgactcta gagaagccat ttaaatcgcc 4140
   gccaccatgg cttctatgat atcctcttcc gctgtgacaa cagtcagccg tgcctctagg 4200
   gggcaatccg ccgcagtggc tccattcggc ggcctcaaat ccatgactgg attcccagtg 4260
   aagaaggtca acactgacat tacttccatt acaagcaatg gtggaagagt aaagtgcatg 4320
   gcagttggct cgaaaagttt tgcgacagcc tcaaagttat ttgatgcaaa aacccggcgc 4380
   agcgtactga tgctctacgc ctggtgccgc cattgtgacg atgttattga cgatcagacg 4440
   ctgggctttc aggcccggca gcctgcctta caaacgcccg aacaacgtct gatgcaactt 4500
   gagatgaaaa cgcgccaggc ctatgcagga tcgcagatgc acgaaccggc gtttgcggct 4560
   tttcaggaag tggctatggc tcatgatatc gccccggctt acgcgtttga tcatctggaa 4620
   ggcttcgcga tggatgtacg cgaagcgcaa tacagccaac tggatgatac gctgcgctat 4680
   tgctatcacg ttgcaggcgt tgtcggcttg atgatggcgc aaatcatggg cgtgcgggat 4740
   aacgccacgc tggaccgcgc ctgtgacctt gggctggcat ttcagttgac caatattgct 4800
   cgcgatattg tggacgatgc gcatgcgggc cgctgttatc tgccggcaag ctggctggag 4860
   catgaaggtc tgaacaaaga gaattatgcg gcacctgaaa accgtcaggc gctgagccgt 4920
   atcgcccgac gtttggtgca ggaagcagaa ccttactatt tgtctgccac agccggcctg 4980
   gcagggttgc ccctgcgttc cgcctgggca atcgctacgg cgaagcaggt ttaccggaaa 5040
   ataggtgtca aagttgaaca ggccggtcag caagcctggg atcagcggca gtcaacgacc 5100
   acgcccgaaa aattaacgct gctgctggcc gcctctggtc aggcccttac ttcccggatg 5160
   cgggctcatc ctccccgccc tgcgcatctc tggcagcgcc cgctctaggg atccgttaag 5220
   ggcgaattcc agcacactgg cggccgttac tagtggatcc gagctcggta cctcgacggc 5280
   catgcaggcc gatccccgat cgttcaaaca tttggcaata aagtttctta agattgaatc 5340
   ctgttgccgg tcttgcgatg attatcatat aatttctgtt gaattacgtt aagcatgtaa 5400
   taattaacat gtaatgcatg acgttattta tgagatgggt ttttatgatt agagtcccgc 5460
   aattatacat ttaatacgcg atagaaaaca aaatatagcg cgcaaactag gataaattat 5520
   cgcgcgcggt gtcatctatg ttactagatc g 5551
<210> 10
   <211> 1233
   <212> DNA
   <213> Zea mays
<400> 10
   atggccatca tactcgtacg agcagcgtcg ccggggctct ccgccgccga cagcatcagc 60
   caccagggga ctctccagtg ctccaccctg ctcaagacga agaggccggc ggcgcggcgg 120
   tggatgccct gctcgctcct tggcctccac ccgtgggagg ctggccgtcc ctcccccgcc 180
   gtctactcca gcctgcccgt caacccggcg ggagaggccg tcgtctcgtc cgagcagaag 240
   gtctacgacg tcgtgctcaa gcaggccgca ttgctcaaac gccagctgcg cacgccggtc 300
   ctcgacgcca ggccccagga catggacatg ccacgcaacg ggctcaagga agcctacgac 360
   cgctgcggcg agatctgtga ggagtatgcc aagacgtttt acctcggaac tatgttgatg 420
   acagaggagc ggcgccgcgc catatgggcc atctatgtgt ggtgtaggag gacagatgag 480
   cttgtagatg ggccaaacgc caactacatt acaccaacag ctttggaccg gtgggagaag 540
   agacttgagg atctgttcac gggacgtcct tacgacatgc ttgatgccgc tctctctgat 600
   accatctcaa ggttccccat agacattcag ccattcaggg acatgattga agggatgagg 660
   agtgatctta ggaagacaag gtataacaac ttcgacgagc tctacatgta ctgctactat 720
   gttgctggaa ctgtcgggtt aatgagcgta cctgtgatgg gcatcgcaac cgagtctaaa 780
   gcaacaactg aaagcgtata cagtgctgcc ttggctctgg gaattgcgaa ccaactcacg 840
   aacatactcc gggatgttgg agaggatgct agaagaggaa ggatatattt accacaagat 900
   gagcttgcac aggcagggct ctctgatgag gacatcttca aaggggtcgt cacgaaccgg 960
   tggagaaact tcatgaagag gcagatcaag agggccagga tgtttttgga ggaggcagag 1020
   agaggggtaa ctgagctctc acaggctagc agatggccag tatgggcttc cctgttgttg 1080
   tacaggcaga tcctggatga gatcgaagcc aacgactaca acaacttcac gaagagggcg 1140
   tatgttggta aagggaagaa gttgctagca cttcctgtgg catatggaaa atcgctactg 1200
   ctcccatgtt cattgagaaa tggccagacc tag 1233
<210> 11
   <211> 1233
   <212> DNA
   <213> Zea mays
<400> 11
   atggccatca tactcgtacg agcagcgtcg ccggggctct ccgccgccga cagcatcagc 60
   caccagggga ctctccagtg ctccaccctg ctcaagacga agaggccggc ggcgcggcgg     120
   tggatgccct gctcgctcct tggcctccac ccgtgggagg ctggccgtcc ctcccccgcc     180
   gtctactcca gcctgcccgt caacccggcg ggagaggccg tcgtctcgtc cgagcagaag 240
   gtctacgacg tcgtgctcaa gcaggccgca ttgctcaaac gccagctgcg cacgccggtc 300
   ctcgacgcca ggccccagga catggacatg ccacgcaacg ggctcaagga agcctacgac 360
   cgctgcggcg agatctgtga ggagtatgcc aagacgtttt acctcggaac tatgttgatg 420
   acagaggagc ggcgccgcgc catatgggcc atctatgtgt ggtgtaggag gacagatgag 480
   cttgtagatg ggccaaacgc caactacatt acaccaacag ctttggaccg gtgggagaag 540
   agacttgagg atctgttcac gggacgtcct tacgacatgc ttgatgccgc tctctctgat 600
   accatctcaa ggttccccat agacattcag ccattcaggg acatgattga agggatgagg 660
   agtgatctta ggaagacaag gtataacaac ttcgacgagc tctacatgta ctgctactat 720
   gttgctggaa ctgtcgggtt aatgagcgta cctgtgatgg gcatcgcaac cgagtctaaa 780
   gcaacaactg aaagcgtata cagtgctgcc ttggctctgg gaattgcgaa ccaactcacg 840
   aacatactcc gggatgttgg agaggatgct agaagaggaa ggatatattt accacaagat 900
   gagcttgcac aggcagggct ctctgatgag gacatcttca aaggggtcgt cacgaaccgg 960
   tggagaaact tcatgaagag gcagatcaag agggccagga tgtttttgga ggaggcagag 1020
   agaggggtaa atgagctctc acaggctagc agatggccag tatgggcttc cctgttgttg 1080
   tacaggcaga tcctggatga gatcgaagcc aacgactaca acaacttcac gaagagggcg 1140
   tatgttggta aagggaagaa gttgctagca cttcctgtgg catatggaaa atcgctactg 1200
   ctcccatgtt cattgagaaa tggccagacc tag 1233
<210> 12
   <211> 1233
   <212> DNA
   <213> Zea mays
<400> 12
   atggccatca tactcgtacg agcagcgtcg ccggggctct ccgccgccga cagcatcagc 60
   caccagggga ctctccagtg ctccaccctg ctcaagacga agaggccggc ggcgcgccgg 120
   tggatgccct gctcgctcct tggcctccac ccgtgggagg ctggccgtcc ctcccccgcc 180
   gtctactcca gcctcgccgt caacccggcg ggagaggccg tcgtctcgtc cgagcagaag 240
   gtctacgacg tcgtgctcaa gcaggccgca ttgctcaaac gccagctgcg cacgccggtc 300
   ctcgacgcca ggccccagga catggacatg ccacgcaacg ggctcaagga agcctacgac 360
   cgctgcggcg agatctgtga ggagtatgcc aagacgtttt acctcggaac tatgttgatg 420
   acagaggagc ggcgccgcgc catatgggcc atctatgtgt ggtgtaggag gacagatgag 480
   cttgtagatg ggccaaacgc caactacatt acaccaacag ctttggaccg gtgggagaag 540
   agacttgagg atctgttcac gggacgtcct tacgacatgc ttgatgccgc tctctctgat 600
   accatctcaa ggttccccat agacattcag ccattcaggg acatgattga agggatgagg 660
   agtgatctta ggaagacaag gtataacaac ttcgacgagc tctacatgta ctgctactat 720
   gttgctggaa ctgtcgggtt aatgagcgta ccagtgatgg gcatcgcatc cgagtctaaa 780
   gcaacaactg aaagcgtgta cagtgctgcc ttggctctcg gaattgcgaa ccaactcacg 840
   aacatactcc gggatgttgg agaggatgct agacgaggaa ggatatattt accacaagat 900
   gagcttgcac aggcagggct ctctgatgag gacatcttca aaggggtcgt cacgaaccgg 960
   tggagaaact tcatgaagag gcagatcaag agggccagga tgtttttgga ggaggcagag 1020
   agaggggtaa ctgagctctc acaggctagc agatggccag tatgggcttc cctgttgttg   1080
   tacaggcaga tcctggatga gatcgaagcc aacgactaca acaacttcac gaagagggcg   1140
   tatgttggta aagggaagaa gttgctagca cttcctgtgg catatggaaa atcgctactg   1200
   ctcccatgtt cattgagaaa tggccagacc tag                                1233
<210> 13
   <211> 1263
   <212> DNA
   <213> Oryza sp.
<400> 13
   atggcggcca tcacgctcct acgttcagcg tctcttccgg gcctctccga cgccctcgcc     60
   cgggacgctg ctgccgtcca acatgtctgc tcctcctacc tgcccaacaa caaggagaag    120
   aagaggaggt ggatcctctg ctcgctcaag tacgcctgcc ttggcgtcga ccctgccccg    180
   ggcgagattg cccggacctc gccggtgtac tccagcctca ccgtcacccc tgctggagag    240
   gccgtcatct cctcggagca gaaggtgtac gacgtcgtcc tcaagcaggc agcattgctc    300
   aaacgccacc tgcgcccaca accacacacc attcccatcg ttcccaagga cctggacctg    360
   ccaagaaacg gcctcaagca ggcctatcat cgctgcggag agatctgcga ggagtatgcc    420
   aagacctttt accttggaac tatgctcatg acggaggacc gacggcgcgc catatgggcc    480
   atctatgtgt ggtgtaggag gacagatgag cttgtagatg gaccaaatgc ctcgcacatc    540
   acaccgtcag ccctggaccg gtgggagaag aggcttgatg atctcttcac cggacgcccc    600
   tacgacatgc ttgatgctgc actttctgat accatctcca agtttcctat agatattcag    660
   cctttcaggg acatgataga agggatgcgg tcagacctca gaaagactag atacaagaac    720
   ttcgacgagc tctacatgta ctgctactat gttgctggaa ctgtggggct aatgagtgtt    780
   cctgtgatgg gtattgcacc cgagtcgaag gcaacaactg aaagtgtgta cagtgctgct    840
   ttggctctcg gcattgcaaa ccagctcaca aatatactcc gtgacgttgg agaggacgcg    900
   agaagaggga ggatatattt accacaagat gaacttgcag aggcagggct ctctgatgag    960
   gacatcttca atggcgttgt gactaacaaa tggagaagct tcatgaagag acagatcaag   1020
   agagctagga tgtttttgga ggaggcagag agaggggtga ccgagctcag ccaggcaagc   1080
   cggtggccgg tctgggcgtc tctgttgtta taccggcaaa tccttgacga gatagaagca   1140
   aacgattaca acaacttcac aaagagggcg tacgttggga aggcgaagaa attgctagcg   1200
   cttccagttg catatggtag atcattgctg atgccctact cactgagaaa tagccagaag   1260
   tag                                                                 1263
<210> 14
   <211> 420
   <212> PRT
   <213> Oryza sp.
<400> 14
   Met Ala Ala Ile Thr Leu Leu Arg Ser Ala Ser Leu Pro Gly Leu Ser
   1               5                   10                  15
   Asp Ala Leu Ala Arg Asp Ala Ala Ala Val Gln His Val Cys Ser Ser
               20                  25                  30
   Tyr Leu Pro Asn Asn Lys Glu Lys Lys Arg Arg Trp Ile Leu Cys Ser
           35                  40                  45
   Leu Lys Tyr Ala Cys Leu Gly Val Asp Pro Ala Pro Gly Glu Ile Ala
       50                  55                  60
   Arg Thr Ser Pro Val Tyr Ser Ser Leu Thr Val Thr Pro Ala Gly Glu
   65                  70                  75                  80
   Ala Val Ile Ser Ser Glu Gln Lys Val Tyr Asp Val Val Leu Lys Gln
                   85                  90                  95
   Ala Ala Leu Leu Lys Arg His Leu Arg Pro Gln Pro His Thr Ile Pro
               100                 105                 110
   Ile Val Pro Lys Asp Leu Asp Leu Pro Arg Asn Gly Leu Lys Gln Ala
           115                 120                 125
   Tyr His Arg Cys Gly Glu Ile Cys Glu Glu Tyr Ala Lys Thr Phe Tyr
       130                 135                 140
   Leu Gly Thr Met Leu Met Thr Glu Asp Arg Arg Arg Ala Ile Trp Ala
   145                 150                 155                 160
   Ile Tyr Val Trp Cys Arg Arg Thr Asp Glu Leu Val Asp Gly Pro Asn
                   165                 170                 175
   Ala Ser His Ile Thr Pro Ser Ala Leu Asp Arg Trp Glu Lys Arg Leu
   180 185 190
   Asp Asp Leu Phe Thr Gly Arg Pro Tyr Asp Met Leu Asp Ala Ala Leu
   195 200 205
   Ser Asp Thr Ile Ser Lys Phe Pro Ile Asp Ile Gln Pro Phe Arg Asp
   210 215 220
   Met Ile Glu Gly Met Arg Ser Asp Leu Arg Lys Thr Arg Tyr Lys Asn
   225 230 235 240
   Phe Asp Glu Leu Tyr Met Tyr Cys Tyr Tyr Val Ala Gly Thr Val Gly
   245 250 255
   Leu Met Ser Val Pro Val Met Gly Ile Ala Pro Glu Ser Lys Ala Thr
   260 265 270
   Thr Glu Ser Val Tyr Ser Ala Ala Leu Ala Leu Gly Ile Ala Asn Gln
   275 280 285
   Leu Thr Asn Ile Leu Arg Asp Val Gly Glu Asp Ala Arg Arg Gly Arg
   290 295 300
   Ile Tyr Leu Pro Gln Asp Glu Leu Ala Glu Ala Gly Leu Ser Asp Glu
   305 310 315 320
   Asp Ile Phe Asn Gly Val Val Thr Asn Lys Trp Arg Ser Phe Met Lys
   325 330 335
   Arg Gln Ile Lys Arg Ala Arg Met Phe Phe Glu Glu Ala Glu Arg Gly
   340 345 350
   Val Thr Glu Leu Ser Gln Ala Ser Arg Trp Pro Val Trp Ala Ser Leu
   355 360 365
   Leu Leu Tyr Arg Gln Ile Leu Asp Glu Ile Glu Ala Asn Asp Tyr Asn
   370 375 380
   Asn Phe Thr Lys Arg Ala Tyr Val Gly Lys Ala Lys Lys Leu Leu Ala
   385 390 395 400
   Leu Pro Val Ala Tyr Gly Arg Ser Leu Leu Met Pro Tyr Ser Leu Arg
   405 410 415
   Asn Ser Gln Lys
   420
<210> 15
   <211> 1260
   <212> DNA
   <213> Capsicum annuum
<400> 15
   atgtctgttg ccttgttatg ggttgtttct ccttgtgacg tctcaaacgg gacaggattc      60
   ttggtatccg ttcgtgaggg aaaccggatt tttgattcgt cggggcgtag gaatttggcg     120
   tgcaatgaga gaatcaagag aggaggtgga aaacaaaggt ggagttttgg ttcttacttg     180
   ggaggagcac aaactggaag tggacggaaa ttttctgtac gttctgctat cgtggctact     240
   ccggctggag aaatgacgat gtcatcagaa cggatggtat atgatgtggt tttgaggcag     300
   gcagccttgg tgaagagaca gctgagatcg accgatgagt tagatgtgaa gaaggatata     360
   cctattccgg ggactttggg cttgttgagt gaagcatatg ataggtgtag tgaagtatgt     420
   gcagagtacg caaagacgtt ttacttagga acgatgctaa tgactccgga gagaagaaag     480
   gctatctggg caatatacgt atggtgcagg agaacagacg aacttgttga tggtccgaat     540
   gcatcacaca ttactccggc ggccttagat aggtgggaag acaggctaga agatgttttc     600
   agtggacggc catttgacat gctcgatgct gctttgtccg acacagtttc caaatttcca     660
   gttgatattc agccattcag agatatgatt gaaggaatgc gtatggactt gaggaagtca     720
   agatacagaa actttgacga actataccta tattgttatt acgttgctgg tacggttggg     780
   ttgatgagtg ttccaattat gggcatcgca cctgaatcaa aggcaacaac ggagagcgta     840
   tataatgctg ctttggcttt ggggatcgca aatcagctga ccaacatact tagagatgtt     900
   ggagaagatg ccagaagagg aagagtctat ttgcctcaag atgaattagc acaggcaggt     960
   ctatccgacg aagacatatt tgctggaaga gtgaccgata aatggagaat cttcatgaag    1020
   aaacaaattc agagggcaag aaagttcttt gacgaggcag agaaaggagt gaccgaattg    1080
   agcgcagcta gtagatggcc tgtgttggca tctctgctgt tgtaccgcag gatactggac    1140
   gagatcgaag ccaatgacta caacaacttc acaaagagag cttatgtgag caaaccaaag    1200
   aagttgattg cattacctat tgcatatgca aaatctcttg tgccttctac aagaacatga    1260
<210> 16
   <211> 1239
   <212> DNA
   <213> Lycopersicon esculentum
<400> 16
   atgtctgttg ccttgttatg ggttgtttct ccttgtgacg tctcaaatgg gacaagtttc     60
   atggaatcag tccgggaggg aaaccgtttt tttgattcat cgaggcatag gaatttggtg    120
   tccaatgaga gaatcaatag aggtggtgga aagcaaacta ataatggacg gaaattttct    180
   gtacggtctg ctattttggc tactccatct ggagaacgga cgatgacatc ggaacagatg    240
   gtctatgatg tggttttgag gcaggcagcc ttggtgaaga ggcaactgag atctaccaat    300
   gagttagaag tgaagccgga tatacctatt ccggggaatt tgggcttgtt gagtgaagca    360
   tatgataggt gtggtgaagt atgtgcagag tatgcaaaga cgtttaactt aggaactatg    420
   ctaatgactc ccgagagaag aagggctatc tgggcaatat atgtatggtg cagaagaaca    480
   gatgaacttg ttgatggccc aaacgcatca tatattaccc cggcagcctt agataggtgg    540
   gaaaataggc tagaagatgt tttcaatggg cggccatttg acatgctcga tggtgctttg    600
   tccgatacag tttctaactt tccagttgat attcagccat tcagagatat gattgaagga    660
   atgcgtatgg acttgagaaa atcgagatac aaaaacttcg acgaactata cctttattgt    720
   tattatgttg ctggtacggt tgggttgatg agtgttccaa ttatgggtat cgcccctgaa    780
   tcaaaggcaa caacagagag cgtatataat gctgctttgg ctctggggat cgcaaatcaa    840
   ttaactaaca tactcagaga tgttggagaa gatgccagaa gaggaagagt ctacttgcct    900
   caagatgaat tagcacaggc aggtctatcc gatgaagata tatttgctgg aagggtgacc    960
   gataaatgga gaatctttat gaagaaacaa atacataggg caagaaagtt ctttgatgag   1020
   gcagagaaag gcgtgacaga attgagctca gctagtagat tccctgtatg ggcatctttg   1080
   gtcttgtacc gcaaaatact agatgagatt gaagccaatg actacaacaa cttcacaaag   1140
   agagcatatg tgagcaaatc aaagaagttg attgcattac ctattgcata tgcaaaatct   1200
   cttgtgcctc ctacaaaaac tgcctctctt caaagataa                          1239
<210> 17
   <211> 891
   <212> DNA
   <213> Erwinia sp.
<400> 17
   atggcagttg gctcgaaaag ttttgcgaca gcctcaaagt tatttgatgc aaaaacccgg 60
   cgcagcgtac tgatgctcta cgcctggtgc cgccattgtg acgatgttat tgacgatcag 120
   acgctgggct ttcaggcccg gcagcctgcc ttacaaacgc ccgaacaacg tctgatgcaa 180
   cttgagatga aaacgcgcca ggcctatgca ggatcgcaga tgcacgaacc ggcgtttgcg 240
   gcttttcagg aagtggctat ggctcatgat atcgccccgg cttacgcgtt tgatcatctg 300
   gaaggcttcg cgatggatgt acgcgaagcg caatacagcc aactggatga tacgctgcgc 360
   tattgctatc acgttgcagg cgttgtcggc ttgatgatgg cgcaaatcat gggcgtgcgg 420
   gataacgcca cgctggaccg cgcctgtgac cttgggctgg catttcagtt gaccaatatt 480
   gctcgcgata ttgtggacga tgcgcatgcg ggccgctgtt atctgccggc aagctggctg 540
   gagcatgaag gtctgaacaa agagaattat gcggcacctg aaaaccgtca ggcgctgagc 600
   cgtatcgccc gacgtttggt gcaggaagca gaaccttact atttgtctgc cacagccggc 660
   ctggcagggt tgcccctgcg ttccgcctgg gcaatcgcta cggcgaagca ggtttaccgg 720
   aaaataggtg tcaaagttga acaggccggt cagcaagcct gggatcagcg gcagtcaacg 780
   accacgcccg aaaaattaac gctgctgctg gccgcctctg gtcaggccct tacttcccgg 840
   atgcgggctc atcctccccg ccctgcgcat ctctggcagc gcccgctcta g 891
<210> 18
   <211> 1479
   <212> DNA
   <213> Erwinia sp.
<400> 18
   atgaaaccaa ctacggtaat tggtgcaggc ttcggtggcc tggcactggc aattcgtcta 60
   caagctgcgg ggatccccgt cttactgctt gaacaacgtg ataaacccgg cggtcgggct 120
   tatgtctacg aggatcaggg gtttaccttt gatgcaggcc cgacggttat caccgatccc 180
   agtgccattg aagaactgtt tgcactggca ggaaaacagt taaaagagta tgtcgaactg 240
   ctgccggtta cgccgtttta ccgcctgtgt tgggagtcag ggaaggtctt taattacgat 300
   aacgatcaaa cccggctcga agcgcagatt cagcagttta atccccgcga tgtcgaaggt 360
   tatcgtcagt ttctggacta ttcacgcgcg gtgtttaaag aaggctatct gaagctcggt 420
   actgtccctt ttttatcgtt cagagacatg cttcgcgccg cacctcaact ggcgaaactg 480
   caggcatgga gaagcgttta cagtaaggtt gccagttaca tcgaagatga acatctgcgc 540
   caggcgtttt ctttccactc gctgttggtg ggcggcaatc ccttcgccac ctcatccatt 600
   tatacgttga tacacgcgct ggagcgtgag tggggcgtct ggtttccgcg tggcggcacc 660
   ggcgcattag ttcaggggat gataaagctg tttcaggatc tgggtggcga agtcgtgtta 720
   aacgccagag tcagccatat ggaaacgaca ggaaacaaga ttgaagccgt gcatttagag 780
   gacggtcgca ggttcctgac gcaagccgtc gcgtcaaatg cagatgtggt tcatacctat 840
   cgcgacctgt taagccagca ccctgccgcg gttaagcagt ccaacaaact gcagactaag 900
   cgcatgagta actctctgtt tgtgctctat tttggtttga atcaccatca tgatcagctc 960
   gcgcatcaca cggtttgttt cggcccgcgt taccgcgagc tgattgacga aatttttaat 1020
   catgatggcc tcgcagagga cttctcactt tatctgcacg cgccctgtgt cacggattcg 1080
   tcactggcgc ctgaaggttg cggcagttac tatgtgttgg cgccggtgcc gcatttaggc 1140
   accgcgaacc tcgactggac ggttgagggg ccaaaactac gcgaccgtat ttttgcgtac 1200
   cttgagcagc attacatgcc tggcttacgg agtcagctgg tcacgcaccg gatgtttacg 1260
   ccgtttgatt ttcgcgacca gcttaatgcc tatcatggct cagccttttc tgtggagccc 1320
   gttcttaccc agagcgcctg gtttcggccg cataaccgcg ataaaaccat tactaatctc 1380
   tacctggtcg gcgcaggcac gcatcccggc gcaggcattc ctggcgtcat cggctcggca 1440
   aaagcgacag caggtttgat gctggaggat ctgatttga 1479
<210> 19
   <211> 1488
   <212> DNA
   <213> Erwinia sp.
<400> 19
   atggcggccg ccaaaccaac tacggtaatt ggtgcaggct tcggtggcct ggcactggca 60
   attcgtctac aagctgcggg gatccccgtc ttactgcttg aacaacgtga taaacccggc 120
   ggtcgggctt atgtctacga ggatcagggg tttacctttg atgcaggccc gacggttatc 180
   accgatccca gtgccattga agaactgttt gcactggcag gaaaacagtt aaaagagtat 240
   gtcgaactgc tgccggttac gccgttttac cgcctgtgtt gggagtcagg gaaggtcttt 300
   aattacgata acgatcaaac ccggctcgaa gcgcagattc agcagtttaa tccccgcgat 360
   gtcgaaggtt atcgtcagtt tctggactat tcacgcgcgg tgtttaaaga aggctatctg 420
   aagctcggta ctgtcccttt tttatcgttc agagacatgc ttcgcgccgc acctcaactg 480
   gcgaaactgc aggcatggag aagcgtttac agtaaggttg ccagttacat cgaagatgaa 540
   catctgcgcc aggcgttttc tttccactcg ctgttggtgg gcggcaatcc cttcgccacc 600
   tcatccattt atacgttgat acacgcgctg gagcgtgagt ggggcgtctg gtttccgcgt 660
   ggcggcaccg gcgcattagt tcaggggatg ataaagctgt ttcaggatct gggtggcgaa 720
   gtcgtgttaa acgccagagt cagccatatg gaaacgacag gaaacaagat tgaagccgtg 780
   catttagagg acggtcgcag gttcctgacg caagccgtcg cgtcaaatgc agatgtggtt 840
   catacctatc gcgacctgtt aagccagcac cctgccgcgg ttaagcagtc caacaaactg 900
   cagactaagc gcatgagtaa ctctctgttt gtgctctatt ttggtttgaa tcaccatcat 960
   gatcagctcg cgcatcacac ggtttgtttc ggcccgcgtt accgcgagct gattgacgaa 1020
   atttttaatc atgatggcct cgcagaggac ttctcacttt atctgcacgc gccctgtgtc 1080
   acggattcgt cactggcgcc tgaaggttgc ggcagttact atgtgttggc gccggtgccg 1140
   catttaggca ccgcgaacct cgactggacg gttgaggggc caaaactacg cgaccgtatt 1200
   tttgcgtacc ttgagcagca ttacatgcct ggcttacgga gtcagctggt cacgcaccgg 1260
   atgtttacgc cgtttgattt tcgcgaccag cttaatgcct atcatggctc agccttttct 1320
   gtggagcccg ttcttaccca gagcgcctgg tttcggccgc ataaccgcga taaaaccatt 1380
   actaatctct acctggtcgg cgcaggcacg catcccggcg caggcattcc tggcgtcatc 1440
   ggctcggcaa aagcgacagc aggtttgatg ctggaggatc tgatttga 1488
<210> 20
   <211> 839
   <212> DNA
   <213> Oryza sp.
<400> 20
   gttaatcatg gtgtaggcaa cccaaataaa acaccaaaat atgcacaagg cagtttgttg 60
   tattctgtag tacagacaaa actaaaagta atgaaagaag atgtggtgtt agaaaaggaa 120
   acaatatcat gagtaatgtg tgagcattat gggaccacga aataaaaaga acattttgat 180
   gagtcgtgta tcctcgatga gcctcaaaag ttctctcacc ccggataaga aacccttaag 240
   caatgtgcaa agtttgcatt ctccactgac ataatgcaaa ataagatatc atcgatgaca 300
   tagcaactca tgcatcatat catgcctctc tcaacctatt cattcctact catctacata 360
   agtatcttca gctaaatgtt agaacataaa cccataagtc acgtttgatg agtattaggc 420
   gtgacacatg acaaatcaca gactcaagca agataaagca aaatgatgtg tacataaaac 480
   tccagagcta tatgtcatat tgcaaaaaga ggagagctta taagacaagg catgactcac 540
   aaaaattcat ttgcctttcg tgtcaaaaag aggagggctt tacattatcc atgtcatatt 600
   gcaaaagaaa gagagaaaga acaacacaat gctgcgtcaa ttatacatat ctgtatgtcc 660
   atcattattc atccaccttt cgtgtaccac acttcatata tcatgagtca cttcatgtct 720
   ggacattaac aaactctatc ttaacattta gatgcaagag cctttatctc actataaatg 780
   cacgatgatt tctcattgtt tctcacaaaa agcattcagt tcattagtcc tacaacaac 839
<210> 21
   <211> 642
   <212> DNA
   <213> Oryza sp.
<400> 21
   aagcttgcgc gcggaatacg gtggagatgg gttgggaacc ctggattcca aacacagccc 60
   aagtctatcc aaaatgttta gacaagaaaa tacgtaacaa gttggtttac agaaatacga 120
   attagatcaa tcctgcacta caagtagagt aaagtggtga tttctcttaa atctctcgaa 180
   tggtgattta agaattcagt gcaaaccaaa tccttgctat aatcaaatgt tcggtaccgc 240
   atcaacggaa caataaaaag cgcctggcgt accataattt tgtcattctt gttgaaattt 300
   gtaatttaag atgcatgagg ccacacgacc ttaatgttca acgtgtcatg cattagtgaa 360
   ataatagctc acaaaacgca acaaatatag ctagataacg gttgcaatcc ttaccaaact 420
   aacgtataaa gtgagcgatg agtcatatca ttatctcccg cctgctaacc atcgtgtaca 480
   ccatccgatc acaaaaatga caacttctag ggatgaacct ggacaaggtt tagggtttag 540
   ggatgaatct ggacaaatga ttgttcaggt tcatccctag atgttggttt ctcctgacgg 600
   gacggaggga gtatatgtga tggacacaaa agttactttc at 642
<210> 22
   <211> 190
   <212> DNA
   <213> artificial sequence
<220>
   <223> SYNTHETIC - INTRON
<400> 22
   gtaaatttct agtttttctc cttcattttc ttggttagga cccttttctc tttttatttt 60
   tttgagcttt gatctttctt taaactgatc tattttttaa ttgattggtt atcgtgtaaa 120
   tattacatag ctttaactga taatctgatt actttatttc gtgtgtcttt gatcatcttg 180
   atagttacag 190
<210> 23
   <211> 171
   <212> DNA
   <213> artificial sequence
<220>
   <223> TRANSIT PEPTIDE
<400> 23
   atggcttcta tgatatcctc ttccgctgtg acaacagtca gccgtgcctc tagggggcaa 60
   tccgccgcag tggctccatt cggcggcctc aaatccatga ctggattccc agtgaagaag 120
   gtcaacactg acattacttc cattacaagc aatggtggaa gagtaaagtg c 171
<210> 24
   <211> 254
   <212> DNA
   <213> artificial sequence
<220>
   <223> NOS TERMINATOR
<400> 24
   gatcgttcaa acatttggca ataaagtttc ttaagattga atcctgttgc cggtcttgcg 60
   atgattatca tataatttct gttgaattac gttaagcatg taataattaa catgtaatgc 120
   atgacgttat ttatgagatg ggtttttatg attagagtcc cgcaattata catttaatac 180
   gcgatagaaa acaaaatata gcgcgcaaac taggataaat tatcgcgcgc ggtgtcatct 240
   atgttactag atcg 254
<210> 25
   <211> 193
   <212> DNA
   <213> artificial sequence
<220>
   <223> CAMV 35S TERMINATOR REGION
<400> 25
   gctgaaatca ccagtctctc tctacaaatc tatctctctc tataataatg tgtgagtagt 60
   tcccagataa gggaattagg gttcttatag ggtttcgctc atgtgttgag catataagaa 120
   acccttagta tgtatttgta tttgtaaaat acttctatca ataaaatttc taattcctaa 180
   aaccaaaatc cag 193
<210> 26
   <211> 238
   <212> DNA
   <213> artificial sequence
<220>
   <223> POTATO PROTEINASE INHIBITOR GENE TERMINATOR REGION
<400> 26
   ccctagactt gtccatcttc tggattggcc aacttaatta atgtatgaaa taaaaggatg     60
   cacacatagt gacatgctaa tcactataat gtgggcatca aagttgtgtg ttatgtgtaa    120
   ttactaatta tctgaataag agaaagagat catccatatt tcttatccta aatgaatgtc    180
   acgtgtcttt ataattcttt gatgaaccag atgcatttta ttaaccaatt ccatatac      238
<210> 27
   <211> 2321
   <212> DNA
   <213> Lycopersicon esculentum
<400> 27
   gggtttatct cgcaagtgtg gctatggtgg gacgtgtcaa attttggatt gtagccaaac     60
   atgagatttg atttaaaggg aattggccaa atcaccgaaa gcaggcatct tcatcataaa    120
   ttagtttgtt tatttataca gaattatacg cttttactag ttatagcatt cggtatcttt    180
   ttctgggtaa ctgccaaacc accacaaatt tcaagtttcc atttaactct tcaacttcaa 240
   cccaaccaaa tttatttgct taattgtgca gaaccactcc ctatatcttc taggtgcttt 300
   cattcgttcc gagtaaaatg cctcaaattg gacttgtttc tgctgttaac ttgagagtcc 360
   aaggtagttc agcttatctt tggagctcga ggtcgtcttc tttgggaact gaaagtcgag 420
   atggttgctt gcaaaggaat tcgttatgtt ttgctggtag cgaatcaatg ggtcataagt 480
   taaagattcg tactccccat gccacgacca gaagattggt taaggacttg gggcctttaa 540
   aggtcgtatg cattgattat ccaagaccag agctggacaa tacagttaac tatttggagg 600
   ctgcattttt atcatcaacg ttccgtgctt ctccgcgccc aactaaacca ttggagattg 660
   ttattgctgg tgcaggtttg ggtggtttgt ctacagcaaa atatttggca gatgctggtc 720
   acaaaccgat actgctggag gcaagggatg ttctaggtgg aaaggtagct gcatggaaag 780
   atgatgatgg agattggtac gagactggtt tgcatatatt ctttggggct tacccaaata 840
   ttcagaacct gtttggagaa ttagggatta acgatcgatt gcaatggaag gaacattcaa 900
   tgatatttgc aatgccaagc aagccaggag aattcagccg ctttgatttc tccgaagctt 960
   tacccgctcc tttaaatgga attttagcca tcttaaagaa taacgaaatg cttacatggc 1020
   cagagaaagt caaatttgca attggactct tgccagcaat gcttggaggg caatcttatg 1080
   ttgaagctca agatgggata agtgttaagg actggatgag aaagcaaggt gtgccggaca 1140
   gggtgacaga tgaggtgttc attgctatgt caaaggcact caactttata aaccctgacg 1200
   aactttcaat gcagtgcatt ttgatcgcat tgaacaggtt tcttcaggag aaacatggtt 1260
   caaaaatggc ctttttagat ggtaatcctc ctgagagact ttgcatgccg attgttgaac 1320
   acattgagtc aaaaggtggc caagtcagac tgaactcacg aataaaaaag attgagctga 1380
   atgaggatgg aagtgtcaag agttttatac tgagtgacgg tagtgcaatc gagggagatg 1440
   cttttgtgtt tgccgctcca gtggatattt tcaagcttct attgcctgaa gactggaaag 1500
   agattccata tttccaaaag ttggagaagt tagtcggagt acctgtgata aatgtacata 1560
   tatggtttga cagaaaactg aagaacacat atgatcattt gctcttcagc agaagctcac 1620
   tgctcagtgt gtatgctgac atgtctgtta catgtaagga atattacaac cccaatcagt 1680
   ctatgttgga attggttttt gcacctgcag aagagtggat atctcgcagc gactcagaaa 1740
   ttattgatgc aacgatgaag gaactagcaa cgctttttcc tgatgaaatt tcagcagatc 1800
   aaagcaaagc aaaaatattg aagtaccatg ttgtcaaaac tccgaggtct gtttataaaa 1860
   ctgtgccagg ttgtgaaccc tgtcggcctt tacaaagatc cccaatagag ggttttattt 1920
   tagccggtga ctacacgaaa cagaaatact tggcttcaat ggaaggcgct gtcttatcag 1980
   gaaagctttg tgctcaagct attgtacagg attatgagtt acttgttgga cgtagccaaa 2040
   agaagttgtc ggaagcaagc gtagtttagc tttgtggtta ttatttagct tctgtacact 2100
   aaatttatga tgcaagaagc gttgtacaca acatatagaa gaagagtgcg aggtgaagca 2160
   agtaggagaa atgttaggaa agctcctata caaaaggatg gcatgttgaa gattagcatc 2220
   tttttaatcc caagtttaaa tataaagcat attttatgta ccactttctt tatctggggt 2280
   ttgtaatccc tttatatctt tatgcaatct ttacgttagt t 2321
<210> 28
   <211> 1749
   <212> DNA
   <213> Capsicum annuum
<400> 28
   atgccccaaa ttggacttgt ttctgctgtc aacttgagag tccaaggtaa ttcagcttat 60
   ctttggagct cgaggtcttc tttgggaact gatagtcaag atggttgctc gcaaaggaat 120
   tcgttatgtt ttggtggtag tgactcaatg agtcataggt taaagattcg taatccccat 180
   tccataacga gaagattggc taaggatttc cggcctttaa aggttgtttg cattgattat 240
   ccaaggccag agctagacaa tacagttaac tatttggagg ctgcattctt atcatcatca 300
   ttccgatctt ctccgcgccc aaccaaacca ctggagattg ttattgctgg tgcaggtttg 360
   ggtggtttgt ctacagcaaa atatttggca gatgctggtc acaaaccaat actgctggag 420
   gcaagggatg ttctaggtgg aaaggtagct gcatggaaag atgatgatgg agattggtat 480
   gagactggtt tgcacatatt ctttggggct tacccaaata tgcagaacct atttggagaa 540
   ttagggataa atgatcgatt gcaatggaag gaacattcga tgatatttgc aatgccaaac 600
   aagccaggag aattcagccg ctttgatttc cccgaagctt tacctgctcc tttaaatgga 660
   attttggcaa tcctaaagaa caatgaaatg cttacatggc cagaaaaagt caaatttgca 720
   attggactct tgccagcaat gcttggtggg caatcttatg ttgaagctca agacgggata 780
   agtgttaagg actggatgag aaaacaaggt gtgccggata gggtgacgga tgaggtgttc 840
   atcgccatgt caaaggcact taacttcata aatcctgatg agctttcgat gcagtgcatc 900
   ttgatcgcgt tgaacagatt tcttcaggag aaacatggtt caaaaatggc ctttttagat 960
   ggtaatcctc ctgagagact ttgcatgccg attgttgaac atatcgagtc aaaaggtgga 1020
   caagtcagac tgaactcacg aataaaaaag attgagctga atgaggatgg aagtgtcaag 1080
   tgttttatac tgaacgatgg tagtacaatt gagggagatg cttttgtgtt tgcgactcca 1140
   gtggatattt tcaagcttct tttgcctgaa gactggaaag agattccata tttccaaaag 1200
   ttggagaagt tagttggagt acctgtgata aatgtccata tatggtttga cagaaaactg 1260
   aagaacacat ctgataattt gctcttcagc agaagcccac tgctcagtgt gtatgctgac 1320
   atgtccgtca catgtaagga atattacgac cccaacaagt ccatgttgga attggtcttt 1380
   gcgcctgcag aagagtgggt atctcgcagt gactctgaaa ttattgatgc tacaatgaag 1440
   gaactagcaa agctatttcc tgatgaaatt tcggcggatc agagcaaagc aaaaatattg 1500
   aagtatcatg ttgtcaaaac tccaaggtct gtatataaaa ctgtgccagg ttgtgaaccc 1560
   tgtcggctct tgcaaagatc ccctgtagag gggttttatt tagctggtga ctacacgaaa 1620
   cagaaatact tggcttcaat ggaaggtgct gtcttatcag gaaagctttg tgcacaagct 1680
   attgtacagg attacgagtt acttgttggc cggagccaga ggaagttggc agaaacaagt 1740
   gtagtttag 1749
<210> 29
   <211> 2264
   <212> DNA
   <213> Zea mays
<400> 29
   ctccaaatgc ggaggtctcg actcttctct cttcctccat ctttatcatc gccccacgta 60
   cacacccaat tcctcgcaac tgggctcccc cgcctccacg acactgcccc ccgtctcaag 120
   tccgccgcct ccattcttca gctctcctat cctccgccta gaatatcttc atcggtattt 180
   taccaacctg gatcaattta ctcacgatac tctgaagcgt atacatatgc catatgggaa 240
   atgacttcat agctgtgggt tgtcttatgg ctccttgaat ttgcagtagt ctgcctgtac 300
   ctattggctg aagcagagct gacccccact ttatcaagag ttgctcaacg atggacactg 360
   gctgcctgtc atctatgaat attactggag ctagccagac aagatctttt gcggggcaac 420
   ttcctcctca gagatgtttt gcgagtagtc actatacaag ctttgccgtg aaaaaacttg 480
   tctcaaggaa taaaggaagg agatcacacc gtagacatcc tgccttgcag gttgtctgca 540
   aggattttcc aagacctcca ctagaaagca caataaacta tttggaagct ggacagctct 600
   cttcattttt tagaaacagc gaacgcccca gtaagccgtt gcaggtcgtg gttgctggtg 660
   caggattggc tggtctatca acagcgaagt atctggcaga tgctggccat aaacccatat 720
   tgcttgaggc aagagatgtt ttgggtggaa aggtagctgc ttggaaggat gaagatggag 780
   attggtacga gactgggctt catatatttt ttggagctta tcccaacata cagaatctgt 840
   ttggcgagct taggattgag gatcgtttgc agtggaaaga acactctatg atattcgcca 900
   tgccaaacaa gccaggagaa ttcagccggt tcgatttccc agaaactttg ccagcaccta 960
   taaatgggat atgggccata ttgagaaaca atgaaatgct tacttggccg gagaaggtga 1020
   agtttgcaat cggacttctg ccagcaatgg ttggtggtca accttatgtt gaagctcaag 1080
   atggcttaac cgtttcagaa tggatgaaaa agcagggtgt tcctgatcgg gtgaacgatg 1140
   aggtttttat tgcaatgtcc aaggcactca atttcataaa tcctgatgag ctatctatgc 1200
   agtgcatttt gattgctttg aaccgatttc ttcaggagaa gcatggttct aaaatggcat 1260
   tcttggatgg taatccgcct gaaaggctat gcatgcctat tgttgatcac attcggtcta 1320
   ggggtggaga ggtccgcctg aattctcgta ttaaaaagat agagctgaat cctgatggaa 1380
   ctgtaaaaca cttcgcactt agtgatggaa ctcaaataac tggagatgct tatgtttgtg 1440
   caacaccagt cgatatcttc aagcttcttg tacctcaaga gtggagtgaa attacttatt 1500
   tcaagaaact ggagaagttg gtgggagttc ctgttatcaa tgttcatata tggtttgaca 1560
   gaaaactgaa caacacatat gaccaccttc ttttcagcag gagttcactt ttaagtgtct 1620
   atgcagacat gtcagtaacc tgcaaggaat actatgaccc aaaccgttca atgctggagt 1680
   tggtctttgc tcctgcagac gaatggattg gtcgaagtga cactgaaatc atcgatgcaa 1740
   ctatggaaga gctagccaag ttatttcctg atgaaattgc tgctgatcag agtaaagcaa 1800
   agattcttaa gtatcatatt gtgaagacac cgagatcggt ttacaaaact gtcccaaact 1860
   gtgagccttg ccggcctctc caaaggtcac ctatcgaagg tttctatcta gctggtgatt 1920
   acacaaagca gaaatacctg gcttctatgg aaggtgcagt cctatccggg aagctttgtg 1980
   cccagtccat agtgcaggat tatagcaggc tcgcactcag gagccagaaa agcctacaat 2040
   caggagaagt tcccgtccca tcttagttgt agttggcttt agctatcgtc atccccactg 2100
   ggtgctatct tatctcctat ttcaatggga acccacccaa tggtcatgtt ggagacaaca 2160
   cctgttatgg tcctttgacc atctcgtggt gactgtagtt gatgtcatat tcggatatat 2220
   atgtaaaagg acctgcatag caattgttag accttggaaa aaaa 2264
<210> 30
   <211> 2027
   <212> DNA
   <213> Oryza sp.
<400> 30
   gtttatgaca gcatctgcca gatattttgc aggacaactt cctactcata ggtgcttcgc   60
   aagtagcagc atccaagcac tgaaaggtag tcagcatgtg agctttggag tgaaatctct  120
   tgtcttaagg aataaaggaa aaagattccg tcggaggctc ggtgctctac aggttgtttg  180
   ccaggacttt ccaagacctc cactagaaaa cacaataaac tttttggaag ctggacaact  240
   atcctcattt ttcagaaaca gtgaacaacc cactaaacca ttacaggtcg tgattgctgg  300
   agcaggatta gctggtttat caacggcaaa atatctggca gatgctggtc ataaacccat  360
   attgcttgag gcaagggatg ttttgggtgg aaagatagct gcttggaagg atgaagatgg  420
   agattggtat gaaactgggc ttcatatctt ttttggagct tatcccaaca tacagaactt  480
   gtttggcgag cttggtatta atgatcggtt gcaatggaag gaacactcca tgatatttgc  540
   catgccaaac aagccaggag aatccagccg gtttgatttt cctgaaacat tgcctgcacc  600
   cttaaatgga atatgggcca tactaagaaa caatgaaatg ctaacttggc cagagaaggt  660
   gaagtttgct cttggacttt tgccagcaat ggttggtggc caagcttatg ttgaagctca  720
   agatggtttt actgtttctg agtggatgaa aaagcagggt gttcctgatc gagtgaacga  780
   tgaagttttc attgcaatgt caaaggcact taatttcata aatcctgatg agttatccat  840
   gcagtgcatt ctgattgctt taaaccgatt tcttcaggag aagcatggtt ctaagatggc  900
   attcttggat ggtaatcctc ctgaaaggtt atgcatgcct attgttgacc atgttcgctc  960
   tttgggtggt gaggttcggc tgaattctcg tattcagaaa atagaactta atcctgatgg 1020
   aacagtgaaa cactttgcac ttaccgatgg aactcaaata actggagatg cttatgtttt 1080
   tgcaacacca gttgatatct tgaagcttct tgtacctcaa gagtggaaag aaatatctta 1140
   tttcaagaag ctggagaagt tggtgggagt tcctgttata aatgttcata tatggtttga 1200
   tagaaaactg aagaacacat atgaccacct tcttttcagc aggagttcac ttttaagtgt 1260
   ttatgcggac atgtcagtaa cttgcaagga atactatgat ccaagccgtt caatgctgga 1320
   gttggtcttt gctcctgcag aggaatgggt tggacggagt gacactgaaa tcatcgaagc 1380
   aactatgcaa gagctagcca agctatttcc tgatgaaatt gctgctgatc agagtaaagc 1440
   aaagattctg aagtatcatg ttgtgaagac accaagatct gtttacaaga ctatcccgga 1500
   ctgtgaacct tgccgacctc tgcaaagatc accgattgaa gggttctatc tagctggtga 1560
   ctacacaaag cagaaatatt tggcttcgat ggagggtgca gttctatctg ggaagctttg 1620
   tgctcagtct gtagtggagg attataaaat gctatctcgt aggagcctga aaagtctgca 1680
   gtccgaagtt cctgttgcct cctagttgta gtcaggacta ttcccaatgg tgtgtgtgtc 1740
   atcatcccct agtcagtttt tttctattta gtgggtgccc aactctccac caatttacac 1800
   atgatggaac ttgaaagatg cctattttgg tcttatcata tttctgtaaa gttgatttgt 1860
   gactgagagc tgatgccgat atgccacgct ggagaaaaag aacattatgt aaaacgacct 1920
   gcatagtaat tcttagactt ttgcaaaagg caaaaggggt aaagcgacct tttttttcta 1980
   tgtgaaggga ttaagagacc ttaaaaaaaa aaaaaaaaaa aaaaaaa 2027
<210> 31
   <211> 1931
   <212> DNA
   <213> Lycopersicon esculentum
<400> 31
   ttttgtcttt ctttcttgtt aacccatttt cttgatattt aacaagaaaa gtttctttct 60
   tttttttcct accctcataa ttgggtagag aacaattccc atggctactt cttcagctta 120
   tctttcttgt cctgcaactt ctgctactgg aaagaaacat gttttcccaa atgggtcacc 180
   tggattcttg gtttttggtg gtacccgttt gtccaaccgg ttagtgaccc gaaagtcggt 240
   tattcgggct gatttggatt ctatggtttc tgatatgagt accaacgctc caaaagggct 300
   atttccaccc gagcctgaac attatcgggg gccaaagctg aaagtagcta ttattggagc 360
   tgggcttgca ggcatgtcga ctgctgtgga gctcttggat caaggacatg aggtggatat 420
   atacgaatca aggactttta ttggtgggaa agtgggttct tttgttgata gacgtgggaa 480
   ccacattgaa atgggactgc acgtgttctt tggttgttat aataatctgt tccgtctgtt 540
   gaaaaaggtg ggtgctgaaa aaaatctgct agtgaaggag catactcaca catttgtaaa 600
   taaagggggt gaaatagggg aacttgattt ccgctttcca gttggagcac ccttacatgg 660
   aattaatgca tttctgtcta ctaatcagtt aaagatttat gataaagcta gaaatgctgt 720
   agctcttgcc cttagtccag tggtgcgggc tttagttgat ccggatggtg cattgcagca 780
   gatacgcgat ctagataatg taagcttttc tgagtggttt ctgtctaaag gtgggacgcg 840
   tgctagcatc cagaggatgt gggatcctgt tgcatatgct cttggattca ttgactgtga 900
   taacatgagt gctcggtgta tgctcactat atttgcatta tttgccacaa aaacagaggc 960
   ttccctatta cgcatgctta aaggttctcc tgacgtttat ttgagtggtc caattaagaa 1020
   gtacatcatg gacaaagggg gcaggttcca tctgaggtgg ggatgcagag aggtactcta 1080
   tgagacgtcc tctgatggaa gcatgtatgt tagtgggctt gccatgtcaa aggccactca 1140
   gaagaaaatt gtaaaagctg atgcatatgt ggctgcatgt gatgtccctg gaattaaaag 1200
   attggttcct cagaagtgga gggaattgga attctttgac aacatttaca aattggtcgg 1260
   agtgcctgtt gttaccgtac aactacgcta caatggctgg gttacagagt tgcaggactt 1320
   ggagcgttcg aggcaattga agcgcgctgc aggattggac aatctcctct atacgccaga 1380
   tgcagatttc tcttgctttg cagatcttgc attggcatct ccagatgatt actacattga 1440
   gggacaaggc tcattgcttc aatgtgtcct tacacctggt gacccttaca tgcctctatc 1500
   aaatgatgaa atcattaaaa gagttacaaa gcaggttttg gcattatttc cttcgtccca 1560
   aggtcttgag gttacctggt catcagtttt gaagatagga caatctttat atcgtgaagg 1620
   acctggtaaa gacccattca gacctgatca gaagacgcca gtggaaaatt tctttcttgc 1680
   tggctcatat acaaaacagg actacatcga tagcatggaa ggagcaactc tttcaggtag 1740
   gcaagcttct gcatacatat gtaatgttgg agagcagctg atggcgttgc gtaaaaagat 1800
   cactgctgct gagttgaatg acatctctaa aggtgtgtcc ctatctgatg agttgagtct 1860
   tgtctgatga cagactgcaa atcatccaaa tacaactcag ttaggcatcg cacaaggaag 1920
   aattcttcta a 1931
<210> 32
   <211> 1982
   <212> DNA
   <213> Capsicum annuum
<400> 32
   cacaattcta tggctacttg ttcagcttat ctttgttgtc ctgccacttc tgcttcttta 60
   aagaaacgtg tttttccaga tgggtccgct ggattcttgt tttttggtgg tcgtcgtttg 120
   tcgaaccggt tagtgacccc aaagtctgtc atccgagctg atttgaactc catggtctct 180
   gacatgagta ccaacgctcc aaaagggcta tttccacctg aacctgaaca ttatcggggg 240
   ccaaagctga aagtagctat tattggagct ggccttgcag gcatgtcgac tgctgtggag 300
   ctcttggatc aaggacatga ggtggatata tatgaatcaa ggaccttcat tggtgggaaa 360
   gtgggttctt ttgttgataa acgtgggaac cacattgaaa tgggactgca cgtgttcttt 420
   ggttgctata ataatctatt ccgtctgatg aaaaaggtgg gtgctgaaaa aaatctgcta 480
   gtgaaggagc atactcacac atttgtaaat aaagggggtg aaatagggga gcttgatttc 540
   cgctttccag ttggagcgcc cttacatgga attaatgcat ttttgtctac taatcaacta 600
   aagacttatg ataaagctag aaatgctgta gctcttgccc ttagtccagt ggtgcgggct 660
   ttagttgatc cagatggcgc attgcagcag atacgtgatc tagatagtgt aagcttttct 720
   gattggttta tgtctaaagg agggacgcgc gctagcatcc agaggatgtg ggatcctgtt 780
   gcatatgctc ttggattcat tgactgtgac aatatcagtg ctcggtgtat gctcactata    840
   tttgcattat ttgccactaa aacggaggct tccctactgc gcatgcttaa aggttctcct    900
   gacgtttatt tgagtggtcc aattaagaag tacatcatag acaagggggg aaggttccat    960
   ctgaggtggg gatgcagaga ggtactctac gagacatcct ctgatggaag catgtatgtt   1020
   agcgggcttg ccatgtcaaa ggccactcag aagaaaattg taaaagctga tgcctatgtt   1080
   gccgcatgtg tagtacctgg aattaaaaga ttagtacctc agaagtggag ggaattggaa   1140
   ttctttggca acatttacaa actgattgga gtgcctgttg ttactgtgca actacgatac   1200
   aatggctggg ttacggagtt gcaggacttg gagcgttcaa ggcaatcaaa gcgcgctaca   1260
   ggtttggaca atctcctgta cacgccagat gcagatttct cttgttttgc agaccttgca   1320
   ttggcatctc cagaagatta ttacattgag ggacaaggct cgttgcttca atgtgtcctt   1380
   acgcctggcg acccttacat gcctctacca aatgaagaaa tcataagaag agtgtcaaag   1440
   caggttttgg cgttatttcc ttcttcccaa ggtcttgagg taacctggtc atcagttgtg   1500
   aagattgggc aatccttata tcgtgaagga cctggtaaag acccgttcag acctgatcaa   1560
   aagacgccag tggaaaattt ctttcttgct ggctcatata caaaacagga ctacatcgat   1620
   agtatggaag gggcaactct ttcaggcaga caagcttctg catacatatg tgatgctgga   1680
   gagcagctgt tggcgctgcg aaaaaagatt gctgctgctg agttaaacga gatctctaaa   1740
   ggtgtatcgc tatcggatga gttgagtctt gtctgatgac tgcaaatcat tcagaaatat   1800
   aattcagtta ggcagtgcat aaggaagaat tcttctaaat ttttgagtct cacaattatg   1860
   gaaatcaaaa tatgttttaa aaatgttgta tgtatgtaat attagtaaat cttcatagtg   1920
   atgtatgtat ctattctgcc acgcttcagt ttagtgaaat ggaacttatt gctgcatcaa   1980
   tc                                                                  1982
<210> 33
   <211> 2265
   <212> DNA
   <213> Zea mays
<400> 33
   ccctgccacg acgcccgcga caaatccctg cgcgacggca tcttcgcctc ccatcccctc     60
   ccagcttccc ctcccactcc ggccctcaca caaattgccc ctcttcttct cctcctcttt    120
   acacgctgcc gaccacggct gccgccaacc acccgcccca cccgtccacc gctgccgagt    180
   gctagccatt tggagctgcc gcgccatggc gtccgtggcc gccaccacca cgctggcacc     240
   ggcactcgcc ccgcgccggg cgcggccagg gactgggctc gtgccgccgc gccgggcctc     300
   ggccgtcgct gctcgctcga ccgtaacgtc tccgacatgg cgtcaacgct cccaaaggtt     360
   attcccaccc gagccagagc actacagggg cccgaagctc aaggtggcca tcataggggc     420
   aggccttgcg ggcatgtcca ccgctgttga gctcttggac cagggccatg aggttgattt     480
   gtacgagtcc cgtccgttta tcggtggcaa ggttggctcc tttgttgaca ggcaaggaaa     540
   ccatatcgag atggggctgc atgtgttctt cgggtgctac agcaatctct tccgcctcat     600
   gaagaaggtt ggcgctgata ataatctgct ggtgaaggaa catacccata cttttgtaaa     660
   taaagggggc acgattggtg aacttgattt tcggttcccg gtgggagctc cgttacatgg     720
   cattcaagca ttcctaagaa ctaatcagct caaggtttat gataaagcaa gaaatgcagt     780
   tgctcttgcc cttagtccag ttgttcgggc tctggttgat cctgatggtg cattgcagca     840
   agtgcgggac ttggatgata taagtttcag tgattggttc atgtccaaag ggggtactcg      900
   ggagagtatc acaagaatgt gggatcctgt tcgttacgct ttgggtttca ttgactgtga     960
   taatatcagt gcacgttgca tgcttactat tttcaccttg tttgccacaa agacagaggc    1020
   atccctgtta cgcatgttaa agggttcacc tgatgtttac ttaagtggtc caataaagaa    1080
   gtatataaca gacaggggtg gtaggtttca cttaaggtgg ggatgcagag aggttctcta    1140
   tgagaagtca cctgatggag agacctatgt taagggcctt ctactcacca aggctacaag    1200
   tagagagata atcaaagctg atgcatacgt cgcagcctgt gatgttccag gtatcaaaag    1260
   attacttcca tcagaatgga gggagtggga aatgtttgac aatatctaca agttagatgg    1320
   tgtccctgtt gtcactgtcc agctccgcta caacggatgg gtcactgaac ttcaagattt    1380
   ggagaaatca agacaactgc aaagggcggt tgggttggat aaccttttgt  acacggcgga    1440
   tgcagacttt tcctgttttt cggaccttgc tctctcatct cctgctgatt actacattga    1500
   agggcaaggt tccctgatcc aagctgtgct gactcctgga gatccataca tgccattgcc    1560
   aaacgaggag atcattagta aggttcaaaa gcaggttgta gaactgttcc catcttcccg    1620
   gggcttagaa gttacatggt ccagtgtggt aaagatcgga caatcgctgt accgtgaggc    1680
   tcctggaaac gacccattca ggcctgatca gaagacgccc gttaaaaact tcttcctctc    1740
   tggatcttac acgaaacagg actacatcga cagcatggaa ggagcaactc tctccggcag    1800
   gcgaacgtcg gcctacatct gcggtgccgg ggaggagctg ctggccctcc gaaagaagct    1860
   actcatcgac gacggcgaga aggcgctggg gaacgttcaa gtcctgcagg ctagctgaac    1920
   aacccctcct gcactgcaga gaagcttgga tctttccaac cacacataca tgctggaatg    1980
   gacaaaccaa ccaaccattg tcttttctcg cttcagggtg ctggcgattc ccgcagcaac    2040
   ctcctgtgta tcgtatccaa tttgagcatt agatctgccc cccccccctg caggcgtttc   2100
   tttcctatcc ctgatccgag aagcagggtg tagtctaggt ggctggcata cgggattaca   2160
   tcaggcagtg tgtaagttca gctggaactc gattggtaat tgggatggat gattgatgat   2220
   atatatatag cacacactgt tcttgcgtct tgcaaaaaaa aaaaa                   2265
<210> 34
   <211> 1632
   <212> DNA
   <213> Oryza sp.
<400> 34
   cccacgcgtc cgcccacgcg tccggattgg tgaacttgat tttcggtttc ctgtgggagc     60
   tccgttacat ggtatccaag cattcctacg aactaaccaa ctcaaggttt atgataaagc    120
   aagaaatgcc gttgctcttg ctctaagccc agttgttcga gctcttgttg atccagatgg    180
   tgcattgcag caagtacggg atttggatga tgtaagtttc agcgattggt tcttgtcgaa    240
   aggtggtact cgagagagca tcacaaggat gtgggatcct gttgcctatg ctcttggttt    300
   cattgactgt gataatatca gtgcacgttg catgcttacc attttcactc tgtttgccac    360
   aaaaacagag gcatctttat tacgcatgct aaagggttca cctgatgttt atctgagtgg    420
   tccaataaag aagtacataa cagacagggg tggtaggttt cacctgaggt ggggatgtag    480
   ggaggttctc tatgataagt cacctgatgg ggaaacctat gttaaaggcc ttctcctatc    540
   caaggctaca agtagagaga taatcaaagc agatgcatat gtcgcagctt gtgatgtccc    600
   ggggatcaaa agacttttac cttctgaatg gaggcaatgg gatacatttg acaacatcta    660
   caagttagat ggtgttcctg tagtcacagt acagcttcgt tataatggat gggttacaga    720
   acttcaagat ttggagaaat caagacaact gaaaaaggca gttggcttgg ataatcttct    780
   ctacactcca gatgcagatt tttcatgttt ttcagacctt gcactttcat ctcctgctga    840
   ctactacatt gaaggacaag gttccttgat ccaagctgtg ctaacccctg gcgatcctta    900
   catgccattg ccgaatgagg agataattag caaggttcaa aagcaggtct tagaattgtt    960
   cccgtcatca caaggcttgg aacttacatg gtcgagtgtg gtgaaaatcg gtcaatcatt   1020
   gtaccgcgag tcaccaggaa atgatccatt tagacctgat caaaagacac cagttaaaaa   1080
   cttcttcctg tctggctctt acacaaaaca ggactacatt gacagcatgg aaggggcaac   1140
   tctctcaggc aggagaaccg cggcctacat ctgtggtgca ggagaggagc tgcttcgccc   1200
   tccgaaagaa gctcattgtc gacgacagcg gagaaggcca ggggtaaggt cgacggccct 1260
   tcagacaagc tgagcttcct caaatgacac atgctggagt gagtggattg ctatgcccaa 1320
   aacaaaaaca gcttcctggg tgtagtaggc gatttccgca gcgactctca tgtaaatcct 1380
   acttgattga gcatttaggt ccaatctgct gctgcccttt ttgccttgac acgatcgttc 1440
   gttcgcccgt caatggtgtg ttcttcgtta ttgtgaattt gtgattggga accaaaggtg 1500
   gcatacggga ttacatcagg cagcgtgtgt tttgttcagc ttaaccgatc attgaaccca 1560
   ttgatgatga tgatgatgtt tatatagtgc acacatcact taaaaaaaaa aaaaaaaaaa 1620
   aaaaaaaaaa aa 1632
<210> 35
   <211> 40
   <212> DNA
   <213> artificial sequence
<220>
   <223> PRIMER
<400> 35
   cgtcggcctg catggcccta cttctggcta tttctcagtg 40
<210> 36
   <211> 26
   <212> DNA
   <213> artificial sequence
<220>
   <223> PRIMER
<400> 36
   ctgtccatgg cggccatcac gctcct 26
<210> 37
   <211> 40
   <212> DNA
   <213> artificial sequence
<220>
   <223> PRIMER
<400> 37
   cgatggcctg catggcccta ggtctggcca tttctcaatg 40
<210> 38
   <211> 32
   <212> DNA
   <213> artificial sequence
<220>
   <223> PRIMER
<400> 38
   taggataaga tagcaaatcc atggccatca ta 32